(19) Europäisches Patentamt
European Patent Office
Office européen des brevets

(11) EP 3 991 749 A2

(12) EUROPEAN PATENT APPLICATION

(43) Date of publication:
**04.05.2022 Bulletin 2022/18**

(21) Application number: **21199914.9**

(22) Date of filing: **14.07.2016**

(51) International Patent Classification (IPC):
***A61K 39/395*** *(2006.01)* ***A61K 31/4245*** *(2006.01)*
***A61K 31/498*** *(2006.01)* ***A61K 45/00*** *(2006.01)*
***A61P 35/00*** *(2006.01)* ***A61P 35/02*** *(2006.01)*

(52) Cooperative Patent Classification (CPC):
(C-Sets available)
**A61K 31/498; A61K 31/4245; A61K 39/39558; A61K 45/06; A61P 35/00; A61P 35/02; C07K 16/28; C07K 16/2803; C07K 16/2863; C07K 16/2866; C07K 16/32;** C07K 2317/24; C07K 2317/732 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.07.2015 US 201562192173 P**

(62) Document number(s) of the earlier application(s) in accordance with Art. 76 EPC:
**16824090.1 / 3 322 444**

(71) Applicant: **Kyowa Kirin Co., Ltd.**
**Tokyo 100-0004 (JP)**

(72) Inventors:
- **TOKUNAGA, Akihiro**
  **Tokyo, 100-8185 (JP)**
- **ISHII, Toshihiko**
  **Tokyo, 100-8185 (JP)**
- **MIE, Motoya**
  **Tokyo, 100-8185 (JP)**
- **ANDO, Munetoshi**
  **Tokyo, 100-8185 (JP)**

(74) Representative: **Vossius & Partner Patentanwälte Rechtsanwälte mbB**
**Siebertstraße 3**
**81675 München (DE)**

Remarks:
This application was filed on 29-09-2021 as a divisional application to the application mentioned under INID code 62.

# (54) A THERAPEUTIC AGENT FOR A TUMOR COMPRISING AN IDO INHIBITOR ADMINISTERED IN COMBINATION WITH AN ANTIBODY

(57) The present invention provides a method for treating a tumor comprising administering an effective amount of an antibody which specifically binds to human CC chemokine receptor 4, human epidermal growth factor receptor 2, human CD20, or epidermal growth factor receptor, and an indoleamine 2,3-dioxygenase inhibitor to a human in need thereof, and the like.

EP 3 991 749 A2

(52) Cooperative Patent Classification (CPC): (Cont.)

C-Sets
**A61K 31/4245, A61K 2300/00;**
**A61K 31/498, A61K 2300/00;**
**A61K 39/39558, A61K 2300/00**

## Description

## TECNICAL FIELD

The present invention relates to a therapeutic agent for a tumor comprising an IDO inhibitor administered in combination with an antibody or the like.

## Background Art

Recently it has been reported that indoleamine 2,3-dioxygenase (IDO), which is a tryptophan-metabolizing enzyme, inhibits the proliferation of T cells and natural killer cells (NK cells) and activates regulatory T cells, thereby causing the depression of the host immune system. The expression of IDO is increased in tumor tissues and induced by IFN-ystimulation in cancer cells and dendritic cells (for example, J. Clin. Invest., vol. 117, No. 5, pp. 1147-1154 (2007)). In a human body, 90% of an essential amino acid, tryptophan, is metabolized into kynurenine and subsequently into 3OH-kynurenine, quinolinic acid, and the like in the kynurenine pathway, the initiation step of which involves IDO.

Activation of IDO decreases the tryptophan concentration and increases the kynurenine concentration in a local or systemic manner, and the tryptophan metabolites including kynurenine induce the death of T cells and NK cells (for example, J. Exp. Med., vol. 196, No. 4, pp. 447-457 (2002)). The tryptophan metabolism also induces the conversion of $CD4^{+}CD25^{-}$ T cells into regulatory T cells (for example, Blood, vol. 109, No. 7, pp. 2871-2877 (2007)). In the culture supernatant of dendritic cells in which the expression of IDO is induced by INF-$\gamma$, the tryptophan concentration is decreased and the kynurenine concentration is increased. When T cells are co-cultured with such dendritic cells, T cell proliferation is suppressed compared to co-culture with unstimulated dendritic cells (for example, J. Exp. Med., vol. 196, No. 4, pp. 447-457 (2002)).

From the above, in the tumor environment with an increased expression of IDO, an increased kynurenine concentration induced by tryptophan metabolism suppresses antitumor effector cells, which is considered to be one of the immune escape mechanisms in tumors (for example, J. Clin. Invest., vol. 117, No. 5, pp. 1147-1154 (2007)).

An increased expression of IDO in the tumor tissues of colorectal cancer and prostate cancer has been reported (for example, Clin. Cancer Res., vol. 12, No. 4, pp. 1144-1151 (2006); and Eur. J. Cancer, vol. 44, No. 15, pp. 2266-2275 (2008)). In acute myeloid leukemia cells, IDO is constantly expressed (for example, Leukemia, vol. 21, pp. 353-355 (2007)). It has also been reported that when patients with endometrial cancer, melanoma or ovarian cancer has an increased expression of IDO, the patients will have a poor prognosis (for example, Br. J. Cancer, vol. 95, No. 11, pp. 1555-1561 (2006); J. Clin. Invest., vol. 114, No. 2, pp. 280-290 (2004); and Clin. Cancer Res., vol. 11, No. 16, pp. 6030-6039 (2005)). In adult T cell leukemia lymphoma and acute myeloid leukemia, the kynurenine/tryptophan ratio in the blood is increased (for example, Leuk. Res., vol. 33, No. 1, pp. 39-45 (2009); and Leuk. Res., vol. 33, No. 3, pp. 490-494 (2009)). It has also been reported that melanoma patients with an increased kynurenine/tryptophan ratio in the blood will have a poor prognosis (for example, Dermatology, vol. 214, No. 1, pp. 8-14 (2007)). As described above, IDO and/or kynurenine is considered to be involved in various types of solid cancers and hematologic cancers.

A tryptophan derivative, 1-methyltryptophan (1-MT), antagonizes tryptophan, thereby inhibiting the production of kynurenine (for example, Cancer Res., vol. 67, No. 2, pp. 792-800 (2007)). 1-MT cancels the suppression of T cell proliferation in the presence of IDO-expressing cancer cells or IDO-expressing dendritic cells (for example, Cancer Res., vol. 67, No. 2, pp. 792-800 (2007)). Further, 1-MT induces major histocompatibility complex (MHC)-restricted rejection in allogeneic pregnant mice (for example, Nat. Immunol., vol. 2, No. 1, pp. 64-68 (2001)). These results suggest that inhibition of IDO suppresses the production of kynurenine and induces immunity.

1-MT shows an antitumor effect in tumor-bearing mice with mouse melanoma cells. This effect disappears in immunodeficient mice (for example, Cancer Res., vol. 67, No. 2, pp. 792-800 (2007)). These results suggest that the antitumor effect of 1-MT is based on immunostimulation by IDO inhibition-mediated inhibitory effect on the production of kynurenine.

In addition, compounds showing an inhibitory effect on the production of kynurenine and/or on IDO are known to exhibit an immunostimulatory effect (for example, Nat. Immunol., vol. 2, pp. 64-68 (2001)).

Examples of IDO inhibitors include above-mentioned tryptophan derivatives including 1-MT described in WO99/29310, quinoxaline derivatives described in WO2010/053182, WO2011/142316 and WO2013/069765, 1,2,5-oxadiazole derivatives described in WO2006/122150 and WO2010/005958, urea derivatives described in WO2015/002918 and anilide derivatives described in WO2015/002918.

IDOI is known to be expressed in tumors or the microenvironment, and plays an important regulatory role in the immunosuppressive mechanisms, which is responsible for tumor's escape from host immune surveillance. It has been reported that tryptophan (Trp) metabolites, such as kynurenine (Kyn), induce NK cell death, and weaken NK cell cytotoxicity by inhibiting the expression of NK cell receptors. Therefore, there is a possibility that IDO1 activity affects antibody dependent cellular cytotoxicity(ADCC).

Lenalidomide is a standard therapeutic agent for multiple myeloma and it is known to enhance an ADCC activity of a therapeutic antibody such as anti-CD20 antibody and anti-CD40 antibody (see Non Patent Literatures 1 and 2).
Imatinib inhibits c-kit (stem cell factor receptor) and platelet-derived growth factor receptor and also activates NK cells through dendritic cells to show antitumor effects (see Non Patent Literature 3).
Further, dacarbazine, which is a therapeutic agent for melanoma, and sorafenib, which is a therapeutic agent for renal cell carcinoma and hepatocarcinoma, is known to activate NK cells (see Non Patent Literatures 4 and 5).

**Citation List**

**Non Patent Literature**

Non Patent Literature 1:Clin. Cancer Res. vol. 11, p5984, 2005
Non Patent Literature 2:Br. J. Hematol. vol. 144, p848, 2009
Non Patent Literature 3:J Clin Invest. 2004 Aug 1; 114(3): 379-388
Non Patent Literature 4:Journal of Investigative Dermatology (2013) 133, 499-508
Non Patent Literature 5:HEPATOLOGY, Vol. 57, No. 6, 2013, 2358-2368

**Summary of Invention**

**Technical Problem**

An object of the present invention is to provide a therapeutic agent for a tumor, comprising an IDO inhibitor administered in combination with an antibody, or the like.

**Solution to Problem**

The present invention relates to the following (1) to (210).

(1) A method for treating a tumor comprising administering an effective amount of an antibody which specifically binds to human CC chemokine receptor 4, human epidermal growth factor receptor 2, human CD20, or epidermal growth factor receptor, and an indoleamine 2,3-dioxygenase inhibitor to a human in need thereof.
(2) The method according to (1), wherein the indoleamine 2,3-dioxygenase inhibitor is Compound (I) represented by formula (I)

[Chem.1]

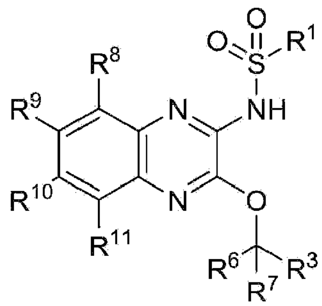

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.2]

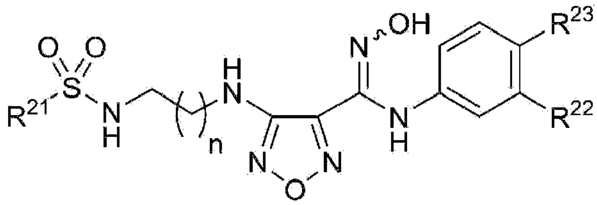

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.

(3) The method according to (1) or (2), wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.

(4) The method according to any one of (1) to (3), wherein the antibody and the indoleamine 2,3-dioxygenase inhibitor are administered simultaneously or sequentially.

(5) The method according to any one of (1) to (4), wherein the tumor is a tumor which expresses human CC chemokine receptor 4, human epidermal growth factor receptor 2, human CD20, or epidermal growth factor receptor.

(6) The method according to (5), wherein the tumor which expresses human CC chemokine receptor 4 is T cell lymphoma.

(7) The method according to (5), wherein the tumor which expresses human CC chemokine receptor 4 is peripheral T cell lymphoma, cutaneous T cell lymphoma or adult T cell leukemic lymphoma.

(8) The method according to (5), wherein the tumor which expresses human epidermal growth factor receptor 2 is breast cancer, gastric cancer, ovarian cancer, osteosarcoma, or endometrial cancer.

(9) The method according to (5), wherein the tumor which expresses human epidermal growth factor receptor 2 is breast cancer.

(10) The method according to (5), wherein the tumor which expresses human CD20 is chronic leukemia, or non-Hodgkin's lymphoma.

(11) The method according to (10), wherein the chronic leukemia is chronic lymphocytic leukemia.

(12) The method according to (10), wherein the non-Hodgkin's lymphoma is B cell lymphoma.

(13) The method according to (12), wherein the B cell lymphoma is mantle cell lymphoma, diffuse large B cell lymphoma or Burkitt's lymphoma.

(14) The method according to (12), wherein the B cell lymphoma is Burkitt's lymphoma.

(15) The method according to (5), wherein the tumor which expresses epidermal growth factor receptor is colon cancer, head and neck cancer, gastric cancer, hepatic cancer.

(16) A method for suppressing decreasing antibody dependent cellular cytotoxicity activity of an antibody which specifically binds to human CC chemokine receptor 4, human epidermal growth factor receptor 2, human CD20, or epidermal growth factor receptor comprising administering an indoleamine 2,3-dioxygenase inhibitor.

(17) The method according to (16), wherein the indoleamine 2,3-dioxygenase inhibitor is Compound (I) represented by formula (I)

[Chem.3]

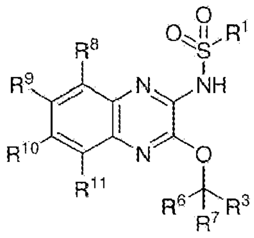

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.4]

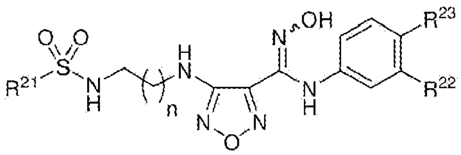

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.
(18) A pharmaceutical composition for use in administering an effective amount of an antibody which specifically binds to human CC chemokine receptor 4, human epidermal growth factor receptor 2, human CD20, or epidermal growth factor receptor, and an indoleamine 2,3-dioxygenase inhibitor.
(19) The pharmaceutical composition according to (18), wherein the indoleamine 2,3-dioxygenase inhibitor is Compound (I) represented by formula (I)

[Chem.5]

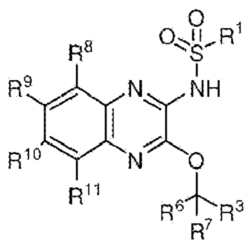

(I)

wherein

$R^6$ and $R^7$ may be the same or different, and each represents a hydrogen atom, or optionally substituted lower alkyl,

$R^8$, $R^9$, $R^{10}$, and $R^{11}$ may be the same or different, and each represents a hydrogen atom, halogen, cyano, or lower alkyl,

$R^1$ represents lower alkyl which may be substituted with lower alkoxy, and

$R^3$ represents an optionally substituted aromatic heterocyclic group,

or a pharmaceutically acceptable salt thereof,
or Compound (II) represented by formula (II)

[Chem.6]

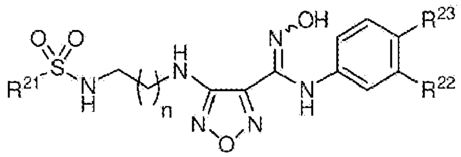

(II)

wherein

$R^{21}$ represents amino or methyl,

$R^{22}$ represents halogen, cyano, trifluoromethyl, or lower alkyl,

$R^{23}$ represents a hydrogen atom, or halogen, and

n represents 1 or 2,

or a pharmaceutically acceptable salt thereof.

(20) The pharmaceutical composition according to (18) or (19), wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.

(21) The pharmaceutical composition according to any one of (18) to (20), which comprises simultaneously or sequentially administering the antibody and the indoleamine 2,3-dioxygenase inhibitor.

(22) The pharmaceutical composition according to any one of (18) to (21), wherein the tumor is a tumor which expresses human CC chemokine receptor 4, human epidermal growth factor receptor 2, human CD20, or epidermal growth factor receptor.

(23) The pharmaceutical composition according to (22), wherein the tumor which expresses human CC chemokine receptor 4 is T cell lymphoma.

(24) The pharmaceutical composition according to (22), wherein the tumor which expresses human CC chemokine receptor 4 is peripheral T cell lymphoma, cutaneous T cell lymphoma or adult T cell leukemic lymphoma.

(25) The pharmaceutical composition according to (22), wherein the tumor which expresses human epidermal growth factor receptor 2 is breast cancer, gastric cancer, ovarian cancer, osteosarcoma, or endometrial cancer.

(26) The pharmaceutical composition according to (22), wherein the tumor which expresses human epidermal growth factor receptor 2 is breast cancer.

(27) The pharmaceutical composition according to (22), wherein the tumor which expresses human CD20 is chronic leukemia, or non-Hodgkin's lymphoma.

(28) The pharmaceutical composition according to (27), wherein the chronic leukemia is chronic lymphocytic leukemia.

(29) The pharmaceutical composition according to (27), wherein the non-Hodgkin's lymphoma is B cell lymphoma.

(30) The pharmaceutical composition according to (29), wherein the B cell lymphoma is mantle cell lymphoma, diffuse large B cell lymphoma or Burkitt's lymphoma.

(31) The pharmaceutical composition according to (29), wherein the B cell lymphoma is Burkitt's lymphoma.

(32) The pharmaceutical composition according to (22), wherein the tumor which expresses epidermal growth factor receptor is colon cancer, head and neck cancer, gastric cancer, hepatic cancer.

(33) A combination of an indoleamine 2,3-dioxygenase inhibitor and an antibody which specifically binds to human CC chemokine receptor 4, human epidermal growth factor receptor 2, human CD20, or epidermal growth factor receptor for use in the treatment of a tumor.

(34) The combination of an indoleamine 2,3-dioxygenase inhibitor and an antibody according to (33), wherein the indoleamine 2,3-dioxygenase inhibitor is Compound (I) represented by formula (I)

[Chem.7]

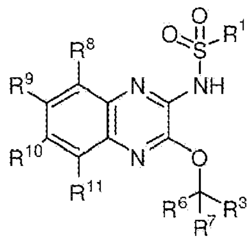

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.8]

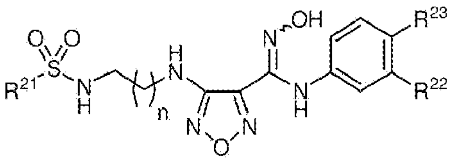

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.
(35) The combination of an indoleamine 2,3-dioxygenase inhibitor and an antibody according to (33) or (34), wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.
(36) The combination of an indoleamine 2,3-dioxygenase inhibitor and an antibody according to any one of (33) to (35), wherein the antibody and the indoleamine 2,3-dioxygenase inhibitor are administered simultaneously or sequentially.
(37) The combination of an indoleamine 2,3-dioxygenase inhibitor and an antibody according to any one of (33) to (36), wherein the tumor is a tumor which expresses human CC chemokine receptor 4, human epidermal growth factor receptor 2, human CD20, or epidermal growth factor receptor.
(38) The combination of an indoleamine 2,3-dioxygenase inhibitor and an antibody according to (37), wherein the tumor which expresses human CC chemokine receptor 4 is T cell lymphoma.
(39) The combination of an indoleamine 2,3-dioxygenase inhibitor and an antibody according to (37), wherein the tumor which expresses human CC chemokine receptor 4 is peripheral T cell lymphoma, cutaneous T cell lymphoma or adult T cell leukemic lymphoma.
(40) The combination of an indoleamine 2,3-dioxygenase inhibitor and an antibody according to (37), wherein the tumor which expresses human epidermal growth factor receptor 2 is breast cancer, gastric cancer, ovarian cancer, osteosarcoma or endometrial cancer.

(41) The combination of an indoleamine 2,3-dioxygenase inhibitor and an antibody according to (37), wherein the tumor which expresses human epidermal growth factor receptor 2 is breast cancer.

(42) The combination of an indoleamine 2,3-dioxygenase inhibitor and an antibody according to (37), wherein the tumor which expresses human CD20 is chronic leukemia or non-Hodgkin's lymphoma.

(43) The combination of an indoleamine 2,3-dioxygenase inhibitor and an antibody according to (42), wherein the chronic leukemia is chronic lymphocytic leukemia.

(44) The combination of an indoleamine 2,3-dioxygenase inhibitor and an antibody according to (42), wherein the non-Hodgkin's lymphoma is B cell lymphoma.

(45) The combination of an indoleamine 2,3-dioxygenase inhibitor and an antibody according to (44), wherein the B cell lymphoma is mantle cell lymphoma, diffuse large B cell lymphoma or Burkitt's lymphoma.

(46) The combination of an indoleamine 2,3-dioxygenase inhibitor and an antibody according to (44), wherein the B cell lymphoma is Burkitt's lymphoma.

(47) The combination of an indoleamine 2,3-dioxygenase inhibitor and an antibody according to (37), wherein the tumor which expresses epidermal growth factor receptor is colon cancer, head and neck cancer, gastric cancer or hepatic cancer.

(48) An indoleamine 2,3-dioxygenase inhibitor for use in suppressing decreasing antibody dependent cellular cytotoxicity activity of an antibody which specifically binds to human CC chemokine receptor 4, human epidermal growth factor receptor 2, human CD20, or epidermal growth factor receptor.

(49) The indoleamine 2,3-dioxygenase inhibitor according to (48), wherein the indoleamine 2,3-dioxygenase inhibitor is Compound (I) represented by formula (I)

[Chem.9]

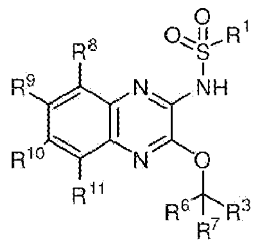

(I)

wherein

$R^6$ and $R^7$ may be the same or different, and each represents a hydrogen atom, or optionally substituted lower alkyl,

$R^8$, $R^9$, $R^{10}$, and $R^{11}$ may be the same or different, and each represents a hydrogen atom, halogen, cyano, or lower alkyl,

$R^1$ represents lower alkyl which may be substituted with lower alkoxy, and

$R^3$ represents an optionally substituted aromatic heterocyclic group,

or a pharmaceutically acceptable salt thereof,
or Compound (II) represented by formula (II)

[Chem.10]

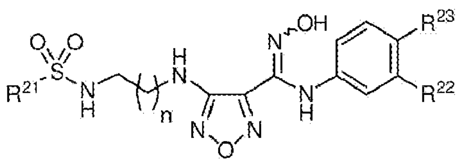

(II)

wherein

$R^{21}$ represents amino or methyl,

$R^{22}$ represents halogen, cyano, trifluoromethyl, or lower alkyl,

$R^{23}$ represents a hydrogen atom, or halogen, and

n represents 1 or 2,

or a pharmaceutically acceptable salt thereof.

(50) A therapeutic agent for a tumor, comprising an indoleamine 2,3-dioxygenase inhibitor as an active ingredient, wherein the therapeutic agent is administered in combination with an effective amount of an antibody which specifically binds to human CC chemokine receptor 4, human epidermal growth factor receptor 2, human CD20, or epidermal growth factor receptor.

(51) The therapeutic agent for a tumor according to (50), wherein the indoleamine 2,3-dioxygenase inhibitor is Compound (I) represented by formula (I)

[Chem.11]

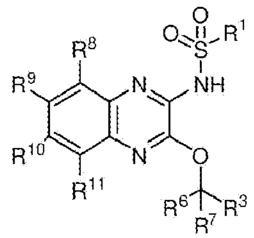

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.12]

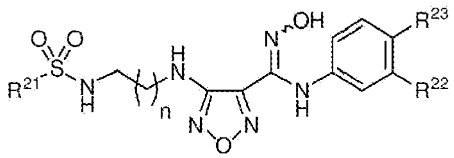

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.

(52) The therapeutic agent for a tumor according to (50) or (51), wherein the antibody which specifically binds to human CC chemokine receptor 4, human epidermal growth factor receptor 2, human CD20, or epidermal growth factor is mogamulizumab, trastuzumab, rituximab or cetuximab, respectively.

(53) The therapeutic agent for a tumor according to any one of (50) to (52), wherein the therapeutic agent and the antibody are administered simultaneously or sequentially.

(54) The therapeutic agent for a tumor according to any one of (50) to (53), wherein the tumor is a tumor which expresses human CC chemokine receptor 4, human epidermal growth factor receptor 2, human CD20, or epidermal growth factor receptor.

(55) The therapeutic agent for a tumor according to (54), wherein the tumor which expresses human CC chemokine

receptor 4 is T cell lymphoma.

(56) The therapeutic agent for a tumor according to (54), wherein the tumor which expresses human CC chemokine receptor 4 is peripheral T cell lymphoma, cutaneous T cell lymphoma or adult T cell leukemic lymphoma.

(57) The therapeutic agent for a tumor according to (54), wherein the tumor which expresses human epidermal growth factor receptor 2 is breast cancer, gastric cancer, ovarian cancer, osteosarcoma, or endometrial cancer.

(58) The therapeutic agent for a tumor according to (54), wherein the tumor which expresses human epidermal growth factor receptor 2 is breast cancer.

(59) The therapeutic agent for a tumor according to (54), wherein the tumor which expresses human CD20 is chronic leukemia, or non-Hodgkin's lymphoma.

(60) The therapeutic agent for a tumor according to (59), wherein the chronic leukemia is chronic lymphocytic leukemia.

(61) The therapeutic agent for a tumor according to (59), wherein the non-Hodgkin's lymphoma is B cell lymphoma.

(62) The therapeutic agent for a tumor according to (61), wherein the B cell lymphoma is mantle cell lymphoma, diffuse large B cell lymphoma or Burkitt's lymphoma.

(63) The therapeutic agent for a tumor according to (61), wherein the B cell lymphoma is Burkitt's lymphoma.

(64) The therapeutic agent for a tumor according to (54), wherein the tumor which expresses epidermal growth factor receptor is colon cancer, head and neck cancer, gastric cancer, or hepatic cancer.

(65) A suppressing agent for decreasing antibody dependent cellular cytotoxicity activity of an antibody which specifically binds to human CC chemokine receptor 4, human epidermal growth factor receptor 2, human CD20, or epidermal growth factor receptor, comprising an indoleamine 2,3-dioxygenase inhibitor as an active ingredient.

(66) The suppressing agent according to (65), wherein the indoleamine 2,3-dioxygenase inhibitor is Compound (I) represented by formula (I)

[Chem.13]

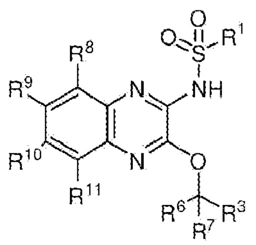

(I)

wherein

$R^6$ and $R^7$ may be the same or different, and each represents a hydrogen atom, or optionally substituted lower alkyl,

$R^8$, $R^9$, $R^{10}$, and $R^{11}$ may be the same or different, and each represents a hydrogen atom, halogen, cyano, or lower alkyl,

$R^1$ represents lower alkyl which may be substituted with lower alkoxy, and

$R^3$ represents an optionally substituted aromatic heterocyclic group,

or a pharmaceutically acceptable salt thereof,
or Compound (II) represented by formula (II)

[Chem.14]

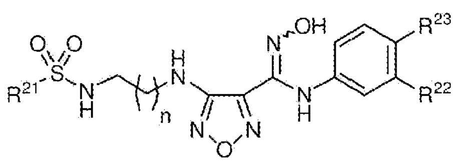

(II)

wherein

$R^{21}$ represents amino or methyl,

$R^{22}$ represents halogen, cyano, trifluoromethyl, or lower alkyl,

$R^{23}$ represents a hydrogen atom, or halogen, and
n represents 1 or 2,

or a pharmaceutically acceptable salt thereof.
(67) The method according to (16) or (17), wherein the antibody and the indoleamine 2,3-dioxygenase inhibitor are administered simultaneously or sequentially.
(68) The method according to (16) or (17), wherein the antibody which specifically binds to human CC chemokine receptor 4, human epidermal growth factor receptor 2, human CD20, or epidermal growth factor is mogamulizumab, trastuzumab, rituximab or cetuximab, respectively.
(69) The indoleamine 2,3-dioxygenase inhibitor according to (48) or (49), wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.
(70) A therapeutic agent for a tumor, comprising an antibody which specifically binds to human CC chemokine receptor 4, human epidermal growth factor receptor 2, human CD20, or epidermal growth factor receptor as an active ingredient, wherein the therapeutic agent is administered in combination with an effective amount of an indoleamine 2,3-dioxygenase inhibitor.
(71) The therapeutic agent for a tumor according to (70), wherein the indoleamine 2,3-dioxygenase inhibitor is Compound (I) represented by formula (I)

[Chem.15]

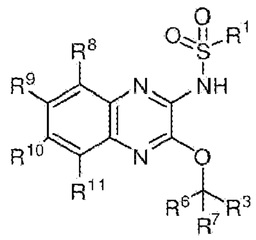

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.16]

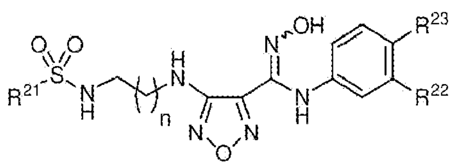

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.

(72) The therapeutic agent for a tumor according to (70) or (71), wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.
(73) The therapeutic agent for a tumor according to any one of (70) to (72), wherein the therapeutic agent and the indoleamine 2,3-dioxygenase inhibitor are administered simultaneously or sequentially.
(74) The therapeutic agent for a tumor according to any one of (70) to (73), wherein the tumor is a tumor which expresses human CC chemokine receptor 4, human epidermal growth factor receptor 2, human CD20, or epidermal growth factor receptor.
(75) The therapeutic agent for a tumor according to (74), wherein the tumor which expresses human CC chemokine receptor 4 is T cell lymphoma.
(76) The therapeutic agent for a tumor according to (74), wherein the tumor which expresses human CC chemokine receptor 4 is peripheral T cell lymphoma, cutaneous T cell lymphoma or adult T cell leukemic lymphoma.
(77) The therapeutic agent for a tumor according to (74), wherein the tumor which expresses human epidermal growth factor receptor 2 is breast cancer, gastric cancer, ovarian cancer, osteosarcoma, or endometrial cancer.
(78) The therapeutic agent for a tumor according to (74), wherein the tumor which expresses human epidermal growth factor receptor 2 is breast cancer.
(79) The therapeutic agent for a tumor according to (74), wherein the tumor which expresses human CD20 is chronic leukemia, or non-Hodgkin's lymphoma.
(80) The therapeutic agent for a tumor according to (79), wherein the chronic leukemia is chronic lymphocytic leukemia.
(81) The therapeutic agent for a tumor according to (79), wherein the non-Hodgkin's lymphoma is B cell lymphoma.
(82) The therapeutic agent for a tumor according to (81), wherein the B cell lymphoma is mantle cell lymphoma, diffuse large B cell lymphoma or Burkitt's lymphoma.
(83) The therapeutic agent for a tumor according to (81), wherein the B cell lymphoma is Burkitt's lymphoma.
(84) The therapeutic agent for a tumor according to (74), wherein the tumor which expresses epidermal growth factor receptor is colon cancer, head and neck cancer, gastric cancer, or hepatic cancer.
(85) The suppressing agent according to (65) or (66), wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.
(86) A method for treating a tumor comprising administering an effective amount of an antibody which specifically binds to human folate receptor 1, human IL-3Rα or human TIM-3 and an indoleamine 2,3-dioxygenase inhibitor to a human in need thereof.
(87) The method according to (86), wherein the indoleamine 2,3-dioxygenase inhibitor is Compound (I) represented by formula (I)

[Chem.17]

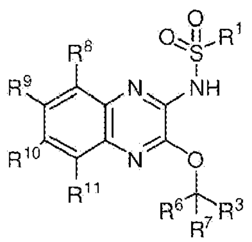

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.18]

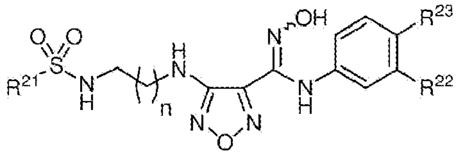 (II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.
(88) The method according to (86) or (87), wherein the antibody which specifically binds to human folate receptor 1 is a monoclonal antibody.
(89) The method according to (86) or (87), wherein the antibody which specifically binds to human folate receptor 1 is a monoclonal antibody that is selected from the following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

(90) The method according to (86) or (87), wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.
(91) The method according to (90), wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs: 12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

(92) The method according to (90), wherein the antibody to a human IL-3Rα chain comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
(93) The method according to (86) or (87), wherein the antibody which specifically binds to human TIM-3 is a monoclonal antibody, which binds to an extracellular region of human TIM-3.
(94) The method according to (93), wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

(95) The method according to (93), wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

(96) The method according to any one of (86) to (95), wherein the antibody and the indoleamine 2,3-dioxygenase inhibitor are administered simultaneously or sequentially.
(97) The method according to any one of (86) to (96), wherein the tumor is a tumor associated with human folate receptor 1, human IL-3Rα or human TIM-3.
(98) The method according to (97), wherein the tumor which is associated with human folate receptor 1 is blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma or pancreatic cancer.
(99) The method according to (97), wherein the tumor which is associated with human folate receptor 1 is ovarian cancer.
(100) The method according to (97), wherein the tumor which is associated with human IL-3Rα is acute myeloid leukemia (AML), acute lymphocytic leukemia, atypical leukemia, chronic lymphocytic leukemia, adult T cell leukemia, NK/T cell lymphoma, granular lymphocytosis (LGL leukemia), polycythemia vera, essential thrombocythemia, hypereosinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma or Castleman disease.
(101) The method according to (97), wherein the tumor which is associated with human IL-3Rα is acute myeloid leukemia (AML).
(102) The method according to (97), wherein the tumor which is associated with human TIM-3 is blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer.
(103) The method according to (97), wherein the tumor which is associated with human TIM-3 is acute myeloid leukemia (AML).
(104) A method for suppressing decreasing antibody dependent cellular cytotoxicity activity of an antibody which specifically binds to human folate receptor 1, human IL-3Rα or human TIM-3 comprising administering an indoleamine 2,3-dioxygenase inhibitor.
(105) The method according to (104), wherein the indoleamine 2,3-dioxygenase inhibitor is Compound (I) represented by formula (I)

[Chem.19]

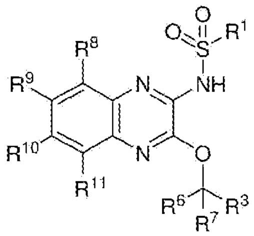

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.20]

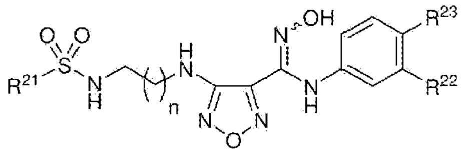

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.
(106) The method according to (104) or (105), wherein the antibody which specifically binds to human folate receptor 1 is a monoclonal antibody.
(107) The method according to (104) or (105), wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

(108) The method according to (104) or (105), wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.

(109) The method according to (108), wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

(110) The method according to (108), wherein the antibody to a human IL-3Rα chain comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
(111) The method according to (104) or (105), wherein the antibody which specifically binds to human TIM-3 is a monoclonal antibody, which binds to an extracellular region of human TIM-3.
(112) The method according to (111), wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs 1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs: 51 to 53, respectively, and comprises an L chain of an antibody which comprises CDRs 1 to 3 comprising the amino acid sequences represented by SEQ ID NOs:54 to 56, respectively.

(113) The method according to (111), wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

(114) The method according to any one of (104) to (113), wherein the antibody and the indoleamine 2,3-dioxygenase inhibitor are administered simultaneously or sequentially.
(115) A pharmaceutical composition for use in administering an effective amount of an antibody which specifically binds to human folate receptor 1, human IL-3Rα or human TIM-3, and an indoleamine 2,3-dioxygenase inhibitor.
(116) The pharmaceutical composition according to (115), wherein the indoleamine 2,3-dioxygenase inhibitor is Compound (I) represented by formula (I)

[Chem.21]

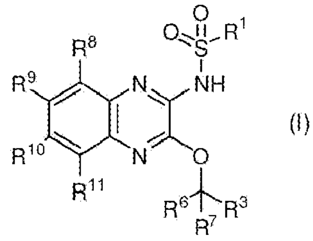

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.22]

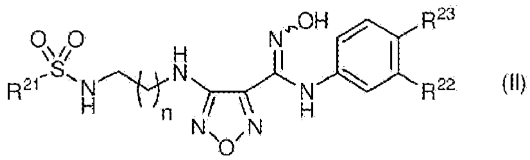

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.
(117) The pharmaceutical composition according to (115) or (116) which specifically binds to human folate receptor 1 is a monoclonal antibody.
(118) The pharmaceutical composition which specifically binds to human folate receptor 1 according to (115) or (116) is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

(119) The pharmaceutical composition according to (115) or (116), wherein the antibody which specifically binds to human IL-3Rα is an antibody to a human IL-3Rα chain, which does not inhibit IL-3 signaling and binds to B domain

of the human IL-3Rα chain but does not bind to C domain of the human IL-3Rα chain.

(120) The pharmaceutical composition according to (119), wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs: 12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

(121) The pharmaceutical composition according to (119), wherein the antibody to a human IL-3Rα chain which comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
(122) The pharmaceutical composition according to (115) or (116), wherein the antibody which specifically binds to human TIM-3 is a monoclonal antibody, which binds to an extracellular region of human TIM-3.
(123) The pharmaceutical composition according to (122), wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs 1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively,
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

(124) The pharmaceutical composition according to (122), wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

(125) The pharmaceutical composition according to (115) or (116), which comprises simultaneously or sequentially administering the antibody and the indoleamine 2,3-dioxygenase inhibitor.
(126) The pharmaceutical composition according to any one of (115) to (125), wherein the tumor is a tumor associated with human folate receptor 1, human IL-3Rα or human TIM-3.
(127) The pharmaceutical composition according to (126), wherein the tumor is associated with human folate receptor 1 is blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma or pancreatic cancer.
(128) The pharmaceutical composition according to (126), wherein the tumor which is associated with human folate receptor 1 is ovarian cancer.

(129) The pharmaceutical composition according to (126), wherein the tumor which is associated with human IL-3Rα is acute myeloid leukemia (AML), acute lymphocytic leukemia, atypical leukemia, chronic lymphocytic leukemia, adult T cell leukemia, NK/T cell lymphoma, granular lymphocytosis (LGL leukemia), polycythemia vera, essential thrombocythemia, hypereosinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma or Castleman disease.

(130) The pharmaceutical composition according to (126), wherein the tumor which is associated with human IL-3Rα is acute myeloid leukemia (AML).

(131) The pharmaceutical composition according to (126), wherein the tumor which is associated with human TIM-3 is blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer.

(132) The pharmaceutical composition according to (126), wherein the tumor which is associated with human TIM-3 is acute myeloid leukemia (AML).

(133) A combination of an indoleamine 2,3-dioxygenase inhibitor and an antibody which specifically binds to human folate receptor 1, human IL-3Rα or human TIM-3, for use in the treatment of a tumor.

(134) The combination according to (133), wherein the indoleamine 2,3-dioxygenase inhibitor is Compound (I) represented by formula (I)

[Chem.23]

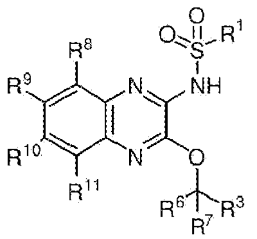

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.24]

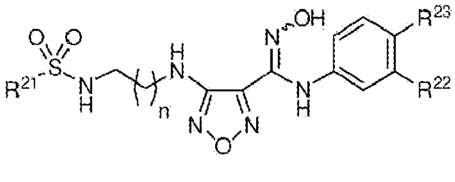

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.

(135) The combination according to (133) or (134), wherein the antibody which specifically binds to human folate receptor 1 is a monoclonal antibody.

(136) The combination according to (133) or (134), wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;

(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and (c1) a monoclonal antibody in which H chain of the antibody comprises the amino acid sequence represented by SEQ ID NO. 7, and L chain of the antibody comprises the amino acid sequence represented by SEQ ID NO. 8.

(137) The combination according to (133) or (134), wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.

(138) The combination according to (137), wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,

(b2) CDR 1 to 3 of heavy chain are the amino acid sequences of SEQ ID NOs: 12 to 14, respectively, and CDR 1 to 3 of light chain are the amino acid sequences of SEQ ID NOs:27 to 29, respectively,

(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,

(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and

(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

(139) The combination according to (137), wherein the antibody to a human IL-3Rα chain comprises amino acid sequences of CDRs of heavy chain and CDRs of light chain consisting of the following (e2);

(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

(140) The combination according to (133) or (134), wherein the antibody which specifically binds to human TIM-3 is a monoclonal antibody, which binds to an extracellular region of human TIM-3.

(141) The combination according to (140), wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs 1 to 3 comprising the amino acid sequences represented by SEQ ID NOs:42 to 44, respectively,

(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and

(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

(142) The combination according to (140), wherein the monoclonal antibody is one selected from the group consisting

of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

(143) The combination according to any one of (133) to (142), wherein the antibody and the indoleamine 2,3-dioxygenase inhibitor are administered simultaneously or sequentially.
(144) The combination according to any one of (133) to (143), wherein the tumor is a tumor associated with human folate receptor 1, human IL-3Rα or human TIM-3.
(145) The combination according to (144), wherein the tumor which is associated with human folate receptor 1 is blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma or pancreatic cancer.
(146) The combination according to (144), wherein the tumor which is associated with human folate receptor 1 is ovarian cancer.
(147) The combination according to (144), wherein the tumor which is associated with human IL-3Rα is acute myeloid leukemia (AML), acute lymphocytic leukemia, atypical leukemia, chronic lymphocytic leukemia, adult T cell leukemia, NK/T cell lymphoma, granular lymphocytosis (LGL leukemia), polycythemia vera, essential thrombocythemia, hypereosinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma or Castleman disease.
(148) The combination according to (144), wherein the tumor which is associated with human IL-3Rα is acute myeloid leukemia (AML).
(149) The combination according to (144), wherein the tumor which is associated with human TIM-3 is blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer.
(150) The combination according to (144), wherein the tumor which is associated with human TIM-3 is acute myeloid leukemia (AML).
(151) An indoleamine 2,3-dioxygenase inhibitor for use in the suppression of decreasing antibody dependent cellular cytotoxicity activity of an antibody which specifically binds to human folate receptor 1, human IL-3Rα or human TIM-3.
(152) The indoleamine 2,3-dioxygenase inhibitor according to (151), wherein the indoleamine 2,3-dioxygenase inhibitor is Compound (I) represented by formula (I)

[Chem.25]

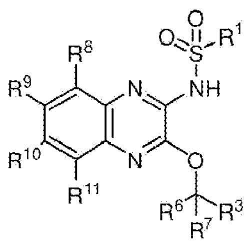

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.26]

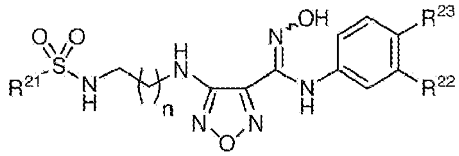

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.
(153) The indoleamine 2,3-dioxygenase inhibitor according to (151) or (152), wherein the antibody and the indoleamine 2,3-dioxygenase inhibitor are administered simultaneously or sequentially.
(154) The indoleamine 2,3-dioxygenase inhibitor according to any one of (151) to (153), wherein the antibody which specifically binds to human folate receptor 1 is a monoclonal antibody.
(155) The indoleamine 2,3-dioxygenase inhibitor according to any one of (151) to (153), wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

(156) The indoleamine 2,3-dioxygenase inhibitor according to any one of (151) to (153), wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.
(157) The indoleamine 2,3-dioxygenase inhibitor according to (156), wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

(158) The indoleamine 2,3-dioxygenase inhibitor according to (156), wherein the antibody to a human IL-3Rα chain comprises amino acid sequences of CDRs of heavy chain and CDRs of light chain consisting of the following (e2);

(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

(159) The indoleamine 2,3-dioxygenase inhibitor according to any one of (151) to (153), wherein the antibody which specifically binds to human TIM-3 is a monoclonal antibody, which binds to an extracellular region of human TIM-3.

(160) The indoleamine 2,3-dioxygenase inhibitor according to (159), wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,

(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and

(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

(161) The indoleamine 2,3-dioxygenase inhibitor according to (159), wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and

(b3) a monoclonal antibody which comprises VH of an antibody comprising the amino acid sequence represented by SEQ ID NO:59 and comprises VL of an antibody comprising the amino acid sequence represented by SEQ ID NO:60.

(162) A therapeutic agent for a tumor, comprising an antibody which specifically binds to human folate receptor 1, human IL-3Rα or human TIM-3, as an active ingredient, wherein the therapeutic agent is administered in combination with an effective amount of an indoleamine 2,3-dioxygenase inhibitor.

(163) The therapeutic agent for a tumor according to (162), wherein the indoleamine 2,3-dioxygenase inhibitor is Compound (I) represented by formula (I)

[Chem.27]

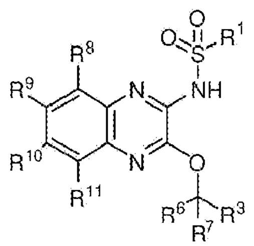

(I)

wherein

$R^6$ and $R^7$ may be the same or different, and each represents a hydrogen atom, or optionally substituted lower alkyl,

$R^8$, $R^9$, $R^{10}$, and $R^{11}$ may be the same or different, and each represents a hydrogen atom, halogen, cyano, or lower alkyl,

$R^1$ represents lower alkyl which may be substituted with lower alkoxy, and

$R^3$ represents an optionally substituted aromatic heterocyclic group,

or a pharmaceutically acceptable salt thereof,

or Compound (II) represented by formula (II)

[Chem.28]

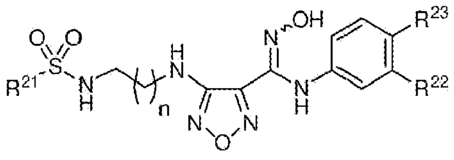

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.
(164) The therapeutic agent for a tumor according to (162) or (163), wherein the antibody which specifically binds to human folate receptor 1 is a monoclonal antibody.
(165) The therapeutic agent for a tumor according to (162) or (163), wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

(166) The therapeutic agent for a tumor according to (162) or (163), wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.
(167) The therapeutic agent for a tumor according to (166), wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

(168) The therapeutic agent for a tumor according to (166), wherein the antibody to a human IL-3Rα chain comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
(169) The therapeutic agent for a tumor according to (162) or (163), wherein the antibody which specifically binds to human TIM-3 is a monoclonal antibody, which binds to an extracellular region of human TIM-3.
(170) The therapeutic agent for a tumor according to (169), wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

(171) The therapeutic agent for a tumor according to (169), wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

(172) The therapeutic agent for a tumor according to any one of (162) to (171), wherein the therapeutic agent and the indoleamine 2,3-dioxygenase inhibitor are administered simultaneously or sequentially.
(173) The therapeutic agent for a tumor according to any one of (162) to (172), wherein the tumor is a tumor associated with human folate receptor 1, human IL-3Rα or human TIM-3.
(174) The therapeutic agent for a tumor according to (173), wherein the tumor which is associated with human folate receptor 1 is blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma or pancreatic cancer.
(175) The therapeutic agent for a tumor according to (173), wherein the tumor which is associated with human folate receptor 1 is ovarian cancer.
(176) The therapeutic agent for a tumor according to (173), wherein the tumor which is associated with human IL-3Rα is acute myeloid leukemia (AML), acute lymphocytic leukemia, atypical leukemia, chronic lymphocytic leukemia, adult T cell leukemia, NK/T cell lymphoma, granular lymphocytosis (LGL leukemia), polycythemia vera, essential thrombocythemia, hypereosinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma or Castleman disease.
(177) The therapeutic agent for a tumor according to (173), wherein the tumor which is associated with human IL-3Rα is acute myeloid leukemia (AML).
(178) The therapeutic agent for a tumor according to (173), wherein the tumor which is associated with human TIM-3 is blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer.
(179) The therapeutic agent for a tumor according to (173), wherein the tumor which is associated with human TIM-3 is acute myeloid leukemia (AML).
(180) A therapeutic agent for a tumor, comprising an indoleamine 2,3-dioxygenase inhibitor as an active ingredient, wherein the therapeutic agent is administered in combination with an effective amount of an antibody which specifically binds to human folate receptor 1, human IL-3Rα or human TIM-3.
(181) The therapeutic agent for a tumor according to (180), wherein the indoleamine 2,3-dioxygenase inhibitor is Compound (I) represented by formula (I)

[Chem.29]

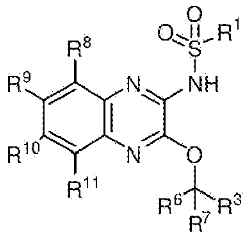

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.30]

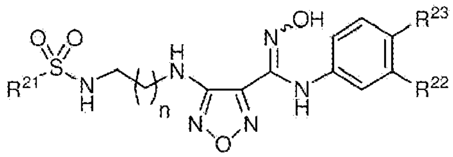

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.
(182) The therapeutic agent for a tumor according to (180) or (181), wherein the antibody which specifically binds to human folate receptor 1 is a monoclonal antibody.
(183) The therapeutic agent for a tumor according to (180) or (181), wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

(184) The therapeutic agent for a tumor according to (180) or (181), wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.

(185) The therapeutic agent for a tumor according to (184), wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs: 12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

(186) The therapeutic agent for a tumor according to (184), wherein the antibody to a human IL-3Rα chain comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
(187) The therapeutic agent for a tumor according to (180) or (181), wherein the antibody which specifically binds to human TIM-3 is a monoclonal antibody, which binds to an extracellular region of human TIM-3.
(188) The therapeutic agent for a tumor according to (187), wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

(189) The therapeutic agent for a tumor according to (187), wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

(190) The therapeutic agent for a tumor according to any one of (180) to (189), wherein the therapeutic agent and the antibody are administered simultaneously or sequentially.
(191) The therapeutic agent for a tumor according to any one of (180) to (190), wherein the tumor is a tumor associated with human folate receptor 1, human IL-3Rα or human TIM-3.
(192) The therapeutic agent for a tumor according to (191), wherein the tumor which is associated with human folate receptor 1 is blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma or pancreatic cancer.
(193) The therapeutic agent for a tumor according to (191), wherein the tumor which is associated with human folate receptor 1 is ovarian cancer.
(194) The therapeutic agent for a tumor according to (191), wherein the tumor which is associated with human IL-3Rα is acute myeloid leukemia (AML), acute lymphocytic leukemia, atypical leukemia, chronic lymphocytic leukemia, adult T cell leukemia, NK/T cell lymphoma, granular lymphocytosis (LGL leukemia), polycythemia vera, essential thrombocythemia, hypereosinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma or Castleman disease.

(195) The therapeutic agent for a tumor according to (191), wherein the tumor which is associated with human IL-3Rα is acute myeloid leukemia (AML).

(196) The therapeutic agent for a tumor according to (191), wherein the tumor which is associated with human TIM-3 is blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer.

(197) The therapeutic agent for a tumor according to (191), wherein the tumor which is associated with human TIM-3 is acute myeloid leukemia (AML).

(198) A suppressing agent for decreasing antibody dependent cellular cytotoxicity activity of an antibody which specifically binds to human folate receptor 1, human IL-3Rα or human TIM-3, comprising an indoleamine 2,3-dioxygenase inhibitor as an active ingredient.

(199) The suppressing agent according to (198), wherein the indoleamine 2,3-dioxygenase inhibitor is Compound (I) represented by formula (I)

[Chem.31]

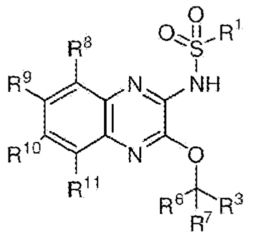

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.32]

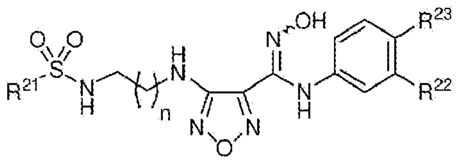

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.
(200) The suppressing agent according to (198) or (199), wherein the antibody which specifically binds to human folate receptor 1 is a monoclonal antibody.
(201) The suppressing agent according to (198) or (199), wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

(202) The suppressing agent according to (198) or (199), wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.
(203) The suppressing agent according to (202), wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

(204) The suppressing agent according to (202), wherein the antibody to a human IL-3Rα chain comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
(205) The suppressing agent according to (198) or (199), wherein the antibody which specifically binds to human TIM-3 is a monoclonal antibody, which binds to an extracellular region of human TIM-3.
(206) The suppressing agent according to (205), wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

(207) The suppressing agent according to (205), wherein the monoclonal antibody is one selected from the group

consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

(208) The suppressing agent according to any one of (198) to (207) wherein the suppressing agent and the antibody are administered simultaneously or sequentially.
(209) The indoleamine 2,3-dioxygenase inhibitor according to (48) or (49), wherein the indoleamine 2,3-dioxygenase inhibitor and the antibody are administered simultaneously or sequentially.
(210) The suppressing agent according to (65) or (66), wherein the suppressing agent and the antibody are administered simultaneously or sequentially.

## Advantageous Effects of Invention

According to the present invention, a therapeutic agent for a tumor comprising an IDO inhibitor administered in combination with an antibody and the like are provided.

## Brief Description of Drawings

**Fig.1**
Fig.1 shows effects of Compound A1 and Compound B1 on kynurenine production and tryptophan consumption. KATO-III cells were treated with 25 ng/mL recombinant human IFN-γ. Cells were treated with Compound A1 (100 nmol/L) or Compound B1 (100 nmol/L). After 3 days, the concentrations of kynurenine (Kyn) and tryptophan (Trp) in the supernatants were measured by LC/MS/MS. (Graph A) The vertical axis shows concentration of kynurenine (Kyn). Each column shows the concentration of Kyn in conditioned medium 1 (KATO-III cells), conditioned medium 2 (KATO-III cells + IFN-γ), conditioned medium 3 (KATO-III cells + IFN-γ + Compound A1), or conditioned medium 4 (KATO-III cells + IFN-γ + Compound B1) from the left. Each column represents the mean + SD. (Graph B) The vertical axis shows concentration of tryptophan (Trp). Each column shows the concentration of Trp in conditioned medium 1 (KATO-III cells), conditioned medium 2 (KATO-III cells + IFN-γ), conditioned medium 3 (KATO-III cells + IFN-γ + Compound A1), or conditioned medium 4 (KATO-III cells + IFN-γ + Compound B1) from the left. Each column represents the mean + SD.
**Fig.2**
Fig.2 shows detection of NK cells. Human PBMCs incubated in (A) conditioned medium 1, (B) conditioned medium 2, (C) conditioned medium 3 or (D) conditioned medium 4 for 7 days. A number in A, B, C and D of Fig. 2 represents $CD16^+$ $CD56^+$ cells in $CD3^-$ cells (NK cells).
**Fig.3**
Fig.3 shows antibody dependent cellular cytotoxicity of mogamulizumab against TL-Om1 cells (A, B) or HH cells (C) by human peripheral blood mononuclear cells (A: donor 1, B, C: donor 2) pre-incubated in various conditioned mediums. All experiments were performed in triplicates and the percentage of cytotoxicity is presented as the mean. The vertical axes of graph A, B, and C show the percentage of cytotoxicity of mogamulizumab, and the horizontal axes of graph A, B, and C show the concentration of mogamulizumab (0, 0.1, 1, and 10 μg/mL from the left). White circles represent the percentage of cytotoxicity of pre-incubated in KATO-III. White triangle marks represent the percentage of cytotoxicity of pre-incubated in KATO-III and IFN-γ. Black circles represent the percentage of cytotoxicity of pre-incubated in KATO-III, IFN-γ and Compound A1. Black triangle marks represent the percentage of cytotoxicity of pre-incubated in KATO-III, IFN-γ and Compound B1.
**Fig.4**
Fig.4 shows antibody dependent cellular cytotoxicity of trastuzumab against SK-BR3 cells by human peripheral blood mononuclear cells (A: donor 1, B: donor 2) pre-incubated in various conditioned mediums. All experiments were performed in triplicates and the percentage of cytotoxicity is presented as the mean. The vertical axes of graphs A and B show the percentage of cytotoxicity of trastuzumab, and the horizontal axes of graph A and B show the concentration of trastuzumab (0, 0.1, 1, and 10 μg/mL from the left). White circles represent the percentage of cytotoxicity of pre-incubated in KATO-III. White triangle marks represent the percentage of cytotoxicity of pre-incubated in KATO-III and IFN-γ. Black circles represent the percentage of cytotoxicity of pre-incubated in KATO-III, IFN-γ and Compound A1. Black triangle marks represent the percentage of cytotoxicity of pre-incubated in KATO-III, IFN-γ and Compound B1.

**Fig.5**

Fig.5 shows antibody dependent cellular cytotoxicity of rituximab against Raji cells by human peripheral blood mononuclear cells pre-incubated in various conditioned mediums. All experiments were performed in triplicates and the percentage of cytotoxicity is presented as the mean. The vertical axis of the graph shows the percentage of cytotoxicity of rituximab, and the horizontal axis of the graph shows the concentration of rituximab (0, 0.1, 1, and 10 μg/mL from the left). White circles represent the percentage of cytotoxicity of pre-incubated in KATO-III. White triangle marks represent the percentage of cytotoxicity of pre-incubated in KATO-III and IFN-γ. Black circles represent the percentage of cytotoxicity of pre-incubated in KATO-III, IFN-γ and Compound A1. Black triangle marks represent the percentage of cytotoxicity of pre-incubated in KATO-III, IFN-γ and Compound B1.

**Fig.6**

Fig.6 shows antibody dependent cellular cytotoxicity of cetuximab against A431 cells by human peripheral blood mononuclear cells pre-incubated in various conditioned mediums. All experiments were performed in triplicates and the percentage of cytotoxicity is presented as the mean. The vertical axis of the graph shows the percentage of cytotoxicity of cetuximab, and the horizontal axis of the graph shows the concentration of cetuximab (0, 0.1, 1, and 10 μg/mL from the left). White circles represent the percentage of cytotoxicity of pre-incubated in KATO-III. White triangle marks represent the percentage of cytotoxicity of pre-incubated in KATO-III and IFN-γ. Black circles represent the percentage of cytotoxicity of pre-incubated in KATO-III, IFN-γ and Compound A1. Black triangle marks represent the percentage of cytotoxicity of pre-incubated in KATO-III, IFN-γ and Compound B1.

**Fig.7**

Fig.7 shows antibody dependent cellular cytotoxicity of mogamulizumab against TL-Om1 cells by human NK cells pre-incubated in various conditioned mediums. All experiments were performed in triplicates and the percentage of cytotoxicity is presented as the mean. The vertical axis of the graph shows the percentage of cytotoxicity of mogamulizumab and the horizontal axis of the graph shows the concentration of mogamulizumab (0, 0.1, 1, and 10 μg/mL from the left). White circles represent the percentage of cytotoxicity of pre-incubated in KATO-III. White triangle marks represent the percentage of cytotoxicity of pre-incubated in KATO-III and IFN-γ. Black circles represent the percentage of cytotoxicity of pre-incubated in KATO-III, IFN-γ and Compound A1. Black triangle marks represent the percentage of cytotoxicity of pre-incubated in KATO-III, IFN-γ and Compound B1.

**Fig.8**

Fig.8 shows antibody dependent cellular cytotoxicity of rituximab against Raji cells by human NK cells pre-incubated in various conditioned mediums. All experiments were performed in triplicates and the percentage of cytotoxicity is presented as the mean. The vertical axis of the graph shows the percentage of cytotoxicity of rituximab, and the horizontal axis of the graph shows the concentration of rituximab (0, 0.1, 1, and 10 μg/mL from the left). White circles represent the percentage of cytotoxicity of pre-incubated in KATO-III. White triangle marks represent the percentage of cytotoxicity of pre-incubated in KATO-III and IFN-γ. Black circles represent the percentage of cytotoxicity of pre-incubated in KATO-III, IFN-γ and Compound A1. Black triangle marks represent the percentage of cytotoxicity of pre-incubated in KATO-III, IFN-γ and Compound B1.

**Fig.9**

Fig.9 shows antibody dependent cellular cytotoxicity of mogamulizumab against TL-Om1 cells by human peripheral blood mononuclear cells pre-incubated in medium containing Compound A1 or Compound B1. All experiments were performed in triplicates and the percentage of cytotoxicity is presented as the mean. The vertical axis of the graph shows the percentage of cytotoxicity of mogamulizumab, and the horizontal axis of the graph shows the concentration of mogamulizumab (0, 0.1, 1, and 10 μg/mL from the left). White circles represent the percentage of cytotoxicity of pre-incubated in medium. White triangle marks represent the percentage of cytotoxicity of pre-incubated in medium and Compound A1. White rectangle marks represent the percentage of cytotoxicity of pre-incubated in medium and Compound B1.

**Fig.10**

Fig.10 shows IDO expression in KATO-III cells stably expressing IDOl shRNA. (Graph A) The vertical axis of the graph A shows IDOl mRNA expression level treated with IFN-γ relative to parent KATO-III cells detected by qPCR analysis. Each column shows IDO1 mRNA level in KATO-III cells introduced with IDOl shRNA #44, #45, #46, #47, vector, NegaCTRL shRNA or none(parent) from the left. (Graph B) The upper part of graph B shows IDOl protein expression level detected by western blot analysis, and the lower part of graph B shows parent(β-actin) protein expression level detected by western blot analysis.

**Fig.11**

Fig.11 shows effects of IDO1 knockdown on kynurenine production and tryptophan consumption. (Graph A) The vertical axis of the graph A shows concentration (μmol/mL)of kynurenine (Kyn). Each column shows the Kyn concentration in conditioned medium 1 [KATO-III cells (parent)], conditioned medium 2 [KATO-III cells (NegaCTRL shRNA), conditioned medium 3 [KATO-III cells (parent)+ IFN-γ), conditioned medium 4 [KATO-III cells (NegaCTRL shRNA) + IFN-γ], conditioned medium 5 [KATO-III cells (IDOl shRNA #44) + IFN-γ],conditioned medium 6 [KATO-III cells (IDOl shRNA #45) + IFN-γ], or conditioned medium 7 [KATO-III cells (IDOl shRNA #46) + IFN-γ], from the left. (Graph B) The vertical axis of the graph shows concentration (μmol/mL)of tryptophan (Trp). Each column shows the Trp concentration in conditioned medium 1 [KATO-III cells (parent)], conditioned medium 2 [KATO-III cells (NegaCTRL shRNA), conditioned medium 3 [KATO-III cells (parent)+ IFN-γ), conditioned medium 4 [KATO-III cells (NegaCTRL shRNA) + IFN-γ], conditioned medium 5 [KATO-III cells (IDO1 shRNA #44) + IFN-γ], conditioned medium 6 [KATO-III cells (IDOl shRNA #45) + IFN-γ], or conditioned medium 7 [KATO-III cells (IDOl shRNA #46) + IFN-γ], from the left. Each column represents the mean + SD.

**Fig.12**

Fig.12 shows detection of NK cells. Human PBMCs were incubated in conditioned medium 1 (A), conditioned medium 2 (B), conditioned medium 3 (C), conditioned medium 4 (D), conditioned medium 5 (E), conditioned medium 6 (F) or conditioned medium 7 (G) for 7 days. A number in A, B, C, D, E, F and G represents $CD16^+$ $CD56^+$ cells in $CD3^-$ cells (NK cells).

**Fig.13**

Fig.13 shows antibody dependent cellular cytotoxicity of mogamulizumab against TL-Om1 cells by human peripheral blood mononuclear cells pre-incubated in various conditioned medium. All experiments were performed in triplicates and the percentage of cytotoxicity is presented as the mean. The vertical axis of the graph shows the percentage of cytotoxicity of mogamulizumab, and horizontal axis of the graph shows the concentration of mogamulizumab (0, 0.1, 1, and 10 μg/mL from the left). White circles represent the percentage of cytotoxicity of pre-incubated in parental KATO-III cells. White triangle marks represent the percentage of cytotoxicity of pre-incubated in KATO-III cells stably expressing negative control shRNA. White rectangle marks represent the percentage of cytotoxicity of pre-incubated in parental KATO-III cells and IFN-γ. White diamond marks represent the percentage of cytotoxicity of pre-incubated in KATO-III cells stably expressing negative control shRNA and IFN-γ. Black circles represent the percentage of cytotoxicity of pre-incubated in KATO-III cells stably expressing IDOl shRNA#44 and IFN-γ. Black triangle marks represent the percentage of cytotoxicity of pre-incubated in KATO-III cells stably expressing IDOl shRNA#45 and IFN-γ. Black rectangle marks represent the percentage of cytotoxicity of pre-incubated in KATO-III cells stably expressing IDO1 shRNA#46 and IFN-γ.

**Fig.14**

Fig.14 shows expression of IDO1 in ovarian cancer cells (OVISE, SKOV3, OVCAR3, and MCAS) detected by Western blot.

**Fig.15**

Fig.15 shows effect of Compound A1 on natural killing activity (A) and cytotoxicity (B) of PBMCs against SKOV3 cells. All experiments were performed in triplicates and the percentage of cytotoxicity is presented as the mean + SD. (Graph A) The vertical axis of the graph A shows the percentage of the cytotoxicity without Antibody C1 (the natural killing activity) of PBMCs, and each column shows the cytotoxicity in the conditioned medium DMSO or Compound A1 from the left. (Graph B) The vertical axis of the graph shows the percentage of cytotoxicity of the antibody C1, and the horizontal axis of the graph shows the concentration of the antibody C1 (0.033, 0.33, 3.3, 33, and 333 ng/mL from the left). Black circles represent the percentage of cytotoxicity of pre-incubated in the conditioned medium of IFN-γ simulated SKOV3 cells and Compound A1. White circles represent the percentage of cytotoxicity of pre-incubated in the conditioned medium of IFN-γ simulated SKOV3 cells and DMSO.

**Fig.16**

Fig.16 shows effect of Compound B1 on natural killing activity (A) and cytotoxicity (B) of PBMCs against SKOV3 cells. All experiments were performed in triplicates and the percentage of cytotoxicity is presented as the mean + SD. (Graph A) The vertical axis of the graph A shows the percentage of the cytotoxicity without Antibody C1 (the natural killing activity) of PBMCs, and each column shows the cytotoxicity in the conditioned medium DMSO or Compound B1 from the left. (Graph B) The vertical axis of the graph shows the percentage of cytotoxicity of the antibody C1, and the horizontal axis of the graph shows the concentration of the antibody C1 (0.033, 0.33, 3.3, 33, and 333 ng/mL from the left). Black circles represent the percentage of cytotoxicity of pre-incubated in the conditioned medium of IFN-γ simulated SKOV3 cells and Compound B1. White circles represent the percentage of cytotoxicity of pre-incubated in the conditioned medium of IFN-γ simulated SKOV3 cells and DMSO.

**Fig.17**

Fig.17 shows effect of Compound A1 on the cytotoxicity of PBMCs against KG-1 cells (lot# A3951). All experiments were performed in triplicates and the percentage of cytotoxicity is presented as the mean +/- SD. The vertical axis of the graph shows the cytotoxicity of PBMCs by Compound A1, and the horizontal axis of the graph shows the concentration of the antibody D1 (0.3, 3, 30, and 300 μg/mL from the left). Black circles represent the percentage of cytotoxicity of pre-incubated in the conditioned medium of IFN-γ stimulated KATO-III cells and Compound A1.

White circles represent the percentage of cytotoxicity of pre-incubated in the conditioned medium of IFN-γ stimulated KATO-III cells and DMSO.

**Fig.18**

Fig.18 shows effect of Compound A1 on the cytotoxicity of PBMCs against EoL-1/human TIM-3 cells. All experiments were performed in triplicates and the percentage of cytotoxicity is presented as the mean +/- SD. The vertical axis of the graph shows the cytotoxicity of PBMCs by Compound A1, and the horizontal axis of the graph shows the concentration of the antibody E1 (0.3, 3, 30, and 300 μg/mL from the left). Black circles represent the percentage of cytotoxicity of pre-incubated in the conditioned medium of IFN-γ stimulated KATO-III cells and Compound A1. White circles represent the percentage of cytotoxicity of pre-incubated in the conditioned medium of IFN-γ stimulated KATO-III cells and DMSO.

## Description of Embodiments

In the present invention, the ADCC activity is a cytotoxic activity in which an antibody bound to a cell surface antigen on a tumor cell in the living body activate an effector cell through an Fc receptor existing on the antibody Fc region and effector cell surface and thereby obstruct the tumor cell and the like [Monoclonal Antibodies: Principles and Applications, Wiley-Liss, Inc., Chapter 2.1 (1955)]. Examples of the effector cell include a killer cell, a natural killer cell, an activated macrophage and the like.

Examples of the antibody having ADCC activity include mogamulizumab, trastuzumab, rituximab, cetuximab, and the like.

An ADCC activity of the antibody which specifically binds to human CC chemokine receptor 4, human epidermal growth factor receptor 2, human CD20, or epidermal growth factor receptor, and ADCC activity of the antibody which specifically binds to human CC chemokine receptor 4 (CCR4), HER2, human CD20, or EGFR in the present invention in combination with an indoleamine 2,3-dioxygenase inhibitor can be examined according to the method described in "Clin. Cancer Res. vol. 11, p5984, 2005.", "Br. J. Hematol. vol. 144, p848, 2009." and the like.

The prognosis with T-cell lymphoma is very poor and there is no therapeutic agent which exhibits sufficient drug efficacy. It is known that human CC chemokine receptor 4 (CCR4) is expressed on some kinds of T-cell lymphoma including adult T-cell leukemia/lymphoma and cutaneous T cell lymphoma (Clinical Cancer Research, vol. 9, p3625, 2003 and J Invest Dermatol, vol.119, p1405, 2002). Therefore, a pharmaceutical composition comprising an antibody composition which specifically binds to CCR4 can be a pharmaceutical composition effective for treating T-cell tumors which expresses CCR4 (WO01/64754, WO03/18635, and WO2005/057341).

Examples of the antibody which specifically binds to CCR4 is mogamulizumab and the like. Mogamulizumab is a humanized anti CCR4 monoclonal antibody and is used for the treatment of leukemia and lymphoma such as ATL, peripheral T cell lymphoma, cutaneous T cell lymphoma. And also mogamulizumab has high ADCC activity.

Mogamulizumab is known to induce reduction of regulatory T cells [Clin Cancer Res; 21(2) January 15, 2015]. The tryptophan metabolism also induces the conversion of $CD4^{+}CD25^{-}$ T cells into regulatory T cells (for example, Blood, vol. 109, No. 7, pp. 2871-2877 (2007)). Therefore, the combination of mogamulizumab and an indoleamine 2,3-dioxygenase inhibitor is thought to be preferable for reduction of regulatory T cells.

Human epidermal growth factor receptor 2 (HER2 or pl85neu), was originally identified as the product of the transforming gene from neuroblastomas of chemically treated rats. The activated form of the neu proto-oncogene results from a point mutation (valine to glutamic acid) in the transmembrane region of the encoded protein. Amplification of the human homologue of neu, which is HER2, is observed in breast and ovarian cancers and correlates with a poor prognosis (Slamon et al., Science, 235: 177-182 (1987); Slamon et al., Science, 244: 707-7 12 (1989); US 4,968, 603). HER2 has a molecular weight of about 185.000, with considerable homology to the epidermal growth factor receptor (EGFR or HER1), although a specific ligand for HER2 has not yet been clearly identified so far.

The antibody 4D5 directed to the HER2 receptor, was further found to sensitize HER2-overexpressing breast tumor cell lines to the cytotoxic effects of TNFα (US 5,677, 171). A recombinant humanized version of the murine anti-ErbB2 antibody 4D5 (huMAb4D5-8, rhuMAb HER2 or trastuzumab; US 5,821, 337) is clinically active in patients with HER2-overexpressing metastatic breast cancers that have received extensive prior anti-cancer therapy (Baselga et al., J. Clin. Oncol. 14: 737-744 (1996)). Trastuzumab received marketing approval in 1998 for the treatment of patients with metastatic breast cancer whose tumors overexpress the HER2 protein. One representative showing high efficacy in clinical trials is gefitinib which can be applied for NSCLC indication (non-small cell lung cancer).

Trastuzumab exerts an antitumor effect by specifically binding to HER2 protein and is used to treat breast cancer, gastric cancer and the like. Also, trastuzumab exerts an antitumor effect because of high ADCC activity.

Lymphocytes are one of many types of white blood cells produced in the bone marrow during the process of hematopoiesis. There are two major populations of lymphocytes: B lymphocytes (B cells) and T lymphocytes (T cells). The lymphocytes of particular interest herein are B cells.

B cells mature within the bone marrow and leave the marrow expressing an antigen-binding antibody on their

cell surface. When a naive B cell first encounters the antigen for which its membranebound antibody is specific, the cell begins to divide rapidly and its progeny differentiate into memory B cells and effector cells called "plasma cells". Memory B cells have a longer life span and continue to express membrane-bound antibody with the same specificity as the original parent cell.

Plasma cells do not produce membrane-bound antibody but instead produce the antibody in a form that can be secreted.

Secreted antibodies are the major effector molecule of humoral immunity.

The CD20 antigen (also called human B-lymphocyte-restricted differentiation antigen, Bp35) is a hydrophobic transmembrane protein with a molecular weight of approximately 35 kD located on pre-B and mature B lymphocytes (Valentine et al. J. Biol. Chem. 264 (19): 11282-11287 (1989); and Einfeld et al. EMBO J. 7 (3): 711-717 (1988)). The antigen is also expressed on greater than 90% of B cell non Hodgkin's lymphomas (NHL) (Anderson et al. Blood 63 (6): 1424-1433 (1984)), but is not found on hematopoietic stem cells, pro-B cells, normal plasma cells or other normal tissues (Tedder et al. J. Immunol. 135 (2): 973-979 (1985)). CD20 regulates an early step(s) in the activation process for cell cycle initiation and differentiation (Tedder et al., supra) and possibly functions as a calcium ion channel (Tedder et al. J. Cell. Biochem. 14D: 195 (1990)).

Examples of the antibody specifically binding to human CD20 include rituximab.

Rituximab is a monoclonal antibody comprising anti human CD20 chimeric human-murine antibody and is used for treating non-Hodgkin's lymphoma, B cell lymphopro-liferative disorder, granulomatosis with polyangiitis, microscopic polyangiitis, and the like. And also, rituximab has high ADCC activity.

EGFR is a 170 kilodalton (kDa) membrane-bound protein expressed on the surface of epithelial cells. EGFR is a member of the growth factor receptor family of protein tyrosine kinases, a class of cell cycle regulatory molecules (W. J. Gullick et al., 1986, Cancer Res. , 46: 285-292).

EGFR is activated when its ligand (either EGF or TGF-$\alpha$) binds to the extracellular domain, resulting in autophosphorylation of the receptor's intracellular tyrosine kinase domain (S. Cohen et al., 1980, J. Biol. Chem., 255: 4834-4842; A. B. Schreiber et al., 1983, J. Biol. Chem., 258: 846-853).

EGFR is the protein product of a growth promoting oncogene, erbB or ErbB1 that is but one member of a family, i. e., the ERBB family of protooncogenes, believed to play pivotal roles in the development and progression of many human cancers. In particular, increased expression of EGFR has been observed in breast, bladder, lung, head, neck and stomach cancer as well as glioblastomas. The ERBB family of oncogenes encodes four, structurally-related transmembrane receptors, namely, EGFR, HER-2/neu (erbB2), HER-3 (erbB3) and HER-4 (erbB4).

Clinically, ERBB oncogene amplification and/or receptor overexpression in tumors have been reported to correlate with disease recurrence and poor patient prognosis, as well as with responsiveness in therapy. (L. Harris et al., 1999, Int. J. Biol. Markers, 14: 8-15; and J. Mendelsohn and J. Baselga, 2000, Oncogene, 19: 6550-6565).

EGFR is composed of three principal domains, namely, the extracellular domain (ECD), which is glycosylated and contains the ligand-binding pocket with two cysteine-rich regions; a short transmembrane domain, and an intracellular domain that has intrinsic tyrosine kinase activity. The transmembrane region joins the ligand-binding domain to the intracellular domain. Amino acid and DNA sequence analysis, as well as studies of nonglycosylated forms of EGFR, indicate that the protein backbone of EGFR has a mass of 132 kDa, with 1186 amino acid residues (A. L. Ullrich et al., 1984, Nature, 307: 418-425; J. Downward et al., 1984, Nature, 307: 521-527; C. R. Carlin et al., 1986, Mol. Cell. Biol., 6: 257-264; and F. L. V. Mayes and M. D. Waterfield, 1984, The EMBO J., 3: 531-537).

The binding of EGF or TGF-$\alpha$ to EGFR activates a signal transduction pathway and results in cell proliferation. The dimerization, conformational changes and internalization of EGFR molecules function to transmit intracellular signals leading to cell growth regulation (G. Carpenter and S. Cohen, 1979, Ann. Rev. Biochem., 48: 193-216). Genetic alterations that affect the regulation of growth factor receptor function, or lead to overexpression of receptor and/or ligand, result in cell proliferation. In addition, EGFR has been determined to play a role in cell differentiation, enhancement of cell motility, protein secretion, neovascularization, invasion, metastasis and resistance of cancer cells to chemotherapeutic agents and radiation. (M. J. Oh et al., 2000, Clin. Cancer Res., 6: 4760-4763).

Examples of the antibody specifically binding to EGFR include cetuximab, and the like. Cetuximab is a monoclonal antibody which binds to EGFR and inhibits the function of EGFR.

Cetuximab is used in the treatment of colon cancer, head and neck cancer, and the like. Cetuximab is also expected to exhibit an antitumor effect because of ADCC activity.

Rituximab is a monoclonal antibody containing anti human CD20 chimeric human-murine antibody and is used for treating non-Hodgkin's lymphoma, B cell lymphopro-liferative disorder, granulomatosis with polyangiitis, microscopic polyangiitis, and the like. And also, rituximab has high ADCC activity.

Human folate receptor 1(FOLR1) is a GPI-anchored membrane protein having a high affinity for folate, and has an important functions relating to cell proliferation or survival [Int J Cancer, 2006. 119(2): p. 243-50.]. FOLR1 shows restricted-expression pattern in the normal tissues of the kidney, lung, intestine or the like [Anal Biochem, 2005. 338(2): p. 284-93.].

The expression region is localized in the lumen. Meanwhile, FOLR1 expression in cancer tissues is not restricted

to the lumen, and its high expression is observed in a variety of cancers such as ovarian cancer [Anal Biochem, 2005. 338(2): p. 284-93., J Thorac Oncol, 2012. 7(5): p. 833-840., J Thorac Cardiovasc Surg, 2001. 121(2): p. 225-33.], renal cancer [Anal Biochem, 2005. 338(2): p. 284-93.], lung cancer [Anal Biochem, 2005. 338(2): p. 284-93., J Thorac Oncol, 2012. 7(5): p. 833-840.], breast cancer [Anal Biochem, 2005. 338(2): p. 284-93.], mesothelioma [J Thorac Cardiovasc Surg, 2001. 121(2): p. 225-33.].

In particular, it was reported that FOLR1 expression level is related to malignancy grade, progression, and prognosis in ovarian cancer [Int J Cancer, 1997. 74(2): p. 193-8., Int J Cancer, 1998. 79(2): p. 121-6.]. Furthermore, it was also reported that soluble FOLR1 was significantly elevated in the serum of ovarian cancer patients compared to the serum of healthy donors [PLoS One, 2009. 4(7): p.e6292.]. Thus, FOLR1 is a promising target molecule for cancer treatment.

IL-3Rα is the α chain of IL-3 receptor, belongs to a cytokine receptor family and shows weak affinity for IL-3 as its ligand. By forming a hetero receptor with its β chain (CD131, hereinafter also referred to as IL-3Rβ), an IL-3 receptor has a strong binding and transfers a signal such as growth, differentiation and the like into a cell through intracellular region of the β chain. IL-5 receptor α chain and GM-CSF receptor α chain share the β chain in common.

IL-3Rα is a type I membrane protein of single-pass transmembrane, and it is known based on the sequence that an IL-3 binding site and a fibronectin type III site are present in the extramembrane region. It is known that there is no structure which can transfer a signal in the intramembrane region. Though three-dimensional structure of IL-3Rα has not been analyzed yet, it can be assumed that structures of cytokine receptors are similar between families since position of cysteine residue which forms the structurally important S-S bond is preserved in most cases. Among the same cytokine receptors, crystalline structures of IL-13 receptor α chain, IL-4 receptor α chain and GM-CSF receptor α chain have been analyzed. Based on the information of these cytokine receptor families, it can be assumed that the extramembrane region of IL-3Rα is roughly divided into 3 domains (A-B-C domains). It is known that an antibody 7G3 which recognizes A domain of human IL-3Rα blocks IL-3 signaling [Sun et al., Blood, 87:83 (1996)]. In addition, expression of an A domain-deficient IL-3Rα molecule has been reported [Chen et al., J Biol Chem, 284: 5763(2009)], and as a matter of course, an antibody which recognizes A domain cannot recognize A domain-deficient IL-3Rα. In addition, it is considered that C domain is the root of IL-3Rα molecule and has a high possibility to three-dimensionally inhibit association of IL-3Rβ with IL-3Rα.

IL-3 is the only a ligand which is known as a ligand of IL-3Rα. IL-3 is a hematopoietic factor which is known to accelerate colony formation of the following: erythrocyte, megakaryocyte, neutrophil, eosinophil, basophil, mast cell and a monocyte system cell. It is known that IL-3 also stimulates a precursor cell having pluripotency, but IL-3 is rather said to accelerate a differentiation of not an immature stem cell having autonomous replication ability but a precursor cell committed to differentiation.

It is known that IL-3Rα relates to the growth and differentiation of myeloid cells by forming a heterodimer with β chain and thereby transferring the IL-3 signaling into the cell via the Serine/Threonine phosphorylation pathway. It is known that IL-3Rα is expressed in Granulocyte-Macrophage Progenitor (GMP) or Common Myeloid Progenitor (CMP) among hematopoietic precursor cells and induces growth and differentiation into neutrophil and macrophage systems via the IL-3 signaling. On the other hand, it has been reported that the Megakaryocyte Erythroid Progenitor (MEP) presenting in the downstream of CMP does not express IL-3Rα different from the GMP which is also present in the downstream.

Regarding the AML stem cell, Bonnet and Dick have reported that the AML stem cell is present in the CD34 positive CD38 negative fraction (Bonnet et al., Nat Med, 1997; 3: 730). Further, by comparing with the same fraction (CD34 positive CD38 negative) of normal stem cell, Jordan et al. have found that IL-3Rα is highly expressed in the AML stem cell (Jordan et al., Leukemia, 2000; 14: 1777). A high potential of IL-3Rα as a marker of not only AML stem cell but also leukemia stem cell has also been reported in the plural of reports thereafter (Haematologica, 2001; 86:1261, and Leuk-Lymphoma, 2006;47:207). In the treatment of cancers including leukemia, it is important that only the cancer cells are removed without injuring normal cells as many as possible, and it is considered that this difference in the expression of IL-3Rα between normal stem cell and leukemia stem cell is useful in the treatment targeting at the leukemia stem cell.

Regarding IL-3Rβ which forms a heterodimer with IL-3Rα, there is no report that IL-3Rβ is highly expressed leukemia stem cell, and also in the case of a microarray in which expression of mRNA in leukemia stem cell and normal stem cell is compared in fact, IL-3Rβ is not identified as a molecule in which its expression is increased in leukemia stem cell (Majeti et al., Proc Natl Acad Sci USA. 2009; 106:3396).

The presence of a leukemia cell which depends on IL-3 has been known for a long time, and the old studies are studies focused on a blast cell which occupies most of the leukemia cells. According to the recent studies on leukemia stem cell, it is said that the leukemia stem cell acquires antitumor agent resistance by exhaustively suppressing its growth. In addition, it is considered that an IL-3 reactive blast cell has high proliferation ability so that it is assumed that such a cell is effective in the general treatment using an antitumor agent.

As a candidate of the agent targeting at an IL-3R receptor, the IL-3 itself was administered for a long time to

patients of hematopoietic insufficiency but it did not become a drug as a result. A clinical trial for a fusion protein in which diphtheria toxin is added to IL-3 is in progress aiming leukemia as a target of the disease. Regarding the IL-3 and diphtheria toxin-IL-3 fusion, these are not suitable as the agents which are targeting at cells in which expression of IL-3Rα is specifically increased, since IL-3 binds strongly not a protein of IL-3Rα alone but a hetero protein of IL-3Rα and β due to properties of IL-3. On the other hand, as a candidate of an agent targeting at IL-3Rα, a first phase result of an IL-3Rα human mouse chimeric antibody 7G3 has been reported (Non-patent Document 19). Since the 7G3 chimeric antibody uses for the purpose of blocking of IL-3 signaling as the mechanism of AML therapy, this is not an agent aimed at removing IL-3Rα positive cells. Also, although some other IL-3Rα antibodies are known (9F5 (Becton Dickinson), 6H6 (SANTA CRUZ BIOTECHNOLOGY) and AC 145 (Miltenyi-Biotech)), these do not have the ability to remove the cells highly expressing IL-3Rα.

However, an monoclonal antibody having the ability to remove the cells highly expressing IL-3Rα has been reported(WO2010/126066).

TIM-3 gene family consists of eight genes in mouse and three genes in human, and each of these genes are located at chromosome 11 and at chromosome 5q33 respectively [Hafler DA et al., JExp Med. 205: 2699-701 (2008)]. These gene regions are known to be related with autoimmune diseases and allergic diseases. TIM protein is a type I transmembrane protein having a structurally conserved immunoglobulin variable (IgV) domain and a mucin domain.

TIM protein was considered to be specifically expressed on T cells and directly regulate the T cell activity, but there are recent reports on expression of TIM-3 protein in antigen-presenting cells and on their functions [Anderson AC et al., Science 318: 1141-3 (2007)]. According to the crystal structure analysis, the TIM protein has a conserved protein structure and has a ligand binding site in the IgV domain.

TIM-3 was identified as a molecule specifically expressed on mouse Th1 cells but not on Th2 cells [Monney L et al., Nature 415: 536-41 (2002)]. The DNA sequence, the amino acid sequence and the three-dimensional structure of TIM-3 is available in the public data base such as the GenBank accession number NM_032782 and NM_134250. TIM-3 is also known as HAVCR2.

In humans, as similar to mice, TIM-3 is expressed on T-cells as well as phagocytic cells such as macrophages and dendritic cells. Binding of TIM-3 to a protein ligand (e.g., galectin-9) can inhibit the Th1 response via mechanism of apoptosis induction, and therefore lead to such as induction of peripheral tolerance.

The reduction in expression of human TIM-3 with siRNA or the inhibition of human TIM-3 by blocking-antibody increased the secretion of interferon γ (IFN-γ) from CD4 positive T-cells, supporting the inhibitory role of TIM-3 in human T cells. In phagocytes, TIM-3 also functions as a receptor for recognizing the apoptosis cells.

Analysis of clinical samples from autoimmune disease patients showed no expression of TIM-3 in CD4 positive cells. In particular, in T cell clones derived from the cerebrospinal fluid of patients with multiple sclerosis, the expression level of TIM-3 was lower and the secretion level of IFN-γ was higher than those of clones derived from normal healthy persons [Koguchi K et al., J Exp Med. 203: 1413-8 (2006)]. There are reports on relation of TIM-3 with allergic diseases or asthma (WO96/27603 and WO2003/063792).

According to the microarray analysis of hematopoietic stem cells from acute myeloid leukemia (hereinafter referred to as "AML") patients and normal hematopoietic stem cells, TIM-3 is expressed on AML stem cells and therefore the analysis suggested involvement of TIM-3 in hematological malignancy [Majeti R et al., Proc Natl Acad Sci USA 2009 Mar 3; 106 (9): 3396-401. and WO2009/091547].

Examples of the anti-TIM-3 monoclonal antibodies which were established up to now include anti-human TIM-3 rat monoclonal antibody (Clone 344823, manufactured by R&D Systems), anti-human TIM-3 mouse monoclonal antibody (Clone F38-2E2, manufactured by R&D Systems), and anti-human TIM-3 mouse monoclonal antibody having ADCC activity (512 antibody, 644 antibody, 4545 antibody and 4177 antibody in WO2011/155607).

Hereinafter, the compound represented by the above formula (I) or (II) is referred to as Compound (I) or (II), respectively. The same applies to the other compounds having different formula numbers.

The definitions of the respective groups in the formula (I) and (II) are as follows.

(i) Examples of the lower alkyl and the lower alkyl moiety of the lower alkoxy include linear or branched alkyl having 1 to 10 carbon atoms. More specific examples thereof include methyl, ethyl, n-propyl, isopropyl, n-butyl, isobutyl, sec-butyl, tert-butyl, n-pentyl, neopentyl, n-hexyl, n-heptyl, n-octyl, n-nonyl, n-decyl, and the like.

(ii) Halogen means each atom of a fluorine atom, a chlorine atom, a bromine atom, and an iodine atom.

(iii) Examples of the aromatic heterocyclic group include a 5- or 6-membered monocyclic aromatic heterocyclic group which contains at least one heteroatom selected from a nitrogen atom, an oxygen atom and a sulfur atom; a bicyclic aromatic heterocyclic group in which 3- to 8-membered rings are fused and which contains at least one heteroatom selected from a nitrogen atom, an oxygen atom and a sulfur atom; a tricyclic aromatic heterocyclic group in which 3- to 8-membered rings are fused and which contains at least one heteroatom selected from a nitrogen atom, an oxygen atom and a sulfur atom; and the like. More specific examples thereof include furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl,

pyridyl-1-oxide, pyridazinyl, pyrimidinyl, pyrazinyl, triazinyl, benzofuranyl, benzothiophenyl, benzoxazolyl, benzoxadiazolyl benzothiazolyl, isoindolyl, indolyl, indazolyl, benzimidazolyl, benzotriazolyl, oxazolopyrimidinyl, thiazolopyrimidinyl, pyrrolopyridyl, pyrrolopyrimidinyl, imidazopyridyl, imidazopyrimidinyl, triazolopyridyl, triazolopyrimidinyl, purinyl, quinolinyl, isoquinolinyl, cinnolinyl, phthalazinyl, quinazolinyl, quinoxalinyl, naphthyridinyl, and the like. Among these, preferred as the bicyclic aromatic heterocyclic groups are benzofuranyl, benzothiophenyl, benzoxazolyl, benzoxadiazolyl, benzothiazolyl, isoindolyl, indolyl, indazolyl, benzimidazolyl, benzotriazolyl, oxazolopyrimidinyl, thiazolopyrimidinyl, pyrrolopyridyl, pyrrolopyrimidinyl, imidazopyridyl, imidazopyrimidinyl, triazolopyridyl, triazolopyrimidinyl, purinyl, quinolinyl, isoquinolinyl, cinnolinyl, phthalazinyl, quinazolinyl, quinoxalinyl, naphthyridinyl, and the like.

(iv) The substituents of the optionally substituted lower alkyl may be the same or different and the number of the substituents of these groups is from 1 to the highest possible number of substitution, preferably 1 to 3, and examples of the substituents include a substituent selected from the group consisting of halogen, hydroxy, cyano, carboxy, carbamoyl, and the like.

(v) The substituents of the optionally substituted aromatic heterocyclic group may be the same or different and the number of the substituents of these groups is from 1 to the highest possible number of substitution, preferably 1 to 3, and examples of the substituents include a substituent selected from the group consisting of halogen, hydroxy, cyano, carboxy, carbamoyl, optionally substituted aryl (examples of the substituents of the optionally substituted aryl include halogen, hydroxy, cyano, carboxy, carbamoyl, $C_{1-10}$ alkyl, and the like), an optionally substituted aromatic heterocyclic group (examples of the substituents of the optionally substituted heterocyclic group include halogen, hydroxy, cyano, carboxy, carbamoyl, $C_{1-10}$ alkyl, and the like).

In the groups exemplified in the above (iv) and (v), examples of the aryl include monocyclic aryl and fused aryl in which two or more rings are fused. More specific examples thereof include aryl having 6 to 14 ring carbon atoms, such as phenyl, naphthyl, indenyl, and anthranil. $C_{1-10}$ alkyl has the same meaning as defined in the above lower alkyl. Halogen has the same meaning as defined in the above halogen. The aromatic heterocyclic group has the same meaning as defined in the above aromatic heterocyclic group.

Examples of the pharmaceutically acceptable salts of Compounds (I) and (II) include pharmaceutically acceptable acid addition salts, metal salts, ammonium salts, organic amine addition salts, amino acid addition salts and the like. The acid addition salts include inorganic acid salts such as hydrochlorides, sulfates and phosphates; and organic acid salts such as acetate, maleate, fumarate, tartrates, citrates, lactates, aspartates, and glutamates. The metal salts include alkali metal salts such as sodium salts and potassium salts; alkaline earth metal salts such as magnesium salts and calcium salts; as well as aluminum salts, zinc salts and the like. The ammonium salts include salts of ammonium, tetramethylammonium and the like. The organic amine addition salts include morpholine salts, piperidine salts and the like. The amino acid addition salts include lysine salts, glycine salts, phenylalanine salts and the like.

When it is desired to obtain salts of Compound (I) and (II), the salt may be purified as it is, if Compound (I) and (II) are obtained in a form of a salt; and if Compound (I) and (II) are obtained in a freeform, it is dissolved or suspended in an appropriate solvent followed by adding an acid or a base thereto to form a salt.

There can be isomers such as positional isomers, geometrical isomers or optical isomers in Compound (I) and (II). All possible isomers including these isomers, and mixtures of the isomers in any ratio can be used as IDO inhibitors of the present invention.

Compound (I) and (II) or the pharmaceutically acceptable salt thereof may exist in a form of adducts to water or various solvents. These adducts can also be used as IDO inhibitors of the present inhibition.

There are concerns that the compound (I), (II) and the antibody having ADCC activity may not give sufficient treatment results in the single administration, and also high-dose administration of the above compound may cause side effects.

By combining the above compound (I) or (II) together with the antibody having ADCC activity, the present invention provides better treatment results than administering either compound, and then any one of the above compound (I) or (II) and the antibody having ADCC activity can be used in a low dosage.

Therefore, the present invention not only provides sufficient effect of treatment but also decreases side effects.

Compounds (I) and (II) or a pharmaceutically acceptable salt thereof used in the present invention can be synthesized based on the methods described in, for example, WO2011/42316 and WO2010/05958.

Examples of the compounds (I) and (II) used in the present invention include Compounds A1 to A5, Compound B1, and the like, respectively.

In the following table A, Me represents methyl.

[Chem.33]

Table A

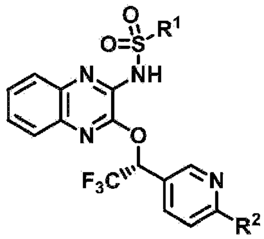

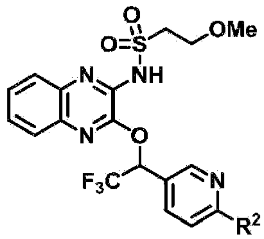

| Compound | $R^1$ | $R^2$ | Compound | $R^2$ |
|---|---|---|---|---|
| A1 | 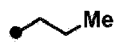 | 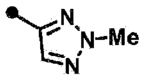 | A4 | 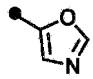 |
| A2 | 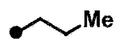 | 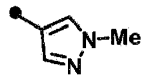 | A5 | 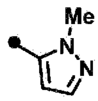 |
| A3 | 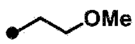 | 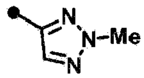 | | |

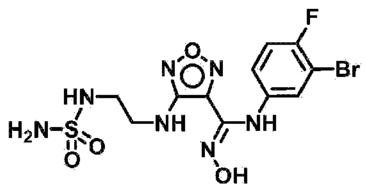

Compound B1

The antibody of the present invention specifically binds to CCR4, HER2, human CD20, EGFR, FOLR1, human IL-3Rα, or human TIM-3.

The ADCC activity in the present invention is a cytolytic reaction in which an antibody bound to CCR4, HER2, human CD20, EGFR, FOLR1, human IL-3Rα or human TIM-3 on the cell surface binds to FcγRIIIa on the surface of mainly natural killer cell (hereinafter, referred to as NK cell) via Fc moiety, and as a result, the reaction is generated by cytotoxic molecules, such as perforin and granzyme, released from the NK cell [Clark M, Chemical Immunology, 65, 88 (1997); Gorter A et al., Immunol. Today, 20, 576 (1999)].

Specific binding of the antibody used in the present invention to CCR4, HER2, human CD20, EGFR, FOLR1, human IL-3Rα, human TIM-3 can be confirmed by a method capable of investigating a particular antigen and a binding of an antibody to the particular antigen such as enzyme-linked immunosorbent assay (ELISA) using a solid-phase CCR4, HER2, human CD20, EGFR, FOLR1, human IL-3Rα or human TIM-3, Western blotting, or immunohistochemistry (IHC), or by a known immunological detection method or a fluorescent cell staining method for CCR4-expressing, HER2-expressing, human CD20-expressing, EGFR-expressing, FOLR1-expressing, human IL-3Rα-expressing or human TIM-3-expressing cells.

An antibody which specifically binds to human folate receptor 1 (FOLR1) is described in WO2014/087863 and WO2015/186823. An antibody which specifically binds to human IL-3Rα is described in WO2010/126066. An antibody which specifically binds to human TIM-3 is described in WO2011/155607.

(A1) An antibody which specifically binds to FOLR1,

(A2) a monoclonal antibody which specifically binds to FOLR1, and

(A3) a monoclonal antibody which specifically binds to FOLR1 selected from the following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;

(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the

amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8, used in the present invention can be produced based on the methods described in, for example, WO2014/087863.

(B1) An antibody which specifically binds to human IL-3Rα,
(B2) the antibody according to (B1), which does not inhibit IL-3 signaling and binds to B domain of the human IL-3Rα chain but does not bind to C domain of the human IL-3Rα chain,
(B3) the antibody according to (B2) which comprises amino acid sequences of CDRs of heavy chain and CDRs of light chain selected from the group consisting of the following (a2) to (e2);
(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs: 12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38,
respectively, and
(B4) the antibody according to (B2) which comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively,
used in the present invention can be produced based on the methods described in, for example, WO2010/126066.
(C1) An antibody which specifically binds to human TIM-3,
(C2) a monoclonal antibody which specifically binds to human TIM-3, which binds to an extracellular region of human TIM-3,
(C3) the monoclonal antibody according to (C2) which is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively, and

(C4) the monoclonal antibody according to (C2) which is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60,
used in the present invention can be produced based on the methods described in, for example, WO2011/155607.

The combination of IDO inhibitor and the antibody which specifically binds to CCR4, HER2, human CD20, or EGFR of the present invention can be used in the treatment of any tumor expressing CCR4, HER2, human CD20 or EGFR. And also the combination of IDO inhibitor and the antibody which specifically binds to FOLR1, human IL-3Rα or human TIM-3 of the present invention can be used in the treatment of tumor.

Examples of the tumor which is associated with FOLR1 include blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma, pancreatic cancer, and the like. Preferred examples of the tumor which is associated with FOLR1 include ovarian cancer.

Examples of the tumor which is associated with human IL-3Rα include acute myeloid leukemia (AML), acute lymphocytic leukemia, atypical leukemia, chronic lymphocytic leukemia, adult T cell leukemia, NK/T cell lymphoma, granular lym-phocytosis (LGL leukemia), polycythemia vera, essential thrombocythemia, hypere-osinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma, Castleman disease, and the like. Preferred examples of the tumor which is associated with human IL-3Rα include acute myeloid leukemia (AML).

Examples of the tumor which is associated with human TIM-3 include blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer, pancreatic cancer, and the like. Preferred examples of the tumor which is associated with human TIM-3 include acute myeloid leukemia (AML).

Examples of the tumor include hematopoietic tumor, ovarian cancer, breast cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, head and neck cancer, osteosarcoma, melanoma, and brain tumor.

Examples of hematopoietic tumor include acute leukemia, chronic leukemia, Hodgkin's disease (or Hodgkin's lymphoma), non-Hodgkin's disease (or non-Hodgkin's lymphoma), and the like.

Examples of the acute leukemia include acute lymphatic leukemia and the like, and examples of the acute lymphatic leukemia include pre-B cell acute lymphatic leukemia, pre-T cell acute lymphatic leukemia and the like.

Examples of the chronic leukemia include chronic lymphatic leukemia and the like.

Examples of the non-Hodgkin's disease include T cell and NK cell lymphoma, B cell lymphoma and the like, and Examples of the T cell and NK cell lymphoma include precursor T lymphoblastic leukemia/lymphoma, mature T cell tumor and the like.

Examples of the B cell lymphoma include Burkitt's lymphoma, mantle cell lymphoma, diffuse large B cell lymphoma, and the like.

Examples of mature T cell tumor include T cell prolymphocytic leukemia, T cell large granular lymphocytic leukemia, Sezary syndrome, mycosis fungoides, primary cutaneous anaplastic large cell lymphoma, subcutaneous panniculitis-like T-cell lymphoma, enteropathy-type T-cell lymphoma, hepatosplenic γδ T-cell lymphoma, an-gioimmunoblastic T-cell lymphoma, peripheral T cell lymphoma, cutaneous T cell lymphoma, anaplastic large cell lymphoma, adult T-cell leukemia/lymphoma, and the like.

Examples of Hodgkin's lymphoma include nodular lymphocyte predominant Hodgkin's lymphoma, classical Hodgkin's lymphoma, and the like. Examples of classical Hodgkin's lymphoma include nodular sclerosis Hodgkin's lymphoma, lymphocyte-rich classical Hodgkin's lymphoma, mixed cellularity Hodgkin's lymphoma, lymphocytic depleted Hodgkin's lymphoma, and the like.

A dose of the antibody which specifically binds to CCR4, HER2, human CD20, EGFR,FOLR1, human IL-3Rα or human TIM-3 varies depending on the desired therapeutic effect, the administration route, the period of treatment, age, body weight, and the like. And a dose of the antibody which specifically binds to CCR4, HER2, human CD20, EGFR, FOLR1, human IL-3Rα or human TIM-3 for an adult is generally 0.1 to 100 mg/kg or 0.1 to 400 mg/$m^2$ per dose. It is preferable that the medicament comprising an IDO inhibitor which is administered with the antibody which specifically binds to CCR4, HER2, human CD20, EGFR, FOLR1, human IL-3Rα or human TIM-3 be the same or less than that administered alone in clinical practice.

An administration frequency of the antibody which specifically binds to CCR4, HER2, human CD20, EGFR, FOLR1, human IL-3Rα or human TIM-3 is once in every two weeks or per week.

The effect of a combination of an IDO inhibitor and an antibody of the present invention can be investigated, using the method according to the following "2.4 Test procedure". By using the method and comparing the effect by the administration of an IDO inhibitor alone with the effect of a combination of the IDO inhibitor and an antibody of the present invention, the effect of the combination can be evaluated.

Examples of the cultured cells to be used include TL-Om1 cells. TL-Om1 cells are derived from a patient with adult T cell leukemia, and can be used as a model of human adult T cell leukemia.

Examples of the cultured cells to be used include HH cells. HH cells are derived from a patient with cutaneous T cell lymphoma, and can be used as a model of cutaneous T cell lymphoma.

Examples of the cultured cells to be used include SK BR-3 cells. SK BR-3 cells are derived from a patient with breast cancer, and can be used as a model of breast cancer.

Examples of the cultured cells to be used include Raji cells. Raji cells are derived from a patient with Burkitt's lymphoma, and can be used as a model of Burkitt's lymphoma.

Examples of the cultured cells to be used include A431 cells. A431 cells are derived from a patient with epidermoid carcinoma, and can be used as a model of EGFR-positive tumor.

Examples of the cultured cells to be used include SKOV3 cells. SKOV3 cells are derived from a patient with ovarian cancer, and can be used as a model of ovarian cancer.

Examples of the cultured cells to be used include KG-1 cells. KG-1 cells are derived from a patient with acute myeloid leukemia and can be used as a model of acute myeloid leukemia.

Examples of the cultured cells to be used include EoL-1 cells which are the parental strains of EoL-1/human TIM-3. EoL-1 cells are derived from a patient with acute myeloid leukemia, and EoL-1/human TIM-3 can be used as a model of acute myeloid leukemia. As long as a combination of the present invention is a combination of an IDO inhibitor such as Compound (I), (II) or a pharmaceutically acceptable salt thereof and an antibody which specifically binds to CCR4, HER2, human CD20, EGFR, FOLR1, human IL-3R$\alpha$ or human TIM-3, the combination can be used, administered, or produced as a single agent (mixture) or as a combination of a plurality of preparations.

The single agent (mixture) or the preparations desirably have unit dose forms suitable for oral administration or parenteral administration, such as injection. When a combination of a plurality of preparations is used or administered, the preparations may be used together or separately at intervals.

The preparations can be produced by an ordinary method using, in addition to the active ingredients, a pharmaceutically acceptable diluent, excipient, disintegrant, lubricant, binder, surfactant, water, physiological saline, vegetable oil solubilizer, isotonizing agent, preservative, antioxidant, and the like.

When tablets are prepared, for example, an excipient such as lactose, a disintegrant such as starch, a lubricant such as magnesium stearate, a binder such as hydroxypropyl cellulose, a surfactant such as a fatty ester, a plasticizer such as glycerin, and the like may be used in the ordinary manner.

When injections are prepared, for example, water, physiological saline, vegetable oil, a solvent, a solubilizer, an isotonizing agent, a preservative, an antioxidant, and the like may be used in the ordinary manner.

When Compound (I), (II) or a pharmaceutically acceptable salt thereof is used for the purposes, described above, it can be administered orally or parenterally as an injection or the like. The effective dose and number of doses thereof may vary depending on the administration form, the age, body weight, symptom, and the like of patients. The daily dose of Compound (I), (II) or a pharmaceutically acceptable salt thereof is usually 0.01 to 100 mg/kg, preferably 0.08 to 100 mg/kg.

TEST EXAMPLE 1

1 SUMMARY

In this study, we examined the effect of IDO1 activity on antibody dependent cellular cytotoxicity (ADCC) using human peripheral blood mononuclear cells (PBMCs) or purified NK cells. Before ADCC assay, human PBMCs were incubated in conditioned medium of KATO-III cell culture. To examine the effect of IDOl activity, IFN-$\gamma$ and/ or IDO1 inhibitors (Compound A1 and Compound B1) were added to the KATO-III cell culture.

When human PBMCs or purified NK cells were pre-incubated in the conditioned medium of KATO-III cells stimulated with IFN-$\gamma$, the ADCC of mogamulizumab was attenuated compared with that in the absence of the stimulation with IFN-$\gamma$. This attenuation was cancelled by Compound A1 or Compound B1, suggesting that the attenuation of ADCC was induced by IDO1 activity. Similar results were observed when trastuzumab, rituximab and cetuximab were used as ADCC inducing antibodies.

2 MATERIALS AND METHODS

2.1 Test and control articles

2.1.1 Compound A1

Compound A1 was obtained as Compound 64A of example 62 in WO2011/142316 and Compound A1 was dissolved in dimethyl sulfoxide (DMSO) at a concentration of 100 mmol/L as a stock solution, and was kept under frozen condition until use. The stock solution of Compound A1 was diluted with assay medium (section 2.4.2).

2.1.2 Compound B1

Compound B1 was obtained as "4-({2-[(aminosulfonyl)amino]ethyl}amino)-N-(3-bromo-4-fluorophenyl)-N'-hydroxy-1,2,5-oxadiazole-3-carboximidamide" of example 1 in WO2010/005958. Compound B1 was dissolved in DMSO at a concentration of 100 mmol/L as a stock solution, and was kept under frozen condition until use. The stock solution of Compound B1 was diluted with assay medium (section 2.4.2).

2.1.3 Mogamulizumab

Mogamulizumab was produced by a similar production method of KM8760 described in WO2011030841. The solution (9.7 mg/mL) was diluted with assay medium (section 2.4.2).

2.1.4 Trastuzumab

Trastuzumab (Herceptin(R), 440 mg, Lot No. N3566B01 B2060) was purchased from F. Hoffmann-La Roche. The solution (9.7 mg/mL) was diluted with assay medium (section 2.4.2).

2.1.5 Rituximab

Rituximab (Mabthera(R), 10 mg/mL, Lot No. B6061B01) was purchased from F. Hoffmann-La Roche. The solution (10 mg/mL) was diluted with assay medium (section 2.4.2).

2.1.6 Cetuximab

Cetuximab (ERBITUX(R), 5 mg/mL, Lot No. MG1201) was purchased from Merck Serono. The solution (5 mg/mL) was diluted with assay medium (section 2.4.2).

2.2 Standard articles, internal standards, and reagents for quantification of kynurenine and tryptophan

2.2.1 Standard articles

L-Tryptophan (Trp, Lot No. PDQ6460) was purchased from Wako Pure Chemical Industries. L-Kynurenine (Kyn, Lot No. BCBJ6934V) was purchased from Sigma-Aldrich.

Trp and Kyn were dissolved in water to prepare stock solutions at a concentration of 10 mmol/L. The stock solutions were stored at 4°C until use.

2.2.2 Internal standards

L-Kynurenine (ring-$D_4$) ($d_4$-Kyn, Lot No. L-KYNU-D4-005) was purchased from Buchem B.V. L-Tryptophan (Indole-D5, 98%) ($d_5$-Trp, Lot No. 1-15891) was purchased from Cambridge Isotope Laboratories. d4-Kyn and d5-Trp were dissolved in water to prepare stock solutions at a concentration of 10 mmol/L. The stock solutions were stored at 4°C. The stock solutions of d4-Kyn and d5-Trp were diluted with water and mixed to make an IS solution (containing $d_4$-Kyn at 0.6 μmol/L and $d_5$-Trp at 2 μmol/L). The IS solutions were stored at 4°C until use. The IS solution was mixed with trifluoroacetic acid (TFA) to make an IS/TFA solution (5/1, v/v) just prior to use.

2.2.3 Reagents

Methanol of HPLC-grade, TFA and formic acid were purchased from Wako Pure Chemical Industries, Ltd. All other reagents used were of analytical grade unless otherwise noted.

2.3 Test system

A human gastric cancer cell line, KATO-III (86093004) was obtained from DS Pharma Biomedical Co., Ltd. A human adult T-cell leukemia/lymphoma cell line, TL Om1 (TKG0289) was obtained from Cell Resource Center for Biomedical Research. A human cutaneous T cell lymphoma cell line, HH (CRL-2105), a human breast cancer cell line, SK-BR-3 (HTB30) and a human lymphoma cell line, Raji (CCL-86) were obtained from ATCC. A human epidermoid carcinoma cell line, A431 (JCRB0004) was obtained from JCRB.

A human gastric cancer cell line, KATO-III (86093004), was subcultured with RPMI1640 (Invitrogen, Cat.

No.11875-093) containing 10 vol% heat-inactivated fetal bovine serum (Invitrogen, Cat. No.10099-141, Lot No. 1108863) and 1 vol% Penicillin-Streptomycin (nacalai tesque, Cat. No.26253-84). A human adult T-cell leukemia/lymphoma cell line, TL-Om1 (TKG 0289), was subcultured with RPMI1640 containing 20 vol% heat-inactivated fetal bovine serum and 1 vol% Penicillin-Streptomycin. A human cutaneous T cell lymphoma cell line, HH (CRL-2105), was subcultured with RPMI1640 containing 10 vol% heat-inactivated fetal bovine serum, 10 mmol/L HEPES (nacalai tesque, Cat. No. 17557-94), 1 mmol/L sodium pyruvate (Invitrogen, Cat. No. 11360-070) and 1 vol% Penicillin-Streptomycin. A human breast cancer cell line, SK-BR-3 (HTB30), was subcultured with McCoy's 5A (Invitrogen, Cat. No. 16600-082) containing 10 vol% heat-inactivated fetal bovine serum and 1 vol% Penicillin-Streptomycin. A human lymphoma cell line, Raji (CCL-86), was subcultured with RPMI1640 containing 10 vol% heat-inactivated fetal bovine serum, 4.5 g/L D-(+)-glucose (Sigma-Aldrich, Cat. No. G8769), 10 mmol/L HEPES and 1 vol% Penicillin-Streptomycin. A human epidermoid carcinoma cell line, A431 (JCRB0004), was subcultured with DMEM (Invitrogen, Cat. No. 11995-065) containing 10 vol% heat-inactivated fetal bovine serum and 1 vol% Penicillin-Streptomycin.

In preparation of conditioned medium, all the cells were cultured in RPMI1640 containing 10 vol% heat-inactivated dialyzed fetal bovine serum (Invitrogen, Cat. No.30067334).

Frozen human peripheral blood mononuclear cells (PBMCs) from 3 individuals were purchased from Allcells [Lot No. A3457 (donor 1) and Lot No. A3951 (donor 2)] and Precision Bioservices [Lot No. 13096 (donor 3)], and used separately.

2.4 Test procedure

2.4.1 Preparation of conditioned medium

KATO-III cells (1.25 $\times 10^6$ cells) were treated with 25 ng/mL recombinant human IFN-$\gamma$. Compound A1 or Compound B1 was added at a final concentration of 100 nmol/L. Cells were incubated in a $CO_2$ incubator. Three days later, cells and supernatants were separated by centrifugation. The supernatants were used as conditioned medium. The conditioned medium was used for incubation of human PBMCs or NK cells (section2.4.2), and measurement of Kyn and Trp (section 2.4.4). The concentration of Kyn and Trp was shown in Fig. 1.

Conditioned medium 1 : KATO-III cells
Conditioned medium 2 : KATO-III cells + IFN-$\gamma$
Conditioned medium 3 : KATO-III cells + IFN-$\gamma$ + Compound A1
Conditioned medium 4 : KATO-III cells + IFN-$\gamma$ + Compound B1

2.4.2 ADCC

NK cells were isolated from human PBMCs using NK isolation kit (Miltenyi Biotec, Cat. No. 130-092-657) and autoMACS instrument. Human PBMCs or NK cells were cultured in the conditioned medium (section 2.4.1) in a $CO_2$ incubator. After 7 days, cells were harvested and counted, and used as effector cells. PBMCs and NK cells used as effector cells were suspended in the assay medium (RPMI1640 containing 10 vol% heat-inactivated fetal bovine serum and 1 vol% Penicillin-Streptomycin) to $5\times10^6$ cells/mL (PBMCs) or $4\times10^5$ cells/mL (NK cells). TL-Om1 cells, HH cells, SK-BR-3 cells, Raji cells or A431 cells ($1\times10^6$ cells/each cell line) were labeled with 1.85 MBq of $Na_2{}^{51}CrO_4$ (PerkinElmer) for over 1 hour at 37 ∘C in a $CO_2$ incubator. The cells were washed 3 times with assay medium. The radiolabeled cells used as target cells were suspended in 5 mL of the assay medium to $2\times10^5$ cells/mL. Mogamulizumab, trastuzumab, rituximab or cetuximab was serially diluted with the assay medium by 10-fold from 30 $\mu$g/mL to 0.3 $\mu$g/mL (final concentrations: from 10 $\mu$g/mL to 0.1 $\mu$g/ mL). Fifty $\mu$L of the target cells ($1\times10^4$ cells) were plated into 96-well round bottomed culture plates and mixed with 50 $\mu$L of antibody dilutions prepared as described above and 50 $\mu$L of the effector cells ($2.5\times10^5$ cells). The culture plates were briefly centrifuged at 4 ∘C and incubated at 37 ∘C in a $CO_2$ incubator for about 4 hours. After centrifugation for 5 minutes at 4 ∘C, the supernatants were transferred to LumaPlate (PerkinElmer) and dried sufficiently. The radioactivity was counted with a microplate scintillation counter (TopCount NXT, PerkinElmer).

2.4.3 Flow cytometric analysis of NK cells

Human PBMCs were cultured in the conditioned medium in a $CO_2$ incubator. After 7 days, cells were harvested and stained with Fixable Viability Dye eFluor(R) 506 (eBioscience, Cat. No. 65-0866-14). After washing twice with MACS buffer [autoMACS Rinsing Solution (Miltenyi Biotec, Cat. No. 130-091-222) containing 1 w/ v% BSA], cells were stained with PerCP anti-human CD3 antibody (Biolegend, Cat. No. 300428), FITC anti-human CD16 antibody (BD Pharmingen™, Cat. No. 556618) and PE/Cy7 anti-human CD56 antibody (BD Pharmingen™, Cat. No. 557747). After washing twice with MACS buffer, cells were suspended in 200 $\mu$L of MACS buffer and analyzed by FACSVerse™ (BD Biosciences)

with the aid of FACSuite software. Proportion of CD16+ CD56+ cells in CD3- cells (NK cells) was determined by FlowJo software (version 7. 6. 5, FlowJo). All the staining processes were performed according to manufacturer's instructions. The Proportion of CD16+ CD56+ cells in CD3- cells was shown in A, B, C and D of Fig. 2.

2.4.4 Quantification of Kyn and Trp in sample

2.4.4.1 Pretreatment of samples

Fifty μL of the conditioned medium (section 2.4.1) and 60 μL of the ice-cold IS/TFA solution (5/1, v/v) were mixed, and the mixture was centrifuged (5000×g, room temperature, 10 min). Sixty μL of the supernatant and 50 μL of 0.05 vol% formic acid/ methanol = 75/25 (v/v) were mixed, and the resulting mixture was injected into a liquid chromatography tandem mass spectrometry (LC/MS/MS) system.

2.4.4.2 Preparation of calibration standard samples

Calibration standard solutions were prepared by dilution of the Trp and Kyn stock solutions with water. The concentration range of calibration standard solutions was 0.1 to 500 (Trp) and 0.05 to 200 (Kyn) μmol/L, and these were stored at 4 ∘C. For blank sample, water was used in place of calibration standard solutions. The 50 μL of calibration standard solution and blank sample were pretreated by the same procedure as described in section 2.4.4.1.

2.4.4.3 LC/MS/MS analysis

<Analytical system>

| | |
|---|---|
| LC: | Agilent 1200 (Agilent Technologies) |
| Autosampler: | HTC PAL (CTC Analytics) |
| MS/MS: | API5000 (AB SCIEX) |
| Analytical software: | Analyst 1.6.1 (AB SCIEX) |
| Column: | Atlantis T3 (3 μm, 4.6 mm × 75 mm, Waters) |
| Pre-filter: | A-103x (0.5 μm Upchurch Scientific) |
| Column temperature: | Room temperature |

<LC conditions>

| | |
|---|---|
| Mobile phase A: | 0.05 vol% formic acid |
| Mobile phase B: | Methanol |

<LC conditions>

| | |
|---|---|
| Mobile phase A: | 0.05 vol% formic acid |
| Mobile phase B: | Methanol |

[Table 1]

| Time (min) | Flow rate (mL/min) | A (vol%) | B (vol%) |
|---|---|---|---|
| 0 | 0.6 | 95 | 5 |
| 0.2 | 0.6 | 95 | 5 |
| 3.5 | 0.6 | 30 | 70 |
| 3.51 | 0.6 | 10 | 90 |
| 5 | 0.6 | 10 | 90 |
| 5.01 | 0.6 | 95 | 5 |
| 7 | 0.6 | 95 | 5 |

Injection volume: 10 μL

<MS/MS conditions>

Ionization mode: Electrospray ionization, positive
Source temperature: 500 °C
Detection: Multiple reaction monitoring
Monitoring ion:

[Table 2]

| | Q1 mass (*m/z*) | Q3 mass (*m/z*) | Declustering potential (V) | Collision energy (eV) |
|---|---|---|---|---|
| Trp | 205.1 | 146.4 | 140 | 40 |
| Kyn | 209.0 | 94.2 | 20 | 20 |
| IS ($d_5$-Trp) | 209.8 | 150.2 | 140 | 30 |
| IS ($d_1$-Kyn) | 212.8 | 98.3 | 20 | 20 |

2.5 Methods for analysis of raw data

The Analyst software (version 1.6.1, AB SCIEX) was used for the calculation of concentrations of Trp and Kyn. Calibration curves were constructed from the peak area ratios (analyte/ IS) obtained from the calibration standard samples by least squares linear regression [Y = aX + b; Y, peak area ratio; X, concentration; weighting factor, $1/Y^2$]. Blank samples were not included in the regression analysis.

The Kyn and Trp concentrations in samples were individually calculated. Values below the lower limit of quantification were regarded as 0 μmol/L.

The percentage of cytotoxicity was calculated according to the following formula:

[Math.1]

$$\text{Cytotoxicity } \% = (E\text{-}S)/(M\text{-}S) \times 100$$

E is the experimental released radioactivity (cpm), S is the mean of spontaneous released radioactivity (cpm) by adding the assay medium instead of effector cells and antibody, and M is the mean of maximum released radioactivity (cpm) by adding 10 vol% Triton™ X-100 (Sigma-Aldrich, Cat. No. T9284-100ML) instead of effector cells and antibody. Cytotoxicity at each concentration of the antibodies was presented as mean of triplicate.

3 RESULTS

3.1 Effect of Compound A1 and Compound B1 on Kyn production and Trp consumption

To confirm IDOl activity in KATO-III cells, concentrations of Kyn and Trp in the conditioned medium of KATO-III cell cultures were measured. As shown in Figure 1A and 1B, an increase of Kyn concentration and a decrease of Trp concentration were observed in conditioned medium 2 (KATO-III cells + IFN-γ) compared with conditioned medium 1 (KATO-III cells). When KATO-III cells were treated with Compound A1 (conditioned medium 3) or Compound B1 (conditioned medium 4), which are IDOl inhibitors, in the presence of IFN-γ, Kyn production and Trp consumption were inhibited (Figure 1A and 1B).

3.2 Detection of NK cells

After incubation of human PBMCs with each conditioned medium, the existence of NK cells (CD3-/CD16+ /CD56+ cells) was confirmed. As shown in Figure 2, NK cells were detected in PBMCs incubated with all conditioned mediums.

3.3 Effect of IDOl activity on ADCC

To examine the effect of IDOl activity on ADCC, ADCC by human PBMCs pre-incubated in each conditioned medium were measured. When human PBMCs from donor1 and donor 2 were pre-incubated in the conditioned medium 2, the ADCC of mogamulizumab against TL-Om1 cells was attenuated compared with the conditioned medium 1 (Figure 3A and 3B). Compound A1 or Compound B1 canceled the attenuation; the ADCCs by human PBMCs from donor 1 and donor 2 pre-incubated in conditioned medium 3 or 4 were almost same as that in conditioned medium 1. Similar results were observed when using the other target cells and antibodies; all the tested ADCCs of mogamulizumab, trastuzumab, rituximab and cetuximab against HH cells, SK-BR-3 cells, Raji cells and A431 cells, respectively, were attenuated by the pre-incubation of human PBMCs with the conditioned medium 2, and Compound A1 or Compound B1 canceled the attenuation (Figure 3B, Figure 4, Figure 5 and Figure 6).

To confirm that the attenuation of ADCC by conditioned medium 2 was not due to the decrease of proportion of NK cells in PBMCs, ADCC by purified NK cells pre-incubated in each conditioned medium were measured. Similar to Figure 3, the ADCC of mogamulizumab by NK cells pre-incubated in conditioned medium 2 was attenuated, and this attenuation was canceled by Compound A1 or Compound B1 (Figure 7). Similar results were observed when using rituximab, Raji cells and purified human NK cells (Figure 8).

Further, to investigate the effect of Compound A1 or Compound B1 itself on ADCC, human PBMCs were pre-incubated in medium containing 100 nmol/L Compound Alor CompoundB1 for 7 days. As shown in Figure 9, Compound A1 or Compound B1 did not influence the ADCC of mogamulizumab.

4 DISCUSSION

The results of the present study showed that ADCC was attenuated by IDOl activity and IDOl inhibitors canceled this attenuation. In a previous study, it was suggested that Trp metabolites, such as Kyn, reduced NK cell number [J Exp Med. 2002;196(4):447-57]. In the present study, NK cells in PBMCs pre-incubated in each conditioned medium were alive before ADCC assay (Figure 2).

Furthermore, attenuation of ADCC was observed when purified NK cells were used (Figure 7 and 8). In this case, NK cell numbers had been counted and the same numbers of NK cells among each group were used for ADCC measurement. These data suggested that ADCC attenuation by IDOl activity was considered to be due to suppression of NK cell function, not due to reduction of NK cell number.

The ADCC attenuation were observed when using trastuzumab, rituximab and cetuximab, suggesting that attenuation of ADCC by IDO activity would occur regardless of antibody formats, i.e. nonfucosylated IgG1 and fucosylated IgG1.

An increase of Kyn concentration and a decrease of Trp concentration in the conditioned medium of KATO-III cells in the presence of IFN-γ was observed (Figure 1) and the increase of Kyn concentration and the decrease of Trp were inhibited by Compound A1 or Compound B1. Further, Compound A1 or Compound B1 itself did not influence ADCC (Figure 9). Taken together, Kyn production or Trp consumption in conditioned medium would attenuate NK cell function.

TEST EXAMPLE 2

5 Summary

In this study, we examined the effect of IDO1 activity on antibody dependent cellular cytotoxicity (ADCC) of mogamulizumab using human peripheral blood mononuclear cells (PBMCs). Before ADCC assay, human PBMCs were incubated in conditioned medium of parental KATO-III or KATO-III cells stably expressing IDOl shRNA or negative control shRNA in the absence or presence of IFN-γ.

When human PBMCs were pre-incubated in the conditioned medium of parental KATO-III or KATO-III cells stably expressing negative control shRNA in the presence of IFN-γ, the ADCC of mogamulizumab by human PBMCs was attenuated compared with that in the absence of IFN-γ. In the case of human PBMCs pre-incubated in conditioned medium of KATO-III cells stably expressing IDOl shRNA in the presence of IFN-γ, the ADCC was not attenuated, suggesting that attenuation of ADCC was caused by IDOl activity.

6 INTRODUCTION

IDOl is known to be expressed in tumors or the microenvironment, and plays an important regulatory role in the immunosuppressive mechanisms, which is responsible for tumor's escape from host immune surveillance [Nat Rev Cancer. 2009;9(6):445-52]. It has been reported that tryptophan (Trp) metabolites, such as kynurenine (Kyn), induce NK cell death [J Exp Med. 2002;196(4):447-57], and weaken NK cell cytotoxicity by inhibiting the expression of NK cell

receptros [Blood. 2006;108(13):4118-25]. Therefore, there is a possibility that IDOl activity affects antibody dependent cellular cytotoxicity (ADCC).

In this study, we examined the effect of IDO1 activity on ADCC of mogamulizumab using IDO1 knock down technology.

## 7 MATERIALS AND METHODS

### 7.1 Test and control articles

#### 7.1.1 Mogamulizumab

Mogamulizumab was produced by a similar production method of KM8760 described in WO2011030841. The solution (9.7 mg/mL) was diluted with assay medium (section 7.4.5).

### 7.2 Standard articles, internal standards, and reagents for quantification of kynurenine and tryptophan

#### 7.2.1 Standard articles

L-Tryptophan (Trp, Lot No. PDQ6460) was purchased from Wako Pure Chemical Industries. L-Kynurenine (Kyn, Lot No. BCBJ6934V) was purchased from Sigma-Aldrich.

Trp and Kyn were dissolved in water to prepare stock solutions at a concentration of 10 mmol/L. The stock solutions were stored at 4 °C until use.

#### 7.2.2 Internal standards

L-Kynurenine (ring-$D_4$) ($d_4$-Kyn, Lot No. L-KYNU-D4-005) was purchased from Buchem B.V. L-Tryptophan (Indole-D5, 98%) ($d_5$-Trp, Lot No. 1-15891) was purchased from Cambridge Isotope Laboratories. d4-Kyn and d5-Trp were dissolved in water to prepare stock solutions at a concentration of 10 mmol/L. The stock solutions were stored at 4°C. The stock solutions of d4-Kyn and d5-Trp were diluted with water and mixed to make an IS solution (containing $d_4$-Kyn at 0.6 μmol/L and $d_5$-Trp at 2 μmol/L). The IS solutions were stored at 4°C until use. The IS solution was mixed with trifluoroacetic acid (TFA) to make an IS/TFA solution (5/1, v/v) just prior to use. 7.2.3 Reagents

Methanol of HPLC-grade, TFA and formic acid were purchased from Wako Pure Chemical Industries, Ltd. All other reagents used were of analytical grade unless otherwise noted.

### 7.3 Test system

A human gastric cancer cell line, KATO-III (86093004) was obtained from DS Pharma Biomedical Co., Ltd. TL Om1 (TKG0289) was obtained from Cell Resource Center for Biomedical Research.

A human gastric cancer cell line, KATO-III (86093004), was subcultured with RPMI1640 (Invitrogen, Cat. No.11875-093) containing 10 vol% heat-inactivated fetal bovine serum (Invitrogen, Cat. No.10099-141, Lot No. 1108863) and 1 vol% Penicillin-Streptomycin (nacalai tesque, Cat. No.26253-84). A human adult T-cell leukemia/lymphoma cell line, TL-Om1 (TKG0289), was subcultured with RPMI1640 containing 20 vol% heat-inactivated fetal bovine serum and 1 vol% Penicillin-Streptomycin.

In preparation of conditioned medium, the cells were suspended in RPMI1640 containing 10 vol% heat-inactivated dialyzed fetal bovine serum (Invitrogen, Cat. No.30067334) Frozen human peripheral blood mononuclear cells (PBMCs) were purchased from Allcells (Lot No. A3951).

### 7.4 Test procedure

#### 7.4.1 Lentiviral infections

Four different shRNA lentiviral particles targeting human IDOl [IDOl shRNA #44, #45, #46 and #47 (Sigma-Aldrich, Cat. No. SHCLNV)], empty vector control lentiviral particles [vector (Sigma-Aldrich, Cat. No. SHC001)] or negative control shRNA lentiviral particles [NegaCTRL shRNA (Sigma-Aldrich, Cat. No. SHC202)] were seeded in RetroNectin$^R$ Dish (TAKARA BIO, Cat. No. T110A). KATO-III cells ($1 \times 10^5$ cells) were added to each dish and were incubated in a $CO_2$ incubator. The infected cells were selected in puromycin (0.25 μg/mL) containing culture medium for a week. The knockdown of IDOl was evaluated by real-time quantitative PCR (qPCR, Section 7.4.2) and western blot analysis (Section 7.4.3).

7.4.2 qPCR analysis

Parental KATO-III or KATO-III cells stably expressing IDOl shRNA, vector or NegaCTRL shRNA ($9 \times 10^3$ cells) were treated with 25 ng/mL recombinant human IFN-γ.

Cells were incubated in a $CO_2$ incubator for about 24 hours. Total RNA extraction and cDNA synthesis were performed using TaqMan(R) Gene Expression Cells-to-CT ™ Kit (Applied Biosystems, Cat. No. AM1728) according to manufacturer's instructions. The cDNA samples were then analyzed by qPCR analysis using primers specific for human IDOl (Applied Biosystems, Cat. No. 4331182) and HPRT1 (Applied Biosystems, Cat. No. 4448489).

7.4.3 Western blot analysis

Parental KATO-III or KATO-III cells stably expressing IDOl shRNA, vector or NegaCTRL shRNA ($5.4 \times 10^5$ cells) were treated with 25 ng/mL recombinant human IFN-γ.

Cells were incubated in a $CO_2$ incubator. After 3 days, cells were harvested and lysed in 100 μL of lysis buffer [NP40 Cell Lysis Buffer (Invitrogen, Cat. No. FNN0221) containing 1 vol% phenylmethanesulfonyl fluoride solution (Sigma-Aldrich, Cat. No. 93482) and 1 vol% protease inhibitor cocktail (Sigma-Aldrich, Cat. No. P8340)] on ice for 30 minutes or more. The lysates were centrifuged at approximately 14000×rpm for 10 minutes at 4°C. The protein concentrations of the supernatants were measured using the Pierce™ BCA Protein Assay Kit (Pierce, Cat. No. 23225). For preparation of SDS-PAGE samples, each lysate was diluted with lysis buffer to the same concentration and mixed with 5×sample buffer (Thermo Fisher, Cat. No. 39000) resulting in the protein concentration of 1.5 μg/μL, and then heated for 5 minutes at 95°C. The protein in each sample was separated by SDS-PAGE and transferred to polyvinylidene fluoride (PVDF) membranes. After blocking of the membranes with PVDF Blocking buffer [for IDOl (TOYOBO, Cat. No.NYPBR01)] or blocking buffer [for β-actin (50 mmol/ L Tris-buffered saline (pH 8.0) containing 0.05 vol% Tween 20 (TBST), 5 w/v% skim milk)], the membranes were incubated with anti-IDO antibody (Upstate Biotechnology, Cat. No. 05-840) diluted at 1/100 in Can Get Signal solution 1 (TOYOBO, Cat. No.NKB-201) or anti-β-actin antibody (Sigma-Aldrich, Cat. No. 061M4808) diluted at 1/3000 in blocking buffer overnight at 4 °C. After washing with TBST for approximately 10 minutes three times, the membranes were incubated for about 1 hour with horseradish peroxidase-linked anti-mouse antibody (GE Healthcare, Cat#NA931V) diluted at 1/2000 in blocking buffer. After washing with TBST for approximately 10 minutes three times, the membranes were soaked in SuperSignal(R) West Pico Chemiluminescent Substrate (Pierce Biotechnology, Cat. No.34080). Chemiluminescent detection was done on a luminescent image analyzer, LAS3000, in accordance with the manufacturer's instructions.

7.4.4 Preparation of conditioned medium

Parental KATO-III or KATO-III cells stably expressing IDOl shRNA or NegaCTRL shRNA ($1.25 \times 10^6$ cells) were treated with 25 ng/mL recombinant human IFN-γ. Cells were incubated in a $CO_2$ incubator. Three days later, cells and supernatants were separated by centrifugation. The supernatants were used as conditioned medium. The conditioned medium was used for incubation of human PBMCs (Section 7.4.5), and measurement of Kyn and Trp (Section 7.4.7).

Conditioned medium 1 : KATO-III cells (parent)
Conditioned medium 2 : KATO-III cells (NegaCTRL shRNA)
Conditioned medium 3 : KATO-III cells (parent) + IFN-γ
Conditioned medium 4 : KATO-III cells (NegaCTRL shRNA) + IFN-γ
Conditioned medium 5 : KATO-III cells (IDOl shRNA #44) + IFN-γ
Conditioned medium 6 : KATO-III cells (IDOl shRNA #45) + IFN-γ
Conditioned medium 7 : KATO-III cells (IDOl shRNA #46) + IFN-γ

7.4.5 ADCC

Human PBMCs were cultured in each conditioned medium (Section 7.4.4) in a $CO_2$ incubator. After 7 days, cells were harvested and counted, and used as effector cells. PBMCs used as effector cells were suspended in the assay medium (RPMI1640 containing 10 vol% heat-inactivated fetal bovine serum and 1 vol% Penicillin-Streptomycin) to $5 \times 10^6$ cells/mL. TL-Om1 cells cells ($1 \times 10^6$ cells) were labeled with 1.85 MBq of $Na_2{}^{51}CrO_4$ (PerkinElmer) for over 1 hour at 37°C in a $CO_2$incubator. The cells were washed 3 times with assay medium. The radiolabeled cells used as target cells were suspended in 5 mL of the assay medium to $2 \times 10^5$ cells/ mL. Mogamulizumab was serially diluted with the assay medium by 10-fold from 30 μg/mL to 0.3 μg/mL (final concentrations: from 10 μg/mL to 0.1 μg/mL). Fifty μL of the target cells ($1 \times 10^4$ cells) were plated into 96-well round bottomed culture plates and mixed with 50 μL of antibody dilutions prepared as described above and 50 μL of the effector cells ($2.5 \times 10^5$ cells). The culture plates were briefly

centrifuged at 4°C and incubated at 37°C in a $CO_2$ incubator for about 4 hours. After centrifugation for 5 minitues at 4°C, the supernatants were transferred to LumaPlate (PerkinElmer) and dried sufficiently. The radioactivity was counted with a microplate scintillation counter (TopCount NXT, PerkinElmer).

7.4.6 Flow cytometric analysis of NK cells

Human PBMCs were cultured in each conditioned medium and incubated in a $CO_2$ incubator. After 7 days, cells were harvested and stained with Fixable Viability Dye eFluor(R) 506 (eBioscience, Cat. No. 65-0866-14). After washing twice with MACS buffer [autoMACS Rinsing Solution (Miltenyi Biotec, Cat. No. 130-091-222) containing 1 w/v% BSA], cells were stained with PerCP anti-human CD3 antibody (Biolegend, Cat. No. 300428), FITC anti-human CD16 antibody (BD Pharmingen™, Cat. No. 556618) and PE/Cy7 anti-human CD56 antibody (BD Pharmingen™, Cat. No. 557747). After washing twice with MACS buffer, cells were suspended in 200 μL of MACS buffer and analyzed by FACSVers™ (BD Biosciences) with the aid of FACSuite software. Proportion of CD16+ CD56+ cells in CD3 cells (NK cells) was determined by FlowJo software (version 7. 6. 5, FlowJo). All the staining processes were performed according to manufacturer's instructions.

7.4.7 Quantification of Kyn and Trp in sample

7.4.7.1 Pretreatment of samples

Fifty μL of the conditioned medium (Section 7.4.4) and 60 μL of the ice-cold IS/TFA solution (5/1, v/v) were mixed, and the mixture was centrifuged (5000×g, room temperature, 10 min). Sixty μL of the supernatant and 50 μL of 0.05 vol% formic acid/ methanol = 75/25 (v/v) were mixed, and the resulting mixture was injected into a liquid chromatography tandem mass spectrometry (LC/MS/MS) system.

7.4.7.2 Preparation of calibration standard samples

Calibration standard solutions were prepared by dilution of the Trp and Kyn stock solutions with water. The concentration range of calibration standard solutions was 1 to 500 (Trp) and 0.05 to 20 (Kyn) μmol/L, and these were stored at 4°C. For blank sample, water was used in place of calibration standard solutions. The 50 μL of calibration standard solution and blank sample were pretreated by the same procedure as described in Section 7.4.7.1.

7.4.7.3 LC/MS/MS analysis

<Analytical system>

| | |
|---|---|
| LC: | Agilent 1200 (Agilent Technologies) |
| Autosampler: | HTC PAL (CTC Analytics) |
| MS/MS: | API5000 (AB SCIEX) |
| Analytical software: | Analyst 1.6.1 (AB SCIEX) |
| Column: | Atlantis (3 μm, 4.6 mm × 75 mm, Waters) |
| Pre-filter: | A-103x (0.5 μm Upchurch Scientific) |
| Column temperature: | Room temperature |

<LC conditions>

| | |
|---|---|
| Mobile phase A: | 0.05 vol% formic acid |
| Mobile phase B: | Methanol |

[Table 3]

| Time (min) | Flow rate (mL/min) | A (vol%) | B (vol%) |
|---|---|---|---|
| 0 | 0.6 | 95 | 5 |
| 0.2 | 0.6 | 95 | 5 |
| 3.5 | 0.6 | 30 | 70 |

(continued)

| Time (min) | Flow rate (mL/min) | A (vol%) | B (vol%) |
|---|---|---|---|
| 3.51 | 0.6 | 10 | 90 |
| 5 | 0.6 | 10 | 90 |
| 5.01 | 0.6 | 95 | 5 |
| 7 | 0.6 | 95 | 5 |

Injection volume: 10 μL

<MS/MS conditions>

| | |
|---|---|
| Ionization mode: | Electrospray ionization, positive |
| Source temperature: | 500°C |
| Detection: | Multiple reaction monitoring |
| Monitoring ion: | |

[Table 4]

| | Q1 mass (*m*/*z*) | Q3 mass (*m*/*z*) | Declustering potential (V) | Collision energy (eV) |
|---|---|---|---|---|
| Trp | 205.1 | 146.4 | 140 | 40 |
| Kyn | 209.0 | 94.2 | 20 | 20 |
| IS ($d_5$-Trp) | 209.8 | 150.2 | 140 | 30 |
| IS ($d_4$-Kyn) | 212.8 | 98.3 | 20 | 20 |

7.5 Methods for analysis of raw data

The Analyst software (version 1.6.1, AB SCIEX) was used for the calculation of concentrations of Trp and Kyn. Calibration curves were constructed from the peak area ratios (analyte/ IS) obtained from the calibration standard samples by least squares linear regression [Y = aX + b; Y, peak area ratio; X, concentration; weighting factor, $1/Y^2$]. Blank samples were not included in the regression analysis.

The Kyn and Trp concentrations in samples were individually calculated. Values below the lower limit of quantification were regarded as 0 μmol/L.

Relative changes in the transcriptional levels of IDO1 between the parental KATO-III cells and KATO-III cells stably expressing IDOl shRNA, vector or NegaCTRL shRNA were measured using the comparative Ct method ($2^{-ddCt}$) [Methods. 2001;25(4):402-8].

The percentage of cytotoxicity was calculated according to the following formula:

[Math.2]

$$\text{Cytotoxicity } \% = (E\text{-}S)/(M\text{-}S) \times 100$$

E is the experimental released radioactivity (cpm), S is the mean of spontaneous released radioactivity (cpm) by adding the assay medium instead of effector cells and antibody, and M is the mean of maximum released radioactivity (cpm) by adding 10 vol% Triton™ X-100 (Sigma-Aldrich, Cat. No. T9284-100ML) instead of effector cells and antibody. Cytotoxicity at each concentration of the antibodies was presented as mean of triplicate.

8 RESULTS

8.1 Validation of IDO1 knockdown in KATO-III cells

To confirm knockdown of IDO1 mRNA in KATO-III cells stably expressing IDOl shRNA, IDOl mRNA level in KATO-III cells treated with IFN-γ was detected by qPCR analysis. In cells introduced with IDOl shRNA, the mRNA expression of IDO1 was decreased to approximately 80% in all the 4 different shRNA-introduced cells compared with parental KATO-III cells (Figure 10A). IDOl mRNA levels were not affected by introduction of a vector or NegaCTRL

shRNA (Figure 10A). Next, the effect of shRNA silencing on IDOl protein level was measured by western blot analysis. IDOl protein expression was markedly decreased in KATO-III cells stably expressing IDO1 shRNA#44, #45 and #46 compared to parental KATO-III cells (Figure 10B). IDOl protein expression in KATO-III cells stably expressing IDOl shRNA#47 was partially decreased (Figure 10B). IDOl protein levels were not affected by introduction of a vector or NegaCTRL shRNA (Figure 10B).

In subsequent experiments, we examined the effect of IDOl activity on ADCC using KATO-III cells stably expressing IDOl shRNA#44, #45 and #46, and NegaCTRL shRNA.

8.2 Effect of IDOl knockdown in KATO-III cells on Kyn production and Trp consumption

To examine effect of IDOl knockdown in KATO-III cells on Kyn production and Trp consumption, concentrations of Kyn and Trp in the conditioned medium of parental KATO-III or KATO-III cells stably expressing IDOl shRNA or NegaCTRL shRNA in the absence or presence of IFN-γ were measured.

As shown in Figure 11, an increase of Kyn concentration and a decrease of Trp concentration were not observed in parental KATO-III or KATO-III cells stably expressing NegaCTRL shRNA in the absence of IFN-γ. When parental KATO-III or KATO-III cells stably expressing NegaCTRL shRNA were treated with IFN-γ, the increase of Kyn concentration and the decrease of Trp concentration were observed. On the other hand, an increase of Kyn concentration and a decrease of Trp concentration were suppressed in all the 3 different IDOl shRNA-introduced cells even in the presence of IFN-γ.

8.3 Detection of NK cells

After incubation of human PBMCs with each conditioned medium, the existence of NK cells ($CD3^-/CD16^+$ $/CD56^+$ cells) was confirmed. As shown in Figure 12, NK cells were detected in PBMCs incubated with all conditioned medium.

8.4 Effect of IDOl activity on ADCC

To examine the effect of IDOl activity on ADCC, ADCC activities by human PBMCs pre-incubated in each conditioned medium were measured. When human PBMCs were pre-incubated in the conditioned medium 3 [KATO-III cells (parent) + IFN-γ] or 4 [KATO-III cells (NegaCTRL shRNA) + IFN-γ], the ADCC of mogamulizumab was attenuated compared with the conditioned medium 1 [KATO-III cells (parent)] or 2 [KATO-III cells (NegaCTRL shRNA)] (Figure 13). In the case of human PBMCs in conditioned medium 5 [KATO-III cells (IDOl shRNA #44) + IFN-γ], 6 [KATO-III cells (IDOl shRNA #45) + IFN-γ] or 7 [KATO-III cells (IDOl shRNA #46) + IFN-γ], the ADCC was not attenuated showing almost the same ADCC compared with the conditioned medium 1 or 2 (Figure 13).

9 DISCUSSION

This study demonstrated that ADCC was decreased by IDOl activity. In a previous study, it was suggested that Trp metabolites, such as Kyn, reduced NK cell number [Exp Med. 2002;196(4):447-57]. However, in this study, there was no marked reduction in the proportion of NK cells in PBMCs incubated in conditioned medium of KATO-III cells treated with IFN-γ, which induced the increase of Kyn concentration (Figure 12). This suggested that ADCC decreased by IDOl activity was considered to be due to suppression of NK cell function, not reduction of NK cell number. An increase of Kyn concentration and a decrease of Trp concentration were suppressed in conditioned medium of IDO1 shRNA expressing KATO-III cells in the presence of IFN-γ, suggesting that Kyn production or Trp consumption would be a reason to decrease NK cell function.

TEST EXAMPLE 3

SUMMARY

In this study, we examined the effect of IDO1 inhibitors on cytotoxicity of human peripheral blood mononuclear cells (PBMCs) in the presence or absence of HuRA15-7CTAcc (Antibody C1), an anti-folate receptor alpha (human folate receptor 1, FOLR1) antibody, against ovarian cancer cells. First, we examined IDOl expression in four types of ovarian cancer cells by Western blot. As results, IDO1 expression was observed in SKOV3, OVCAR3, and MCAS cells after IFNγ treatment, but not observed in OVISE cells in the same condition (Figure 14). Using SKOV3 cells, we prepared conditioned medium. SKOV3 cells were stimulated with IFNγ in the presence or absence of IDO1 inhibitors, Compound A1 or Compound B1, and then the supernatant was recovered as conditioned medium for PBMCs pre-incubation. When

the PBMCs were pre-incubated in the conditioned medium prepared in the presence of the IDOl inhibitors, the natural killing activity and Antibody C1-mediated cytotoxicity of the PBMCs were up-regulated (Figure 15 and Figure 16).

10 INTRODUCTION

FOLR1 is thought to be a promising target for cancer therapy under ongoing investigation (Ann Oncol. 2015 Jun 30). We created HuRA15-7CTAcc (Antibody C1) as a novel anti-FOLR1 antibody with stronger antibody-dependent cellular cytotoxicity (ADCC) and complement-dependent cytotoxicity (CDC) activities in comparison to the conventional anti-FOLR1 antibody.

High expression of indoleamine 2,3-dioxygenase 1 (IDOl) in ovarian cancer was previously reported, and known as one of the poor prognostic factors of patients with ovarian cancer (Gynecologic Oncology 2009;115:185-192). IDOl plays an important regulatory role in the immunosuppressive mechanisms, which is responsible for tumor’s escape from host immune surveillance (Nat Rev Cancer. 2009;9:445-52). It has been reported that tryptophan metabolites, such as kynurenine, induce NK cell death (J Exp Med. 2002;196:447-57), and weaken NK cell cytotoxicity by inhibiting the expression of NK cell receptros (Blood. 2006;108:4118-25). Therefore, there is a possibility that IDOl activity affects ADCC activity.

In fact, we demonstrated that the cytotoxicity of human peripheral blood mononuclear cells (PBMCs) induced by trastuzumab, rituximab, cetuximab, and mogamulizumab was decreased by IDOl activity of KATO-III cells in figures 3 to 8.

Therefore, in this study, we examined the effect of Compound A1 and Compound B1 on cytotoxicity of human PBMCs against ovarian cancer cells in the presence or absence of anti-FOLR1 antibody, Antibody C1.

11 MATERIALS AND METHODS

11.1 Materials

11.1.1 Anti-FOLR1 antibody

Antibody C1 (HuRA15-7CTAcc) was obtained as "HuRA15-7CTAcc antibody" described in examples of WO2014/087863. Antibody C1 is an anti-FOLR1 humanized and CDR-modified antibody made with the AccretaMab(R) technology.

11.1.2 IDO1 inhibitors

Compound A1 and Compound B1 are IDOl inhibitors. Compound A1 was obtained as Compound 64A of example 62 in WO2011/142316 and Compound B1 was obtained as "4-({2-[(aminosulfonyl)amino]ethyl}amino)-N-(3-bromo-4-fluorophenyl)-N’-hydroxy-1,2,5-oxadiazole-3-carboximidamide" of example 1 in WO2010/005958.

Compound A1 and B1 were dissolved in dimethyl sulfoxide (DMSO) at a concentration of 10 mmol/L as a stock solution. These solutions were kept under frozen condition until use. The stock solutions were diluted with culture medium [RPMI1640 (GIBCO) containing 10 vol% heat-inactivated fetal calf serum (FCS, GIBCO) and Penicillin-Streptomycin (GIBCO)] to adjust the concentration for the assay.

11.1.3 Human PBMCs

Frozen human PBMCs were purchased from Precision Bioservices (Lot 2500113169). After thawing, the PBMCs were washed with culture medium containing 1 mg/mL DNase (Roche) and used for the assay.

11.1.4 Cells and medium

Ovarian cancer cells, OVISE (JCRB 1043) and MCAS (JCRB0240) were obtained from JCRB, and ovarian cancer cells, SKOV3 (HTB-77) and OVCAR3 (HTB-161) were obtained from ATCC. These cells were cultured with the culture medium.

11.2 Method

11.2.1 Western blot

Ovarian cancer cells (OVISE, SKOV3, OVCAR3, or MCAS) were cultured by the culture medium with (50 or

100 ng/mL) or without IFNγ (R&D systems) for 4 days. Then, these cells were lysed by SDS sample buffer (Thermo) and boiled (100°C, 5 min). IDO1 or β-actin protein expression in each sample were detected by Western blot method using anti-IDOl antibody (clone 10.1, Millipore) followed by anti-mouse antibody- horseradish peroxidase (HRP, Zymed) or anti-β-actin antibody (rabbit polyclonal, abcam) followed by anti-rabbit antibody-HRP (DAKO), respectively. 11.2.2 Preparation of conditioned medium

SKOV3 cells ($1.0 \times 10^6$ cells) were cultured in 25 mL of the culture medium containing 50 ng/mL IFNγ (PeproTech) with 100 nmol/L IDOl inhibitor (Compound A1 or Compound B1) or DMSO for 3 days. The supernatants of each culture were used as the conditioned medium for pre-incubation of PBMCs.

The list of conditioned medium is described below.

- Conditioned medium DMSO: SKOV3 cells cultured with 50 ng/mL IFNγ and 0.1 vol% DMSO
- Conditioned medium Compound A1: SKOV3 cells cultured with 50 ng/mL IFNγ and 100 nmol/L Compound A1
- Conditioned medium Compound B1: SKOV3 cells cultured with 50 ng/mL IFNγ and 100 nmol/L Compound B1

11.2.3 Cytotoxicity assay

11.2.3.1 Preparation of target cells

SKOV3 cells were detached from culture flasks by TrypLE Express (GIBCO). The live cells were adjusted to $1.0 \times 10^6$cells by the ADCC assay medium [RPMI1640 without phenol red (GIBCO) with 10 vol% of dialyzed and heat-inactivated FCS(GIBCO)]. Then, the cells were labeled with approx. 3.7 MBq of $Na_2{}^{51}CrO_4$ (PerkinElmer) for over 1 hour at 37 °C in a $CO_2$ incubator. The cells were washed three times, and adjusted to $2.0 \times 10^5$ cells/mL ($1 \times 10^4$ cells/50 μL) as the target cells using the ADCC assay medium.

11.2.3.2 Preparation of effector cells

Human PBMCs were cultured in 25 mL of the conditioned medium for 7 days. Two point eight or $2.4 \times 10^7$ cells were pre-incubated in the conditioned medium Compound A1 or the conditioned medium Compound B1, respectively. After washed with the ADCC assay medium, the cells were adjusted to $5.0 \times 10^6$ cells/mL ($2.5 \times 10^5$ cells/50 μL) as the effector cells using the ADCC assay medium.

11.2.3.3 Preparation of antibody solution

Antibody C1 solution was prepared using the ADCC assay medium, and used at the final concentrations of 0.033, 0.33, 3.3, 33, and 333 ng/mL in the ADCC assay. 11.2.3.4 Cytotoxicity assay

The cytotoxicity was quantified by measurement of radioactivity in the supernatant of each assay well. Total volume of each well was 150 μL in a U-bottom 96-well plate.

Total, T spo, M, or Assay sample was defined and prepared as follows.

- Total: Target cell total radioactivity control. This sample was prepared by mixing 50 μL of target cell, 15 μL of detergent (10×Lysis Solution, Promega), and 85 μL of the ADCC assay medium.
- T spo: Target cell spontaneous radioactivity control. This sample was prepared by mixing 50 μL of target cells and 100 μL of the ADCC assay medium.
- M: Reference control for an ADCC assay medium. This sample was prepared as 150 μL of the ADCC assay medium.
- Assay sample: This sample was prepared by mixing 50 μL of target cells, effector cells, and antibody solution.

Each sample was prepared in triplicate. The sample plate was incubated for 4 h in a $CO_2$ incubator. After centrifugation of the plate, 50 μL of each supernatant was transferred into LumaPlate (PerkinElmer) and dried sufficiently. The radioactivity (cpm) was counted with a microplate scintillation counter (TopCount NXT, PerkinElmer).

The cytotoxicity (%) was calculated by the following equations using the cpm of each well. First of all, cpm correction value of each well was calculated by the following equation.

[Math.3]

$$\text{Cpm correction value of each well} = \text{Cpm of each well} - \text{Mean cpm of M}$$

Then, the cytotoxicity of each Assay sample was calculated by the following equation.

[Math.4]

$$\text{Cytotoxicity (\%)} = 100 \times \frac{\left(\begin{array}{c}\text{(Cpm correction value of Assay sample)}\\ -\text{(Mean cpm correction value of T spo)}\end{array}\right)}{\left(\begin{array}{c}\text{(Mean cpm correction value of Total)}\\ -\text{(Mean cpm correction value of T spo)}\end{array}\right)}$$

The cytotoxicity of each Assay sample was presented as mean + standard deviation (SD) of triplicate.

12 RESULTS AND DISCUSSION

12.1 Expression of IDO1 in ovarian cancer cells

We evaluated whether IDOI is induced in ovarian cancer cells by IFNγ stimulation using four types of ovarian cancer cells (OVISE, SKOV3, OVCAR3, and MCAS cells). As shown in Fig. 14, IDOI was detected in 50 or 100 ng/mL IFNγ-stimulated SKOV3, OVCAR3, and MCAS cells. In contrast, the expression of IDO1 was not observed in IFNγ-stimulated OVISE cells. All tested ovarian cancer cells did not express IDOI without IFNγ-stimulation.

12.2 Effect of Compound A1 or Compound B1 on cytotoxicity of PBMCs against SKOV3 cells

To evaluate the effect of IDOI inhibitor activity on cytotoxicity of PBMCs, the PBMCs were pre-incubated for 7 days in the conditioned medium of SKOV3 cells prepared as described in 9.2.2("Preparation of conditioned medium") Then, the cytotoxicity of these pre-incubated PBMCs against radiolabeled SKOV3 cells was evaluated with various concentrations of Antibody C1. As results, the cytotoxicity without Antibody C1 (the natural killing activity) of PBMCs pre-incubated in the conditioned medium Compound A1 or Compound B1 was higher than that in the conditioned medium DMSO (Figure 15A and Figure 16A). Furthermore, in the presence of Antibody C1, the cytotoxicity of PBMCs pre-incubated in the conditioned medium Compound A1 or Compound B1 was also higher than that in the conditioned medium DMSO (Figure 15B and Figure 16B).

In summary, we demonstrated that several ovarian cancer cells including SKOV3 cells expressed IDOI after IFNγ stimulation. Furthermore, the natural killing activity and Antibody C1- mediated cytotoxicity of the PBMCs were up-regulated by IDOI inhibitors, Compound A1 or Compound B1. Taken together, the combination of Antibody C1 and Compound A1 or Compound B1 was expected to exert synergistic cytotoxic effects against ovarian cancer cells.

TEST EXAMPLE 4

SUMMARY

In this study, we examined the effect of an indoleamine-2, 3-dioxygenase (IDOI) inhibitor, Compound A1, on the cytotoxicity of human peripheral blood mononuclear cells (PBMCs) in the absence or presence of an anti-human interleukin-3 receptor alpha chain (IL-3Rα) antibody, Antibody D1, against acute myeloid leukemia (AML) cells.

Using an IDOI expressing cells (KATO-III cells), we prepared conditioned medium. KATO-III cells were stimulated with IFN-γ in the presence or absence of Compound A1, and then the supernatant was recovered as conditioned medium for PBMCs pre-incubation. PBMC was used in this study. Seven days after the pre-incubation in the conditioned medium, the cytotoxicity of the PBMC against AML cells was measured. Clear up-regulation of the cytotoxicity by Compound A1 was observed; the PBMCs incubated in the Compound A1-containing conditioned medium induced higher cytotoxicity than that in the DMSO-containing medium in the absence or presence of Antibody D1.

13 INTRODUCTION

Antibody D1 is a non-fucosylated fully human monoclonal antibody against human interleukin-3 receptor alpha chain (IL-3Rα).

Compound A1 is a small molecule inhibitor of indoleamine-2, 3-dioxygenase (IDO) 1.

In this study, we examined the effect of Compound A1 on the cytotoxicity of PBMCs against acute myeloid leukemia (AML) cells in the presence or absence of Antibody D1.

## 14 MATERIALS AND METHODS

### 14.1 Materials

#### 14.1.1 Antibody D1

Antibody D1 (19.59 mg/mL) was obtained as "New102 antibody" described in the exapmle in WO2010/126066.

#### 14.1.2 IDO1 inhibitors

Compound A1 was obtained as Compound 64A of Example 62 in WO2011/142316, and was dissolved in dimethyl sulfoxide (DMSO) at a concentration of 10 mmol/L as a stock solution. The solution was kept under frozen condition until use. The stock solution was diluted with culture medium [RPMI1640 containing 10 vol% heat-inactivated fetal calf serum and 1 vol% of Penicillin-Streptomycin solution] to adjust the concentration for the assay.

#### 14.1.3 Human PBMCs

Frozen PBMCs were purchased from Precision Bioservices (lot# 13096) and Allcells (lot# A3951). After thawing, the PBMCs were washed with the culture medium and used for the assay.

#### 14.1.4 Cells and medium

Human gastric cancer cells, KATO-III (86093004) was obtained from DS Pharma Biomedical Co. Ltd. and human AML cells, KG-1 (CRL-8031), was obtained from ATCC. The cells were cultured with the culture medium.

### 14.2 Method

#### 14.2.1 Preparation of conditioned medium

KATO-III cells ($1.25\times10^6$ cells) were cultured in 25 mL of the culture medium (RPMI1640 medium containing 10 vol% FBS) containing 50 ng/mL IFN-γ (PeproTech) and 100 nmol/L Compound A1 or DMSO for 3 days. The supernatant of each culture was used as the conditioned medium for pre-incubation of PBMCs.

The conditioned mediums are as follows.

- Conditioned medium DMSO: KATO-III cells cultured with 50 ng/mL IFN-γ and 0.1 vol% DMSO
- Conditioned medium Compound A1: KATO-III cells cultured with 50 ng/mL IFN-γ and 100 nmol/L Compound A1

#### 14.2.2 Cytotoxicity assay

##### 14.2.2.1 Preparation of target cells

KG-1 cells ($1\times10^6$ cells) were labeled with approx. 3.7 MBq of $Na_2{}^{51}CrO_4$ (PerkinElmer) for 1 hour at 37°C in a $CO_2$ incubator. The cells were washed twice, and adjusted to $1.0\times10^5$ cells/mL ($0.5\times10^4$ cells/50 μL) as the target cells using the assay medium.

##### 14.2.2.2 Preparation of effector cells

Two lots of PBMCs were cultured in the conditioned medium for 7 days at $5\times10^5$ cells/mL. After washed with the assay medium, the cells were adjusted to $2.5\times10^6$ cells/mL ($12.5\times10^4$ cells/50 μL) as the effector cells using the assay medium.

##### 14.2.2.3 Preparation of antibody solution

Antibody D1 solution was diluted with the assay medium to concentrations of 0, 0.3, 3, or 30 μg/mL.

##### 14.2.2.4 Cytotoxicity assay

PBMC was used for the cytotoxicity assay. The cytotoxicity was quantified by measurement of radioactivity in

the supernatant of each assay well. Total volume of each well was 150 μL in a V-bottom 96-well plate.

Total, T spo, M, or Assay sample was defined and prepared as follows.

- Total: Target cell total radioactivity control. This sample was prepared by mixing 50 μL of target cell and 100 μL of 1 vol% NP-40 (diluted in the assay medium).
- T spo: Target cell spontaneous radioactivity control. This sample was prepared by mixing 50 μL of target cells and 100 μL of the assay medium.
- Assay sample: This sample was prepared by mixing 50 μL of target cells, effector cells, and antibody solution.

Final concentration of the Antibody D1 in the assay samples was 0, 0.1, 1, and 10 μg/mL.

Each sample was prepared in triplicate. After brief centrifugation, the sample plate was incubated for 4 h in a $CO_2$ incubator. After centrifugation of the plate, 50 μL of each supernatant was transferred into LumaPlate (PerkinElmer) and dried sufficiently. The radioactivity (cpm) was counted with a microplate scintillation counter (TopCount NXT, PerkinElmer).

The cytotoxicity (%) was calculated by the following equation.

[Math.5]

$$\text{Cytotoxicity (\%)} = 100 \times \frac{\text{cpm value of the assay sample} - \text{mean cpm value of T spo}}{\text{mean cpm value of Total} - \text{mean cpm value of T spo}}$$

The cytotoxicity of each assay sample was presented as mean +/- standard deviation (SD) of triplicate.

15 RESULTS AND DISCUSSION

To evaluate the effect of Compound A1 on the cytotoxicity of PBMCs, PBMC (lot# A3951) is pre-incubated for 7 days in the conditioned medium of KATO-III cells prepared as described in 9.2.2. After the incubation, the cytotoxicity of the pre-incubated PBMC against radiolabeled KG-1 cells was evaluated in the absence or presence of various concentrations of Antibody D1.

Clear up-regulation of the cytotoxicity by Compound A1 was observed; the PBMC incubated in the Compound A1-containing conditioned medium induced higher cytotoxicity than that in the DMSO-containing medium in the absence or presence of Antibody D1 (Figure 17).

TEST EXAMPLE 5

16 Summary

In this study, we examined the effect of an indoleamine-2, 3-dioxygenase (IDOI) inhibitor, Compound A1, on the cytotoxicity of human peripheral blood mononuclear cells (PBMCs) in the absence or presence of an anti-human T cell immunoglobulin and mucin domain 3 (TIM-3) antibody, Antibody E1, against TIM-3-transfected acute myeloid leukemia (AML) cells.

Using an IDOI expressing cells (KATO-III cells), we prepared conditioned medium. KATO-III cells were stimulated with IFN-γ in the presence or absence of Compound A1, and then the supernatant was recovered as conditioned medium for PBMCs pre-incubation. Seven days after the pre-incubation in the conditioned medium, the cytotoxicity of the PBMC against TIM-3-transfected AML cells was measured. Clear up-regulation of the cytotoxicity by Compound A1 was observed; the PBMCs incubated in the Compound A1-containing conditioned medium induced higher cytotoxicity than that in the DMSO-containing medium in the absence or presence of Antibody E1.

17 INTRODUCTION

Antibody E1 is a non-fucosylated fully human monoclonal antibody against human T cell immunoglobulin and mucin domain 3 (TIM-3).

Compound A1 is a small molecule inhibitor of indoleamine-2, 3-dioxygenase (IDO) 1.

In this study, we examined the effect of Compound A1 on the cytotoxicity of PBMCs against TIM-3-transfected acute myeloid leukemia (AML) cells in the presence or absence of Antibody E1.

18 MATERIALS AND METHODS

18.1 Materials

18.1.1 Antibody E1

Antibody E1 (82.31 mg/mL) was obtained as "4545 antibody" described in examples of WO2011/155607.

18.1.2 IDO1 inhibitors

Compound A1 was obtained as Compound 64A of Example 62 in WO2011/142316, and was dissolved in dimethyl sulfoxide (DMSO) at a concentration of 10 mmol/L as a stock solution. The solution was kept under frozen condition until use. The stock solution was diluted with culture medium [RPMI1640 containing 10 vol% heat-inactivated fetal calf serum and 1 vol% of Penicillin-Streptomycin solution] to adjust the concentration for the assay.

18.1.3 Human PBMCs

Frozen PBMCs were purchased from Precision Bioservices (lot# 13096). After thawing, the PBMCs were washed with the culture medium and used for the assay. 18.1.4 Cells and medium

Human gastric cancer cells, KATO-III (86093004) was obtained from DS pharma biomedical Co., Ltd. EoL-1 (RCB0641) was obtained from Cell Engineering Division of Riken Bio Resource Center. Human TIM-3 genes were transfected to EoL-1 cells to give EoL-1/human TIM-3 cells,. The cells were cultured with the culture medium. 18.2 Method

18.2.1 Preparation of conditioned medium

KATO-III cells ($1.25\times10^6$cells) were cultured in 25 mL of the culture medium (RPMI1640 medium containing 10 vol% FBS) containing 50 ng/mL IFN-$\gamma$ (PeproTech) and 100 nmol/L Compound A1 or DMSO for 3 days. The supernatant of each culture was used as the conditioned medium for pre-incubation of PBMCs.

The conditioned mediums are as follows.

- Conditioned medium DMSO: KATO-III cells cultured with 50 ng/mL IFN-$\gamma$ and 0.1 vol% DMSO
- Conditioned medium Compound A1: KATO-III cells cultured with 50 ng/mL IFN-$\gamma$ and 100 nmol/L Compound A1

18.2.2 Cytotoxicity assay

18.2.2.1 Preparation of target cells

EoL-1/human TIM-3 cells ($1\times10^6$ cells) were labeled with approx. 3.7 MBq of $Na_2{}^{51}CrO_4$ (PerkinElmer) for 1 hour at 37 °C in a $CO_2$ incubator. The cells were washed twice, and adjusted to $1.0\times10^5$ cells/mL ($0.5\times10^4$ cells/50 $\mu$L) as the target cells using the assay medium.

18.2.2.2 Preparation of effector cells

Human PBMCs were cultured in the conditioned medium for 7 days at $5\times10^5$ cells/ mL. After washed with the assay medium, the cells were adjusted to $2.5\times10^6$ cells/mL ($12.5\times10^4$ cells/50 $\mu$L) as the effector cells using the assay medium.

18.2.2.3 Preparation of antibody solution

Antibody E1 solution was diluted with the assay medium to concentrations of 0, 0.3, 3, or 30 $\mu$g/mL.

18.2.2.4 Cytotoxicity assay

The cytotoxicity was quantified by measurement of radioactivity in the supernatant of each assay well. Total volume of each well was 150 $\mu$L in a V-bottom 96-well plate.

Total, T spo, M, or Assay sample was defined and prepared as follows.

- Total: Target cell total radioactivity control. This sample was prepared by mixing 50 $\mu$L of target cell and 100 $\mu$L of

1 vol% NP-40 (diluted in the assay medium).

- T spo: Target cell spontaneous radioactivity control. This sample was prepared by mixing 50 μL of target cells and 100 μL of the assay medium.
- Assay sample: This sample was prepared by mixing 50 μL of target cells, effector cells, and antibody solution.

Final concentration of the Antibody E1 in the assay samples was 0, 0.1, 1, and 10 μg/ mL

Each sample was prepared in triplicate. After brief centrifugation, the sample plate was incubated for 4 h in a $CO_2$ incubator. After centrifugation of the plate, 50 μL of each supernatant was transferred into LumaPlate (PerkinElmer) and dried sufficiently. The radioactivity (cpm) was counted with a microplate scintillation counter (TopCount NXT, PerkinElmer).

The cytotoxicity (%) was calculated by the following equation.

[Math.6]

$$\text{Cytotoxicity (\%)} = 100 \times \frac{\text{cpm value of the assay sample} - \text{mean cpm value of T spo}}{\text{mean cpm value of Total} - \text{mean cpm value of T spo}}$$

The cytotoxicity of each assay sample was presented as mean ± standard deviation (SD) of triplicate.

19 RESULTS AND DISCUSSION

To evaluate the effect of Compound A1 on the cytotoxicity of PBMCs, PBMCs were pre-incubated for 7 days in the conditioned medium of KATO-III cells prepared as described in 9.2.2. After the incubation, the cytotoxicity of the pre-incubated PBMCs against radiolabeled EoL-1/human TIM-3 cells was evaluated in the absence or presence of various concentrations of Antibody E1.

Clear up-regulation of the cytotoxicity by Compound A1 was observed; the PBMCs incubated in the Compound A1-containing conditioned medium induced higher cytotoxicity than that in the DMSO-containing medium in the absence or presence of Antibody E1 (Figure 18).

**Sequence Listing Free Text**

SEQ ID NO: 1: The amino acid sequence of Antibody C1 HCDR1
SEQ ID NO: 2: The amino acid sequence of Antibody C1 HCDR2
SEQ ID NO: 3: The amino acid sequence of Antibody C1 HCDR3
SEQ ID NO: 4: The amino acid sequence of Antibody C1 LCDR1
SEQ ID NO: 5: The amino acid sequence of Antibody C1 LCDR2
SEQ ID NO: 6: The amino acid sequence of Antibody C1 LCDR3
SEQ ID NO: 7: The amino acid sequence of Antibody C1 H chain
SEQ ID NO: 8: The amino acid sequence of Antibody C1 L chain
SEQ ID NO: 9: The amino acid sequence of Antibody D1-1 HCDR1
SEQ ID NO: 10: The amino acid sequence of Antibody D1-1 HCDR2
SEQ ID NO: 11: The amino acid sequence of Antibody D1-1 HCDR3
SEQ ID NO: 12: The amino acid sequence of Antibody D1-2 HCDR1
SEQ ID NO: 13: The amino acid sequence of Antibody D1-2 HCDR2
SEQ ID NO: 14: The amino acid sequence of Antibody D1-2 HCDR3
SEQ ID NO: 15: The amino acid sequence of Antibody D1-3 HCDR1
SEQ ID NO: 16: The amino acid sequence of Antibody D1-3 HCDR2
SEQ ID NO: 17: The amino acid sequence of Antibody D1-3 HCDR3
SEQ ID NO: 18: The amino acid sequence of Antibody D1-4 HCDR1
SEQ ID NO: 19: The amino acid sequence of Antibody D1-4 HCDR2
SEQ ID NO: 20: The amino acid sequence of Antibody D1-4 HCDR3
SEQ ID NO: 21: The amino acid sequence of Antibody D1-5 HCDR1
SEQ ID NO: 22: The amino acid sequence of Antibody D1-5 HCDR2
SEQ ID NO: 23: The amino acid sequence of Antibody D1-5 HCDR3
SEQ ID NO: 24: The amino acid sequence of Antibody D1-1 LCDR1

SEQ ID NO: 25: The amino acid sequence of Antibody D1-1 LCDR2
SEQ ID NO: 26: The amino acid sequence of Antibody D1-1 LCDR3
SEQ ID NO: 27: The amino acid sequence of Antibody D1-2 LCDR1
SEQ ID NO: 28: The amino acid sequence of Antibody D1-2 LCDR2
SEQ ID NO: 29: The amino acid sequence of Antibody D1-2 LCDR3
SEQ ID NO: 30: The amino acid sequence of Antibody D1-3 LCDR1
SEQ ID NO: 31: The amino acid sequence of Antibody D1-3 LCDR2
SEQ ID NO: 32: The amino acid sequence of Antibody D1-3 LCDR3
SEQ ID NO: 33: The amino acid sequence of Antibody D1-4 LCDR1
SEQ ID NO: 34: The amino acid sequence of Antibody D1-4 LCDR2
SEQ ID NO: 35: The amino acid sequence of Antibody D1-4 LCDR3
SEQ ID NO: 36: The amino acid sequence of Antibody D1-5 LCDR1
SEQ ID NO: 37: The amino acid sequence of Antibody D1-5 LCDR2
SEQ ID NO: 38: The amino acid sequence of Antibody D1-5 LCDR3
SEQ ID NO: 39: The amino acid sequence of Antibody E1-1 HCDR1
SEQ ID NO: 40: The amino acid sequence of Antibody E1-1 HCDR2
SEQ ID NO: 41: The amino acid sequence of Antibody E1-1 HCDR3
SEQ ID NO: 42: The amino acid sequence of Antibody E1-1 LCDR1
SEQ ID NO: 43: The amino acid sequence of Antibody E1-1 LCDR2
SEQ ID NO: 44: The amino acid sequence of Antibody E1-1 LCDR3
SEQ ID NO: 45: The amino acid sequence of Antibody E1-2 HCDR1
SEQ ID NO: 46: The amino acid sequence of Antibody E1-2 HCDR2
SEQ ID NO: 47: The amino acid sequence of Antibody E1-2 HCDR3
SEQ ID NO: 48: The amino acid sequence of Antibody E1-2 LCDR1
SEQ ID NO: 49: The amino acid sequence of Antibody E1-2 LCDR2
SEQ ID NO: 50: The amino acid sequence of Antibody E1-2 LCDR3
SEQ ID NO: 51: The amino acid sequence of Antibody E1-3 HCDR1
SEQ ID NO: 52: The amino acid sequence of Antibody E1-3 HCDR2
SEQ ID NO: 53: The amino acid sequence of Antibody E1-3 HCDR3
SEQ ID NO: 54: The amino acid sequence of Antibody E1-3 LCDR1
SEQ ID NO: 55: The amino acid sequence of Antibody E1-3 LCDR2
SEQ ID NO: 56: The amino acid sequence of Antibody E1-3 LCDR3
SEQ ID NO: 57: The amino acid sequence of Antibody E1-4 VH
SEQ ID NO: 58: The amino acid sequence of Antibody E1-4 VL
SEQ ID NO: 59: The amino acid sequence of Antibody E1-5 VH
SEQ ID NO: 60: The amino acid sequence of Antibody E1-5 VL

**Furthermore, the present invention relates to the following items:**

**[ Item 1 ]** A method for treating a tumor comprising administering an effective amount of an antibody which specifically binds to human CC chemokine receptor 4, human epidermal growth factor receptor 2, human CD20, or epidermal growth factor receptor, and an indoleamine 2,3-dioxygenase inhibitor to a human in need thereof.

**[Item 2 ]** The method according to **item** 1, wherein the indoleamine 2,3-dioxygenase inhibitor is Compound (I) represented by formula (I)

[Chem.1]

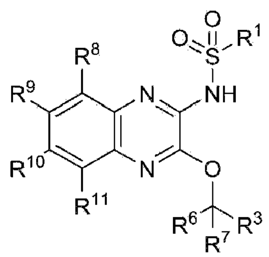

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.2]

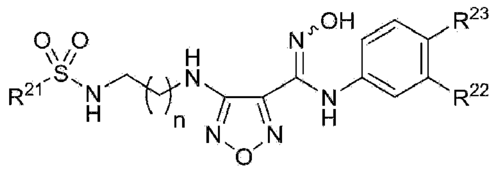

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.
[ **Item 3** ] The method according to **item** 1 or 2, wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.
[ **Item 4** ] The method according to any one of **items** 1 to 3, whereinthe antibody and the indoleamine 2,3-dioxygenase inhibitor are administered simultaneously or sequentially.
**[ Item 5 ]** The method according to any one of **items** 1 to 4, wherein the tumor is a tumor which expresses human CC chemokine receptor 4, human epidermal growth factor receptor 2, human CD20, or epidermal growth factor receptor.
**[ Item 6 ]** The method according to **item** 5, wherein the tumor which expresses human CC chemokine receptor 4 is T cell lymphoma.
**[ Item 7 ]** The method according to **item** 5, wherein the tumor which expresses human CC chemokine receptor 4 is peripheral T cell lymphoma, cutaneous T cell lymphoma or adult T cell leukemic lymphoma.
**[ Item 8 ]** The method according to **item** 5, wherein the tumor which expresses human epidermal growth factor receptor 2 is breast cancer, gastric cancer, ovarian cancer, osteosarcoma, or endometrial cancer.
**[ Item 9 ]** The method according to **item** 5, wherein the tumor which expresses human epidermal growth factor receptor 2 is breast cancer.
**[ Item 10 ]** The method according to **item** 5, wherein the tumor which expresses human CD20 is chronic leukemia, or non-Hodgkin's lymphoma.
**[ Item 11 ]** The method according to **item** 10, wherein the chronic leukemia is chronic lymphocytic leukemia.
**[ Item 12 ]** The method according to **item** 10, wherein the non-Hodgkin's lymphoma is B cell lymphoma.
**[ Item 13 ]** The method according to **item** 12, wherein the B cell lymphoma is mantle cell lymphoma, diffuse large B cell lymphoma or Burkitt's lymphoma.
**[ Item 14 ]** The method according to **item** 12, wherein the B cell lymphoma is Burkitt's lymphoma.
**[ Item 15 ]** The method according to **item** 5, wherein the tumor which expresses epidermal growth factor receptor is colon cancer, head and neck cancer, gastric cancer, hepatic cancer.
**[ Item 16 ]** A method for suppressing decreasing antibody dependent cellular cytotoxicity activity of an antibody which specifically binds to human CC chemokine receptor 4, human epidermal growth factor receptor 2, human CD20, or epidermal growth factor receptor comprising administering an indoleamine 2,3-dioxygenase inhibitor.
[ **Ite**m **17 ]** The method according to **item** 16, wherein the indoleamine 2,3-dioxygenase inhibitor is Compound (I)

represented by formula (I)

[Chem.3]

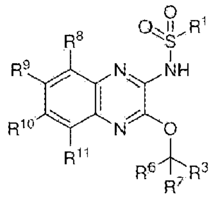

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.4]

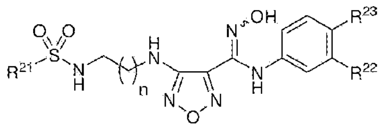

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.

**[ Item 18]** A pharmaceutical composition for use in administering an effective amount of an antibody which specifically binds to human CC chemokine receptor 4, human epidermal growth factor receptor 2, human CD20, or epidermal growth factor receptor, and an indoleamine 2,3-dioxygenase inhibitor.

**[ Item 19 ]** The pharmaceutical composition according to **item** 18, wherein the indoleamine 2,3-dioxygenase inhibitor is Compound (I) represented by formula (I)

[Chem.5]

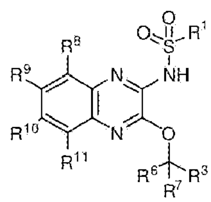

(I)

wherein

$R^6$ and $R^7$ may be the same or different, and each represents a hydrogen atom, or optionally substituted lower

alkyl,

$R^8$, $R^9$, $R^{10}$, and $R^{11}$ may be the same or different, and each represents a hydrogen atom, halogen, cyano, or lower alkyl,

$R^1$ represents lower alkyl which may be substituted with lower alkoxy, and

$R^3$ represents an optionally substituted aromatic heterocyclic group,

or a pharmaceutically acceptable salt thereof,

or Compound (II) represented by formula (II)

[Chem.6]

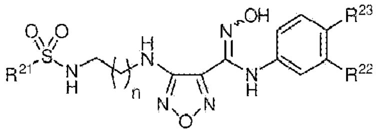

(II)

wherein

$R^{21}$ represents amino or methyl,

$R^{22}$ represents halogen, cyano, trifluoromethyl, or lower alkyl,

$R^{23}$ represents a hydrogen atom, or halogen, and

n represents 1 or 2,

or a pharmaceutically acceptable salt thereof.

**[ Item 20 ]** The pharmaceutical composition according to **item** 18 or 19, wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.

**[ Item 21 ]** The pharmaceutical composition according to any one of **items** 18 to 20, which comprises simultaneously or sequentially administering the antibody and the indoleamine 2,3-dioxygenase inhibitor.

**[ Item 22 ]** The pharmaceutical composition according to any one of **items** 18 to 21, wherein the tumor is a tumor which expresses human CC chemokine receptor 4, human epidermal growth factor receptor 2, human CD20, or epidermal growth factor receptor.

**[ Item 23 ]** The pharmaceutical composition according to **item** 22, wherein the tumor which expresses human CC chemokine receptor 4 is T cell lymphoma.

**[ Item 24 ]** The pharmaceutical composition according to **item** 22, wherein the tumor which expresses human CC chemokine receptor 4 is peripheral T cell lymphoma, cutaneous T cell lymphoma or adult T cell leukemic lymphoma.

**[ Item 25 ]** The pharmaceutical composition according to **item** 22, wherein the tumor which expresses human epidermal growth factor receptor 2 is breast cancer, gastric cancer, ovarian cancer, osteosarcoma, or endometrial cancer.

**[ Item 26** ] The pharmaceutical composition according to **item** 22, wherein the tumor which expresses human epidermal growth factor receptor 2 is breast cancer.

**[ Item 27 ]** The pharmaceutical composition according to **item** 22, wherein the tumor which expresses human CD20 is chronic leukemia, or non-Hodgkin's lymphoma.

**[ Item 28 ]** The pharmaceutical composition according to **item** 27, wherein the chronic leukemia is chronic lymphocytic leukemia.

[ **Item 29** ] The pharmaceutical composition according to **item** 27, wherein the non-Hodgkin's lymphoma is B cell lymphoma.

**[ Item 30 ]** The pharmaceutical composition according to **item** 29, wherein the B cell lymphoma is mantle cell lymphoma, diffuse large B cell lymphoma or Burkitt's lymphoma.

**[ Item 31** ] The pharmaceutical composition according to **item** 29, wherein the B cell lymphoma is Burkitt's lymphoma.

**[ Item 32 ]** The pharmaceutical composition according to **item** 22, wherein the tumor which expresses epidermal growth factor receptor is colon cancer, head and neck cancer, gastric cancer, hepatic cancer.

**[ Item 33 ]** A combination of an indoleamine 2,3-dioxygenase inhibitor and an antibody which specifically binds to human CC chemokine receptor 4, human epidermal growth factor receptor 2, human CD20, or epidermal growth factor receptor for use in the treatment of a tumor.

**[ Item 34 ]** The combination of an indoleamine 2,3-dioxygenase inhibitor and an antibody according to **item** 33, wherein the indoleamine 2,3-dioxygenase inhibitor is Compound (I) represented by formula (I)

[Chem.7]

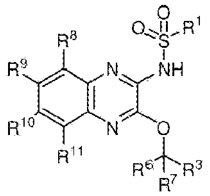

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.8]

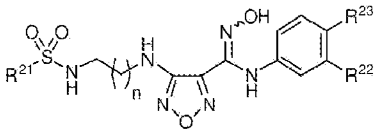

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.

[ **Item 35** ] The combination of an indoleamine 2,3-dioxygenase inhibitor and an antibody according to **item** 33 or 34, wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.

**[ Item 36** ] The combination of an indoleamine 2,3-dioxygenase inhibitor and an antibody according to any one of **items** 33 to 35, wherein the antibody and the indoleamine 2,3-dioxygenase inhibitor are administered simultaneously or sequentially.

**[ Item 37** ] The combination of an indoleamine 2,3-dioxygenase inhibitor and an antibody according to any one of **items** 33 to 36, wherein the tumor is a tumor which expresses human CC chemokine receptor 4, human epidermal growth factor receptor 2, human CD20, or epidermal growth factor receptor.

**[ Item 38** ] The combination of an indoleamine 2,3-dioxygenase inhibitor and an antibody according to **item** 37, wherein the tumor which expresses human CC chemokine receptor 4 is T cell lymphoma.

**[ Item 39** ] The combination of an indoleamine 2,3-dioxygenase inhibitor and an antibody according to **item** 37, wherein the tumor which expresses human CC chemokine receptor 4 is peripheral T cell lymphoma, cutaneous T cell lymphoma or adult T cell leukemic lymphoma.

**[ [ Item 40 ]** The combination of an indoleamine 2,3-dioxygenase inhibitor and an antibody according to **item** 37, wherein the tumor which expresses human epidermal growth factor receptor 2 is breast cancer, gastric cancer, ovarian cancer, osteosarcoma or endometrial cancer.

[ **Item 41 ]** The combination of an indoleamine 2,3-dioxygenase inhibitor and an antibody according to **item** 37, wherein the tumor which expresses human epidermal growth factor receptor 2 is breast cancer.

**[ Item 42 ]** The combination of an indoleamine 2,3-dioxygenase inhibitor and an antibody according to **item** 37, wherein the tumor which expresses human CD20 is chronic leukemia or non-Hodgkin's lymphoma.

[ **Item 43** ] The combination of an indoleamine 2,3-dioxygenase inhibitor and an antibody according to **item** 42, wherein the chronic leukemia is chronic lymphocytic leukemia.

[ **Item 44** ] The combination of an indoleamine 2,3-dioxygenase inhibitor and an antibody according to **item** 42, wherein the non-Hodgkin's lymphoma is B cell lymphoma.

[ **Item 45** ] The combination of an indoleamine 2,3-dioxygenase inhibitor and an antibody according to **item** 44, wherein the B cell lymphoma is mantle cell lymphoma, diffuse large B cell lymphoma or Burkitt's lymphoma.

**[ Item 46 ]** The combination of an indoleamine 2,3-dioxygenase inhibitor and an antibody according to **item** 44, wherein the B cell lymphoma is Burkitt's lymphoma.

[ **Item 47** ] The combination of an indoleamine 2,3-dioxygenase inhibitor and an antibody according to **item** 37, wherein the tumor which expresses epidermal growth factor receptor is colon cancer, head and neck cancer, gastric cancer or hepatic cancer.

[ **Item 48 ]** An indoleamine 2,3-dioxygenase inhibitor for use in suppressing decreasing antibody dependent cellular cytotoxicity activity of an antibody which specifically binds to human CC chemokine receptor 4, human epidermal growth factor receptor 2, human CD20, or epidermal growth factor receptor.

**[ Item 49 ]** The indoleamine 2,3-dioxygenase inhibitor according to **item** 48, wherein the indoleamine 2,3-dioxygenase inhibitor is Compound (I) represented by formula (I)

[Chem.9]

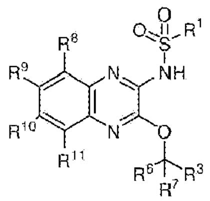

(I)

wherein

- $R^6$ and $R^7$ may be the same or different, and each represents a hydrogen atom, or optionally substituted lower alkyl,
- $R^8$, $R^9$, $R^{10}$, and $R^{11}$ may be the same or different, and each represents a hydrogen atom, halogen, cyano, or lower alkyl,
- $R^1$ represents lower alkyl which may be substituted with lower alkoxy, and
- $R^3$ represents an optionally substituted aromatic heterocyclic group,

or a pharmaceutically acceptable salt thereof,
or Compound (II) represented by formula (II)

[Chem.10]

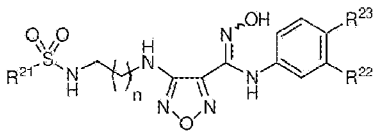

(II)

wherein

- $R^{21}$ represents amino or methyl,
- $R^{22}$ represents halogen, cyano, trifluoromethyl, or lower alkyl,
- $R^{23}$ represents a hydrogen atom, or halogen, and
- n represents 1 or 2,

or a pharmaceutically acceptable salt thereof.

**[ Item 50 ]** A therapeutic agent for a tumor, comprising an indoleamine 2,3-dioxygenase inhibitor as an active

ingredient, wherein the therapeutic agent is administered in combination with an effective amount of an antibody which specifically binds to human CC chemokine receptor 4, human epidermal growth factor receptor 2, human CD20, or epidermal growth factor receptor.

**[ Item 51 ]** The therapeutic agent for a tumor according to item 50, wherein the indoleamine 2,3-dioxygenase inhibitor is Compound (I) represented by formula (I)

[Chem.11]

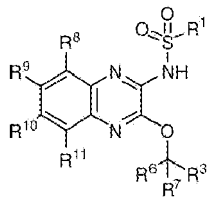

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.12]

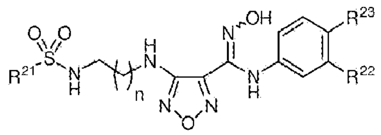

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.

**[ Item 52 ]** The therapeutic agent for a tumor according to **item** 50 or 51, wherein the antibody which specifically binds to human CC chemokine receptor 4, human epidermal growth factor receptor 2, human CD20, or epidermal growth factor is mogamulizumab, trastuzumab, rituximab or cetuximab, respectively.

[ **Item 53** ] The therapeutic agent for a tumor according to any one of **items** 50 to 52, wherein the therapeutic agent and the antibody are administered simultaneously or sequentially.

**[ Item 54** ] The therapeutic agent for a tumor according to any one of **items** 50 to 53, wherein the tumor is a tumor which expresses human CC chemokine receptor 4, human epidermal growth factor receptor 2, human CD20, or epidermal growth factor receptor.

[ **Item 55** ] The therapeutic agent for a tumor according to **item** 54, wherein the tumor which expresses human CC chemokine receptor 4 is T cell lymphoma.

[ **Item 56** ] The therapeutic agent for a tumor according to **item** 54, wherein the tumor which expresses human CC chemokine receptor 4 is peripheral T cell lymphoma, cutaneous T cell lymphoma or adult T cell leukemic lymphoma.

**[ Item 57 ]** The therapeutic agent for a tumor according to **item** 54, wherein the tumor which expresses human epidermal growth factor receptor 2 is breast cancer, gastric cancer, ovarian cancer, osteosarcoma, or endometrial

cancer.

[ **Item 58** ] The therapeutic agent for a tumor according to **item** 54, wherein the tumor which expresses human epidermal growth factor receptor 2 is breast cancer.

[ **Item 59** ] The therapeutic agent for a tumor according to **item** 54, wherein the tumor which expresses human CD20 is chronic leukemia, or non-Hodgkin's lymphoma.

**[ Item 60** ] The therapeutic agent for a tumor according to **item** 59, wherein the chronic leukemia is chronic lymphocytic leukemia.

**[ Item 61 ]** The therapeutic agent for a tumor according to **item** 59, wherein the non-Hodgkin's lymphoma is B cell lymphoma.

**[ Item 62 ]** The therapeutic agent for a tumor according to **item** 61, wherein the B cell lymphoma is mantle cell lymphoma, diffuse large B cell lymphoma or Burkitt's lymphoma.

**[ Item 63 ]** The therapeutic agent for a tumor according to **item** 61, wherein the B cell lymphoma is Burkitt's lymphoma.

**[ Item 64** ] The therapeutic agent for a tumor according to **item** 54, wherein the tumor which expresses epidermal growth factor receptor is colon cancer, head and neck cancer, gastric cancer, or hepatic cancer.

**[ Item 65** ] A suppressing agent for decreasing antibody dependent cellular cytotoxicity activity of an antibody which specifically binds to human CC chemokine receptor 4, human epidermal growth factor receptor 2, human CD20, or epidermal growth factor receptor, comprising an indoleamine 2,3-dioxygenase inhibitor as an active ingredient.

**[ Item 66 ]** The suppressing agent according to **item** 65, wherein the indoleamine 2,3-dioxygenase inhibitor is Compound (I) represented by formula (I)

[Chem.13]

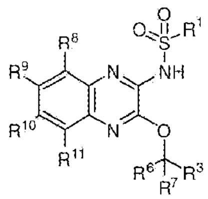

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.14]

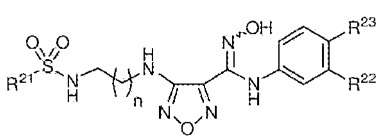

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.

[ **Item 67** ] The method according to **item** 16 or 17, wherein the antibody and the indoleamine 2,3-dioxygenase

inhibitor are administered simultaneously or sequentially.

**[ Item 68** ] The method according to **item** 16 or 17, wherein the antibody which specifically binds to human CC chemokine receptor 4, human epidermal growth factor receptor 2, human CD20, or epidermal growth factor is mogamulizumab, trastuzumab, rituximab or cetuximab, respectively.

**[ Item 69** ] The indoleamine 2,3-dioxygenase inhibitor according to **item** 48 or 49, wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.

**[ Item 70** ] A therapeutic agent for a tumor, comprising an antibody which specifically binds to human CC chemokine receptor 4, human epidermal growth factor receptor 2, human CD20, or epidermal growth factor receptor as an active ingredient, wherein the therapeutic agent is administered in combination with an effective amount of an indoleamine 2,3-dioxygenase inhibitor.

**[ Item 71** ] The therapeutic agent for a tumor according to **item** 70, wherein the indoleamine 2,3-dioxygenase inhibitor is Compound (I) represented by formula (I)

[Chem.15]

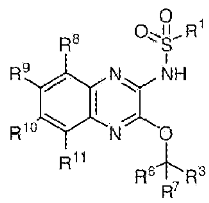

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.16]

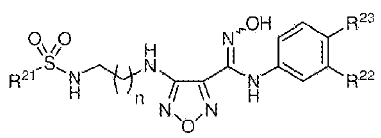

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.

**[ Item 72 ]** The therapeutic agent for a tumor according to **item** 70 or 71, wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.

[ **Item 73** ] The therapeutic agent for a tumor according to any one of **items** 70 to 72, wherein the therapeutic agent and the indoleamine 2,3-dioxygenase inhibitor are administered simultaneously or sequentially.

**[ Item 74 ]** The therapeutic agent for a tumor according to any one of **items** 70 to 73, wherein the tumor is a tumor which expresses human CC chemokine receptor 4, human epidermal growth factor receptor 2, human CD20, or

epidermal growth factor receptor.

**[ Item 75** ] The therapeutic agent for a tumor according to **item** 74, wherein the tumor which expresses human CC chemokine receptor 4 is T cell lymphoma.

**[ item 76 ]** The therapeutic agent for a tumor according to **item** 74, wherein the tumor which expresses human CC chemokine receptor 4 is peripheral T cell lymphoma, cutaneous T cell lymphoma or adult T cell leukemic lymphoma.

**[ Item 77 ]** The therapeutic agent for a tumor according to **item** 74, wherein the tumor which expresses human epidermal growth factor receptor 2 is breast cancer, gastric cancer, ovarian cancer, osteosarcoma, or endometrial cancer.

**[ Item 78 ]** The therapeutic agent for a tumor according to **item** 74, wherein the tumor which expresses human epidermal growth factor receptor 2 is breast cancer.

**[ Item 79** ] The therapeutic agent for a tumor according to **item** 74, wherein the tumor which expresses human CD20 is chronic leukemia, or non-Hodgkin's lymphoma.

**[ Item 80 ]** The therapeutic agent for a tumor according to **item** 79, wherein the chronic leukemia is chronic lymphocytic leukemia.

**[ Item 81** ] The therapeutic agent for a tumor according to **item** 79, wherein the non-Hodgkin's lymphoma is B cell lymphoma.

**[ Item 82** ] The therapeutic agent for a tumor according to **item** 81, wherein the B cell lymphoma is mantle cell lymphoma, diffuse large B cell lymphoma or Burkitt's lymphoma.

**[ Item 83 ]** The therapeutic agent for a tumor according to **item** 81, wherein the B cell lymphoma is Burkitt's lymphoma.

**[ Item 84 ]** The therapeutic agent for a tumor according to **item** 74, wherein the tumor which expresses epidermal growth factor receptor is colon cancer, head and neck cancer, gastric cancer, or hepatic cancer.

[Item 85] The suppressing agent according to **item** 65 or 66, wherein the antibody which specifically binds to human CC chemokine receptor 4, human epidermal growth factor receptor 2, human CD20, or epidermal growth factor receptor is mogamulizumab, trastuzumab, rituximab or cetuximab, respectively.

[ Item 86] A method for treating a tumor comprising administering an effective amount of an antibody which specifically binds to human folate receptor 1, human IL-3Rα or human TIM-3 and an indoleamine 2,3-dioxygenase inhibitor to a human in need thereof.

[Item 87 ] The method according to **item** 86, wherein the indoleamine 2,3-dioxygenase inhibitor is Compound (I) represented by formula (I)

[Chem.17]

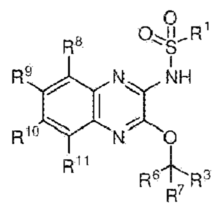

(I)

wherein

$R^6$ and $R^7$ may be the same or different, and each represents a hydrogen atom, or optionally substituted lower alkyl,

$R^8$, $R^9$, $R^{10}$, and $R^{11}$ may be the same or different, and each represents a hydrogen atom, halogen, cyano, or lower alkyl,

$R^1$ represents lower alkyl which may be substituted with lower alkoxy, and

$R^3$ represents an optionally substituted aromatic heterocyclic group,

or a pharmaceutically acceptable salt thereof,
or Compound (II) represented by formula (II)

[Chem.18]

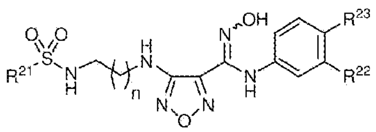

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.
[ Item 88] The method according to item 86 or 87, wherein the antibody which specifically binds to human folate receptor 1 is a monoclonal antibody.
[Item 89] The method according to item 86 or 87, wherein the antibody which specifically binds to human folate receptor 1 is a monoclonal antibody that is selected from the following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

[Item 90] The method according to item 86 or 87, wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.
**[ Item** 91] The method according to item 90, wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

**[ Item** 92] The method according to **item** 90, wherein the antibody to a human IL-3Rα chain comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
[Item 93] The method according to **item** 86 or 87, wherein the antibody which specifically binds to human TIM-3 is a monoclonal antibody, which binds to an extracellular region of human TIM-3.
[ Item 94 ] The method according to **item** 93, wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48

to 50, respectively, and
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

**[ Item** 95] The method according to **item** 93, wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

**[ Item** 96 ] The method according to any one of items 86 to 95, wherein the antibody and the indoleamine 2,3-dioxygenase inhibitor are administered simultaneously or sequentially.
[ Item 97 ] The method according to any one of **items** 86 to 96, wherein the tumor is a tumor associated with human folate receptor 1, human IL-3Rα or human TIM-3.
[ Item 98 ] The method according to **item** 97, wherein the tumor which is associated with human folate receptor 1 is blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma or pancreatic cancer.
**[ Item** 99 ] The method according to **item** 97, wherein the tumor which is associated with human folate receptor 1 is ovarian cancer.
**[ Item** 100 ] The method according to **item** 97, wherein the tumor which is associated with human IL-3Rα is acute myeloid leukemia (AML), acute lymphocytic leukemia, atypical leukemia, chronic lymphocytic leukemia, adult T cell leukemia, NK/T cell lymphoma, granular lymphocytosis (LGL leukemia), polycythemia vera, essential thrombocythemia, hypereosinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma or Castleman disease.
**[ Item** 101 ] The method according to **item** 97, wherein the tumor which is associated with human IL-3Rα is acute myeloid leukemia (AML).
**[ Item** 102 ] The method according to **item** 97, wherein the tumor which is associated with human TIM-3 is blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer.
**[ Item** 103 ] The method according to item 97, wherein the tumor which is associated with human TIM-3 is acute myeloid leukemia (AML).
[ Item 104 ] A method for suppressing decreasing antibody dependent cellular cytotoxicity activity of an antibody which specifically binds to human folate receptor 1, human IL-3Rα or human TIM-3 comprising administering an indoleamine 2,3-dioxygenase inhibitor.
**[ Item** 105 ] The method according to item 104, wherein the indoleamine 2,3-dioxygenase inhibitor is Compound (I) represented by formula (I)

[Chem.19]

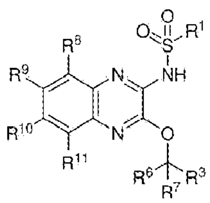

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.20]

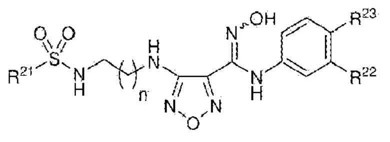

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.
**[ Item** 106 ] The method according to **item** 104 or 105, wherein the antibody which specifically binds to human folate receptor 1 is a monoclonal antibody.
**[ Item** 107 ] The method according to **item** 104 or 105, wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

[ Item 108 ] The method according to **item** 104 or 105, wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.
**[ Item** 109 ] The method according to **item** 108, wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR

1 to 3 of light chain are the amino acid sequences of SEQ ID NOs:36 to 38, respectively.

**[ Item** 110 ] The method according to item 108, wherein the antibody to a human IL-3Rα chain comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
**[ Item** 111 ] The method according to item 104 or 105, wherein the antibody which specifically binds to human TIM-3 is a monoclonal antibody, which binds to an extracellular region of human TIM-3.
**[ Item** 112] The method according to item 111, wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs 1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs: 51 to 53, respectively, and comprises an L chain of an antibody which comprises CDRs 1 to 3 comprising the amino acid sequences represented by SEQ ID NOs:54 to 56, respectively.

**[ Item** 113] The method according to item 111, wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

**[ Item** 114 ] The method according to any one of items 104 to 113, wherein the antibody and the indoleamine 2,3-dioxygenase inhibitor are administered simultaneously or sequentially.
**[ Item** 115 ] A pharmaceutical composition for use in administering an effective amount of an antibody which specifically binds to human folate receptor 1, human IL-3Rα or human TIM-3, and an indoleamine 2,3-dioxygenase inhibitor.
**[ Item** 116] The pharmaceutical composition according to item 115, wherein the indoleamine 2,3-dioxygenase inhibitor is Compound (I) represented by formula (I)

[Chem.21]

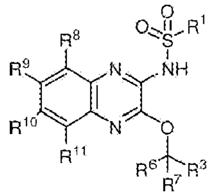

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and

$R^3$ represents an optionally substituted aromatic heterocyclic group,

or a pharmaceutically acceptable salt thereof,
or Compound (II) represented by formula (II)

[Chem.22]

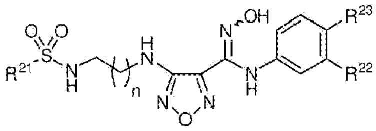

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.

**[ Item** 117] The pharmaceutical composition according to item 115 or 116 which specifically binds to human folate receptor 1 is a monoclonal antibody.

**[ Item** 118] The pharmaceutical composition which specifically binds to human folate receptor 1 according to item 115 or 116 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

[ Item 119 ] The pharmaceutical composition according to item 115 or 116, wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.

**[ Item** 120 ] The pharmaceutical composition according to item 119, wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

**[ Item** 121 ] The pharmaceutical composition according to item 119, wherein the antibody to a human IL-3Rα chain which comprises amino acid sequences of CDRs of heavy chain and CDRs of light chain consisting of the following (e2);

(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

[ Item 122 ] The pharmaceutical composition according to 115 or 116, wherein the antibody which specifically binds to human TIM-3 is a monoclonal antibody, which binds to an extracellular region of human TIM-3.

**[ Item** 123 ] The pharmaceutical composition according to item 122, wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs 1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively,
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

**[ Item** 124 ] The pharmaceutical composition according to item 122, wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

**[ Item** 125 ] The pharmaceutical composition according to item 115 or 116, which comprises simultaneously or sequentially administering the antibody and the indoleamine 2,3-dioxygenase inhibitor.

**[ Item** 126 ] The pharmaceutical composition according to any one of items 115 to 125, wherein the tumor is a tumor associated with human folate receptor 1, human IL-3Rα or human TIM-3.

**[ Item** 127 ] The pharmaceutical composition according to item 126, wherein the tumor is associated with human folate receptor 1 is blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma or pancreatic cancer.

**[ Item** 128 ] The pharmaceutical composition according to item 126, wherein the tumor which is associated with human folate receptor 1 is ovarian cancer.

**[ Item** 129 ] The pharmaceutical composition according to **item** 126, wherein the tumor which is associated with human IL-3Rα is acute myeloid leukemia (AML), acute lymphocytic leukemia, atypical leukemia, chronic lymphocytic leukemia, adult T cell leukemia, NK/T cell lymphoma, granular lymphocytosis (LGL leukemia), polycythemia vera, essential thrombocythemia, hypereosinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma or Castleman disease.

**[ Item** 130 ] The pharmaceutical composition according to **item** 126, wherein the tumor which is associated with human IL-3Rα is acute myeloid leukemia (AML).

**[ Item** 131 ] The pharmaceutical composition according to **item** 126, wherein the tumor which is associated with human TIM-3 is blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer.

**[ Item** 132 ] The pharmaceutical composition according to **item** 126, wherein the tumor which is associated with human TIM-3 is acute myeloid leukemia (AML).

**[ Item** 133 ] A combination of an indoleamine 2,3-dioxygenase inhibitor and an antibody which specifically binds to human folate receptor 1, human IL-3Rα or human TIM-3, for use in the treatment of a tumor.

**[ Item** 134 ] The combination according to **item** 133, wherein the indoleamine 2,3-dioxygenase inhibitor is Compound (I) represented by formula (I)

[Chem.23]

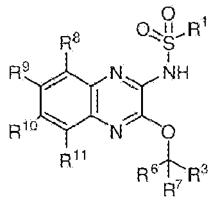

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.24]

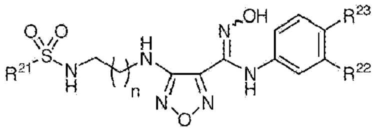

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.

[ Item 135 ] The combination according to item 133 or 134, wherein the antibody which specifically binds to human folate receptor 1 is a monoclonal antibody.

**[ Item** 136 ] The combination according to item 133 or 134, wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

**[ Item** 137 ] The combination according to item 133 or 134, wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.

**[ Item** 138 ] The combination according to item 137, wherein the antibody to a human IL-3Rα chain comprises amino

acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

[ Item 139 ] The combination according to item 137, wherein the antibody to a human IL-3Rα chain comprises amino acid sequences of CDRs of heavy chain and CDRs of light chain consisting of the following (e2); (e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
**[ Item** 140] The combination according to item 133 or 134, wherein the antibody which specifically binds to human TIM-3 is a monoclonal antibody, which binds to an extracellular region of human TIM-3.
**[ Item** 141 ] The combination according to item 140, wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs 1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

**[ Item** 142 ] The combination according to **item** 140, wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

**[ Item** 143 ] The combination according to any one of **items** 133 to 142, wherein the antibody and the indoleamine 2,3-dioxygenase inhibitor are administered simultaneously or sequentially.
**[ Item** 144 ] The combination according to any one of **items** 133 to 143, wherein the tumor is a tumor associated with human folate receptor 1, human IL-3Rα or human TIM-3.
**[ Item** 145 ] The combination according to **item** 144, wherein the tumor which is associated with human folate receptor 1 is blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma or pancreatic cancer.
**[ Item** 146 ] The combination according to **item** 144, wherein the tumor which is associated with human folate receptor 1 is ovarian cancer.
[ Item 147 ] The combination according to **item** 144, wherein the tumor which is associated with human IL-3Rα is acute myeloid leukemia (AML), acute lymphocytic leukemia, atypical leukemia, chronic lymphocytic leukemia, adult

T cell leukemia, NK/T cell lymphoma, granular lymphocytosis (LGL leukemia), polycythemia vera, essential thrombocythemia, hypereosinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma or Castleman disease.

**[ Item** 148 ] The combination according to **item** 144, wherein the tumor which is associated with human IL-3R$\alpha$ is acute myeloid leukemia (AML).

**[ Item** 149 ] The combination according to **items** 144, wherein the tumor which is associated with human TIM-3 is blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer.

**[ Item** 150 ] The combination according to **item** 144, wherein the tumor which is associated with human TIM-3 is acute myeloid leukemia (AML).

**[ Item** 151 ] An indoleamine 2,3-dioxygenase inhibitor for use in the suppression of decreasing antibody dependent cellular cytotoxicity activity of an antibody which specifically binds to human folate receptor 1, human IL-3R$\alpha$ or human TIM-3.

**[ Item** 152 ] The indoleamine 2,3-dioxygenase inhibitor according to **item** 151, wherein the indoleamine 2,3-dioxygenase inhibitor is Compound (I) represented by formula (I)

[Chem.25]

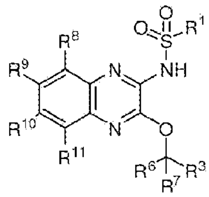

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.26]

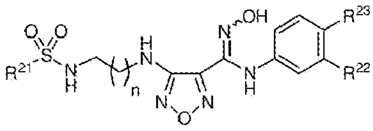

(II)

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.

**[ Item** 153 ] The indoleamine 2,3-dioxygenase inhibitor according to **item** 151 or 152, wherein the antibody and the indoleamine 2,3-dioxygenase inhibitor are administered simultaneously or sequentially.

**[ Item** 154 ] The indoleamine 2,3-dioxygenase inhibitor according to any one of **items** 151 to 153, wherein the

antibody which specifically binds to human folate receptor 1 is a monoclonal antibody.

**[ Item** 155 ] The indoleamine 2,3-dioxygenase inhibitor according to any one of **items** 151 to 153, wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

**[ Item** 156 ] The indoleamine 2,3-dioxygenase inhibitor according to any one of **items** 151 to 153, wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.

**[ Item** 157 ] The indoleamine 2,3-dioxygenase inhibitor according to item 156, wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

**[ Item** 158 ] The indoleamine 2,3-dioxygenase inhibitor according to **item** 156, wherein the antibody to a human IL-3Rα chain comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

**[ Item** 159 ] The indoleamine 2,3-dioxygenase inhibitor according to any one of **items** 151 to 153, wherein the antibody which specifically binds to human TIM-3 is a monoclonal antibody, which binds to an extracellular region of human TIM-3.

**[ Item** 160 ] The indoleamine 2,3-dioxygenase inhibitor according to **item** 159, wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

[Item 161 ] The indoleamine 2,3-dioxygenase inhibitor according to **item** 159, wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

[ Item 162] A therapeutic agent for a tumor, comprising an antibody which specifically binds to human folate receptor 1, human IL-3Rα or human TIM-3, as an active ingredient, wherein the therapeutic agent is administered in combination with an effective amount of an indoleamine 2,3-dioxygenase inhibitor.
**[ Item** 163 ] The therapeutic agent for a tumor according to item 162, wherein the indoleamine 2,3-dioxygenase inhibitor is Compound (I) represented by formula (I)

[Chem.27]

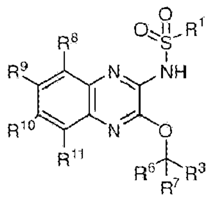

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.28]

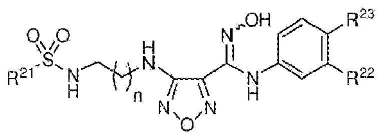

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.
**[ Item** 164 ] The therapeutic agent for a tumor according to item 162 or 163, wherein the antibody which specifically binds to human folate receptor 1 is a monoclonal antibody.
**[ Item** 165 ] The therapeutic agent for a tumor according to item 162 or 163, wherein the antibody which specifically binds to human folate receptor 1 is a monoclonal antibody that is selected from the group consisting of following

(a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

**[ Item** 166 ] The therapeutic agent for a tumor according to **item** 162 or 163, wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.
**[ Item** 167 ] The therapeutic agent for a tumor according to **item** 166, wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

**[ Item** 168 ] The therapeutic agent for a tumor according to **item** 166, wherein the antibody to a human IL-3Rα chain comprises amino acid sequences of CDRs of heavy chain and CDRs of light chain consisting of the following (e2); (e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
**[ Item** 169 ] The therapeutic agent for a tumor according to **item** 162 or 163, wherein the antibody which specifically binds to human TIM-3 is a monoclonal antibody, which binds to an extracellular region of human TIM-3.
**[ Item 170]** The therapeutic agent for a tumor according to **item** 169, wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

**[ Item** 171 ] The therapeutic agent for a tumor according to **item** 169, wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ

ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

**[ Item** 172 ] The therapeutic agent for a tumor according to any one of **items** 162 to 171, wherein the therapeutic agent and the indoleamine 2,3-dioxygenase inhibitor are administered simultaneously or sequentially.

[ Item 173 ] The therapeutic agent for a tumor according to any one of **items** 162 to 172, wherein the tumor is a tumor associated with human folate receptor 1, human IL-3Rα or human TIM-3.

**[ Item** 174] The therapeutic agent for a tumor according to **item** 173, wherein the tumor which is associated with human folate receptor 1 is blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma or pancreatic cancer.

**[ Item** 175 ] The therapeutic agent for a tumor according to **item** 173, wherein the tumor which is associated with human folate receptor 1 is ovarian cancer.

**[ Item** 176 ] The therapeutic agent for a tumor according to **item** 173, wherein the tumor which is associated with human IL-3Rα is acute myeloid leukemia (AML), acute lymphocytic leukemia, atypical leukemia, chronic lymphocytic leukemia, adult T cell leukemia, NK/T cell lymphoma, granular lymphocytosis (LGL leukemia), polycythemia vera, essential thrombocythemia, hypereosinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma or Castleman disease.

**[ Item** 177 ] The therapeutic agent for a tumor according to **item** 173, wherein the tumor which is associated with human IL-3Rα is acute myeloid leukemia (AML).

**[ Item** 178 ] The therapeutic agent for a tumor according to **item** 173, wherein the tumor which is associated with human TIM-3 is blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer.

**[ Item** 179 ] The therapeutic agent for a tumor according to **item** 173, wherein the tumor which is associated with human TIM-3 is acute myeloid leukemia (AML).

**[ Item** 180 ] A therapeutic agent for a tumor, comprising an indoleamine 2,3-dioxygenase inhibitor as an active ingredient, wherein the therapeutic agent is administered in combination with an effective amount of an antibody which specifically binds to human folate receptor 1, human IL-3Rα or human TIM-3.

**[ Item** 181 ] The therapeutic agent for a tumor according to **item** 180, wherein the indoleamine 2,3-dioxygenase inhibitor is Compound (I) represented by formula (I)

[Chem.29]

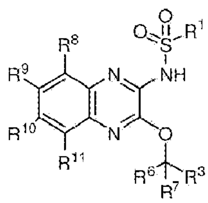

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.30]

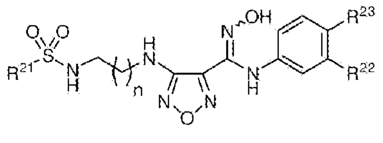

(II)

wherein

$R^{21}$ represents amino or methyl,
$R^{22}$ represents halogen, cyano, trifluoromethyl, or lower alkyl,
$R^{23}$ represents hydrogen atom, or halogen, and
n represents 1 or 2,

or a pharmaceutically acceptable salt thereof.
**[ Item** 182 ] The therapeutic agent for a tumor according to **item** 180 or 181, wherein the antibody which specifically binds to human folate receptor 1 is a monoclonal antibody.
**[ Item** 183 ] The therapeutic agent for a tumor according to **item** 180 or 181,
wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

**[ Item** 184 ] The therapeutic agent for a tumor according to item 180 or 181, wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.
[ Item 185 ] The therapeutic agent for a tumor according to item 184, wherein the antibody to a human IL-3Rα chain comprises amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

**[ Item** 186 ] The therapeutic agent for a tumor according to **item** 184, wherein the antibody to a human IL-3Rα chain comprises amino acid sequences of CDRs of heavy chain and CDRs of light chain consisting of the following (e2);
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
**[ Item** 187 ] The therapeutic agent for a tumor according to **item** 180 or 181, wherein the antibody which specifically binds to human TIM-3 is a monoclonal antibody, which binds to an extracellular region of human TIM-3.
**[ Item** 188 ] The therapeutic agent for a tumor according to **item** 187, wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,

(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and

(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

**[ Item** 189 ] The therapeutic agent for a tumor according to **item** 187, wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and

(b3) a monoclonal antibody which comprises VH of an antibody comprising the amino acid sequence represented by SEQ ID NO:59 and comprises VL of an antibody comprising the amino acid sequence represented by SEQ ID NO:60.

**[ Item** 190 ] The therapeutic agent for a tumor according to any one of **items** 180 to 189, wherein the therapeutic agent and the antibody are administered simultaneously or sequentially.

**[ Item** 191 ] The therapeutic agent for a tumor according to any one of **items** 180 to 190, wherein the tumor is a tumor associated with human folate receptor 1, human IL-3Rα or human TIM-3.

**[ Item** 192 ] The therapeutic agent for a tumor according to **item** 191, wherein the tumor which is associated with human folate receptor 1 is blood cancer, breast cancer, uterine cancer, colorectal cancer, esophageal cancer, stomach cancer, ovarian cancer, lung cancer, renal cancer, rectal cancer, thyroid cancer, uterine cervix cancer, small intestinal cancer, prostate cancer, mesothelioma or pancreatic cancer.

**[ Item** 193 ] The therapeutic agent for a tumor according to **item** 191, wherein the tumor which is associated with human folate receptor 1 is ovarian cancer.

**[ Item** 194 ] The therapeutic agent for a tumor according to **item** 191, wherein the tumor which is associated with human IL-3Rα is acute myeloid leukemia (AML), acute lymphocytic leukemia, atypical leukemia, chronic lymphocytic leukemia, adult T cell leukemia, NK/T cell lymphoma, granular lymphocytosis (LGL leukemia), polycythemia vera, essential thrombocythemia, hypereosinophilic syndrome, Hodgkin lymphoma, non-Hodgkin lymphoma, follicular lymphoma, MALT lymphoma, mantle cell lymphoma, diffuse large B-cell lymphoma, Burkitt lymphoma, lymphoblastic lymphoma or Castleman disease.

[ Item 195 ] The therapeutic agent for a tumor according to **item** 191, wherein the tumor which is associated with human IL-3Rα is acute myeloid leukemia (AML).

[ Item 196 ] The therapeutic agent for a tumor according to **item** 191, wherein the tumor which is associated with human TIM-3 is blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer.

**[ Item** 197 ] The therapeutic agent for a tumor according to **item** 191, wherein the tumor which is associated with human TIM-3 is acute myeloid leukemia (AML).

**[ Item** 198 ] A suppressing agent for decreasing antibody dependent cellular cytotoxicity activity of an antibody which specifically binds to human folate receptor 1, human IL-3Rα or human TIM-3, comprising an indoleamine 2,3-dioxygenase inhibitor as an active ingredient.

**[ Item** 199 ] The suppressing agent according to **item** 198, wherein the indoleamine 2,3-dioxygenase inhibitor is Compound (I) represented by formula (I)

[Chem.31]

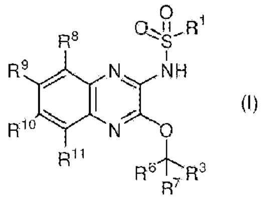

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,

or a pharmaceutically acceptable salt thereof,
or Compound (II) represented by formula (II)

[Chem.32]

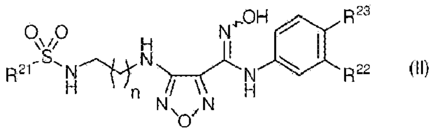

wherein

$R^{21}$ represents amino or methyl,
$R^{22}$ represents halogen, cyano, trifluoromethyl, or lower alkyl,
$R^{23}$ represents a hydrogen atom, or halogen, and
n represents 1 or 2,

or a pharmaceutically acceptable salt thereof.
**[ Item** 200 ] The suppressing agent according to **item** 198 or 199, wherein the antibody which specifically binds to human folate receptor 1 is a monoclonal antibody.
[ Item 201 ] The suppressing agent according to **item** 198 or 199, wherein the antibody which specifically binds to human folate receptor 1 is a monoclonal antibody that is selected from the group consisting of following (a1)-(c1):

(a1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively;
(b1) a monoclonal antibody in which CDRs 1-3 of H chain of the antibody comprise the amino acid sequences represented by SEQ ID NOs. 1, 2, and 3, respectively and CDRs 1-3 of L chain of the antibody comprise the amino acid sequences represented by SEQ ID NOs. 4, 5, and 6, respectively, and cysteine in the amino acid sequence represented by SEQ ID NO. 3 (CDR3 of antibody H chain) is substituted with threonine, methionine, isoleucine, valine, phenylalanine, or glutamine; and
(c1) a monoclonal antibody in which H chain of the antibody comprises the amino acid sequence represented by SEQ ID NO. 7, and L chain of the antibody comprises the amino acid sequence represented by SEQ ID NO. 8.

**[ Item** 202 ] The suppressing agent according to **item** 198 or 199, wherein the antibody which specifically binds to human IL-3Rα is an antibody to a human IL-3Rα chain, which does not inhibit IL-3 signaling and binds to B domain of the human IL-3Rα chain but does not bind to C domain of the human IL-3Rα chain.
[ Item 203 ] The suppressing agent according to **item** 202, wherein the antibody to a human IL-3Rα chain comprises

amino acid sequences of CDRs of heavy chain and CDRs of light chain selected from the group consisting of the following (a2) to (e2);

(a2) CDR 1 to 3 of heavy chain are the amino acid sequences of SEQ ID NOs:9 to 11, respectively, and CDR 1 to 3 of light chain are the amino acid sequences of SEQ ID NOs:24 to 26, respectively,
(b2) CDR 1 to 3 of heavy chain are the amino acid sequences of SEQ ID NOs:12 to 14, respectively, and CDR 1 to 3 of light chain are the amino acid sequences of SEQ ID NOs:27 to 29, respectively,
(c2) CDR 1 to 3 of heavy chain are the amino acid sequences of SEQ ID NOs:15 to 17, respectively, and CDR 1 to 3 of light chain are the amino acid sequences of SEQ ID NOs:30 to 32, respectively,
(d2) CDR 1 to 3 of heavy chain are the amino acid sequences of SEQ ID NOs:18 to 20, respectively, and CDR 1 to 3 of light chain are the amino acid sequences of SEQ ID NOs:33 to 35, respectively, and
(e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.

[ Item 204 ] The suppressing agent according to **item** 202, wherein the antibody to a human IL-3Rα chain comprises amino acid sequences of CDRs of heavy chain and CDRs of light chain consisting of the following (e2); (e2) CDR 1 to 3 of heavy chain are the amino acid sequences of SEQ ID NOs:21 to 23, respectively, and CDR 1 to 3 of light chain are the amino acid sequences of SEQ ID NOs:36 to 38, respectively.
**[ Item** 205 ] The suppressing agent according to **item** 198 or 199, wherein the antibody which specifically binds to human TIM-3 is a monoclonal antibody, which binds to an extracellular region of human TIM-3.
**[ Item** 206 ] The suppressing agent according to **item** 205, wherein the monoclonal antibody is one selected from the group consisting of following (i) to (iii):

(i) a monoclonal antibody which comprises an H chain of an antibody which comprises CDRs 1 to 3 comprising the amino acid sequences represented by SEQ ID NOs:39 to 41, respectively, and comprises an L chain of an antibody which comprises CDRs1 to 3 comprising the amino acid sequences represented by SEQ ID NOs:42 to 44, respectively,
(ii) a monoclonal antibody which comprises an H chain of an antibody which comprises CDRs 1 to 3 comprising the amino acid sequences represented by SEQ ID NOs:45 to 47, respectively, and comprises an L chain of an antibody which comprises CDRs 1 to 3 comprising the amino acid sequences represented by SEQ ID NOs:48 to 50, respectively, and
(iii) a monoclonal antibody which comprises an H chain of an antibody which comprises CDRs 1 to 3 comprising the amino acid sequences represented by SEQ ID NOs:51 to 53, respectively, and comprises an L chain of an antibody which comprises CDRs 1 to 3 comprising the amino acid sequences represented by SEQ ID NOs:54 to 56, respectively.

**[ Item** 207 ] The suppressing agent according to **item** 205, wherein the monoclonal antibody is one selected from the group consisting of following (a3) and (b3):

(a3) a monoclonal antibody which comprises VH of an antibody comprising the amino acid sequence represented by SEQ ID NO:57 and comprises VL of an antibody comprising the amino acid sequence represented by SEQ ID NO:58, and
(b3) a monoclonal antibody which comprises VH of an antibody comprising the amino acid sequence represented by SEQ ID NO:59 and comprises VL of an antibody comprising the amino acid sequence represented by SEQ ID NO:60.

**[ Item** 208 ] The suppressing agent according to any one of **items** 198 to 207 wherein the suppressing agent and the antibody are administered simultaneously or sequentially.
[ Item 209 ] The indoleamine 2,3-dioxygenase inhibitor according to **item** 48 or 49, wherein the indoleamine 2,3-dioxygenase inhibitor and the antibody are administered simultaneously or sequentially.
**[ Item 210]** The suppressing agent according to **item** 65 or 66, wherein the suppressing agent and the antibody are administered simultaneously or sequentially.

## Claims

1. A pharmaceutical composition comprising an effective amount of an indoleamine 2,3-dioxygenase inhibitor for use as a pharmaceutical, wherein said pharmaceutical composition is administered in combination with an effective

amount of an antibody which specifically binds to human CD20,wherein the indoleamine 2,3-dioxygenase inhibitor is Compound (I) represented by formula (I)

{Chem. 1}

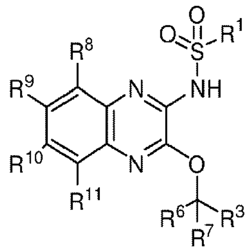

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,
or a pharmaceutically acceptable salt thereof.

**2.** The pharmaceutical composition for use according to claim 1, wherein the composition and the antibody are administered simultaneously, sequentially, or separately at intervals.

**3.** The pharmaceutical composition for use according to claim 1 or 2, wherein the composition is for use in the treatment of a tumor.

**4.** The pharmaceutical composition for use according to claim 3, wherein the tumor is a tumor which expresses human CD20.

**5.** The pharmaceutical composition for use according to claim 4, wherein the tumor which expresses human CD20 is chronic leukemia, or non-Hodgkin's lymphoma.

**6.** The pharmaceutical composition for use according to claim 5, wherein the chronic leukemia is chronic lymphocytic leukemia.

**7.** The pharmaceutical composition for use according to claim 5, wherein the non-Hodgkin's lymphoma is B cell lymphoma.

**8.** The pharmaceutical composition for use according to claim 7, wherein the B cell lymphoma is mantle cell lymphoma, diffuse large B cell lymphoma or Burkitt's lymphoma.

**9.** The pharmaceutical composition for use according to claim 7, wherein the B cell lymphoma is Burkitt's lymphoma.

**10.** The pharmaceutical composition for use according to any one of claims 1 to 9, wherein the antibody which specifically binds to human CD20 is rituximab.

**11.** A combination of an indoleamine 2,3-dioxygenase inhibitor and an antibody which specifically binds to human CD20, for use in the treatment of a tumor,
wherein the indoleamine 2,3-dioxygenase inhibitor is Compound (I) represented by formula (I)

{Chem. 2}

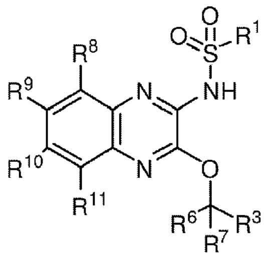

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,
or a pharmaceutically acceptable salt thereof.

**12.** A therapeutic agent for use in the treatment of a tumor, comprising an indoleamine 2,3-dioxygenase inhibitor as an active ingredient, wherein the therapeutic agent is administered in combination with an effective amount of an antibody which specifically binds to human CD20 and
wherein the indoleamine 2,3-dioxygenase inhibitor is Compound (I) represented by formula (I)

{Chem. 3}

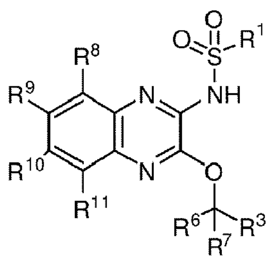

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,
or a pharmaceutically acceptable salt thereof.

**13.** A therapeutic agent for use in the treatment of a tumor, comprising an antibody which specifically binds to human CD20 as an active ingredient, wherein the therapeutic agent is administered in combination with an effective amount of an indoleamine 2,3-dioxygenase inhibitor, and
wherein the indoleamine 2,3-dioxygenase inhibitor is Compound (I) represented by formula (I)

{Chem. 4}

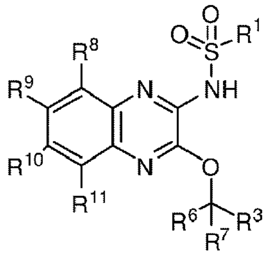

(I)

wherein

$R^6$ and $R^7$ may be the same or different, and each represents a hydrogen atom, or optionally substituted lower alkyl,
$R^8$, $R^9$, $R^{10}$, and $R^{11}$ may be the same or different, and each represents a hydrogen atom, halogen, cyano, or lower alkyl,
$R^1$ represents lower alkyl which may be substituted with lower alkoxy, and
$R^3$ represents an optionally substituted aromatic heterocyclic group,
or a pharmaceutically acceptable salt thereof.

[Fig. 1]

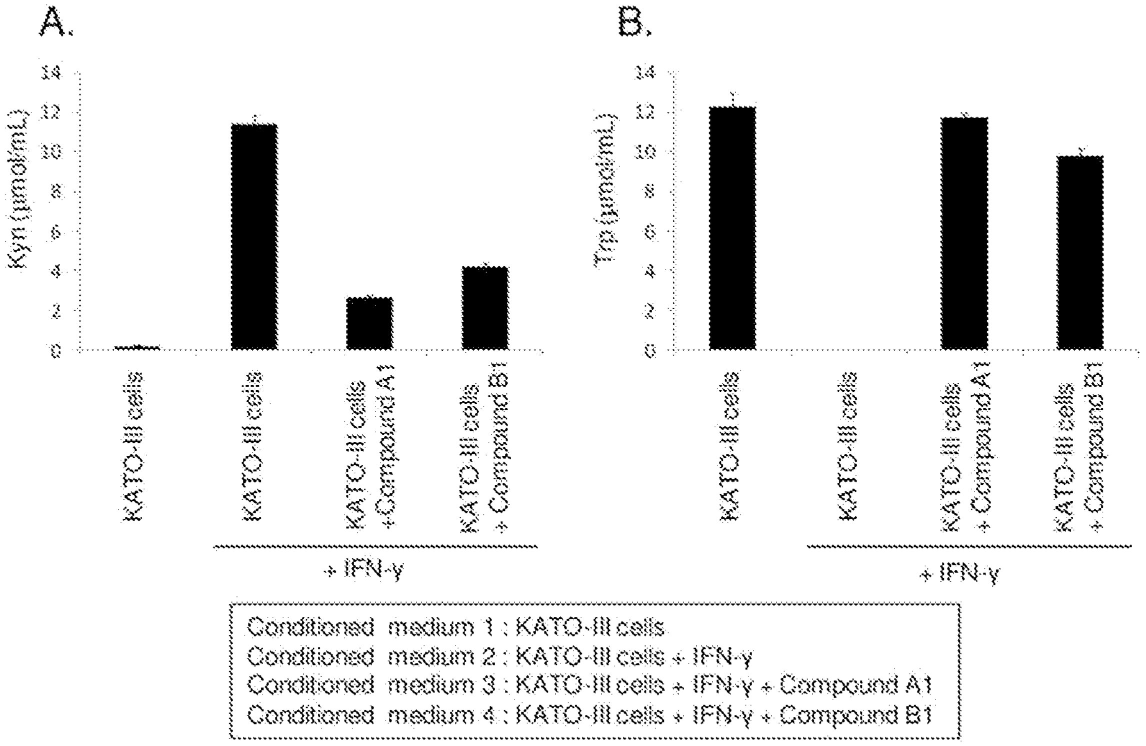
A.
Kyn (μmol/mL)
14
12
10
8
6
4
2
0
KATO-III cells
KATO-III cells
KATO-III cells +Compound A1
KATO-III cells + Compound B1
+ IFN-γ
B.
Trp (μmol/mL)
14
12
10
8
6
4
2
0
KATO-III cells
KATO-III cells
KATO-III cells + Compound A1
KATO-III cells + Compound B1
+ IFN-γ
Conditioned medium 1 : KATO-III cells
Conditioned medium 2 : KATO-III cells + IFN-γ
Conditioned medium 3 : KATO-III cells + IFN-γ + Compound A1
Conditioned medium 4 : KATO-III cells + IFN-γ + Compound B1

[Fig. 2]

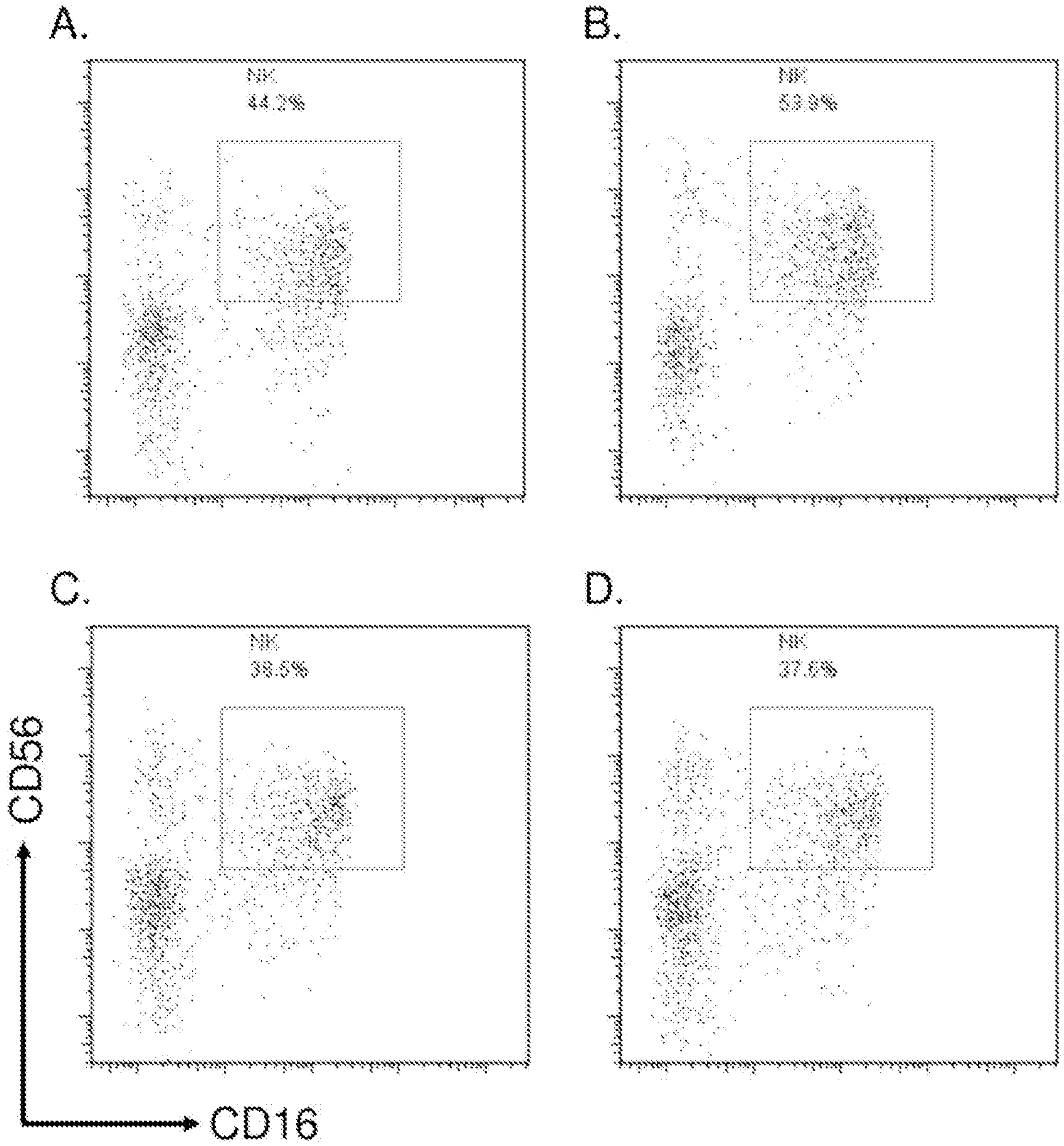

[Fig. 3]

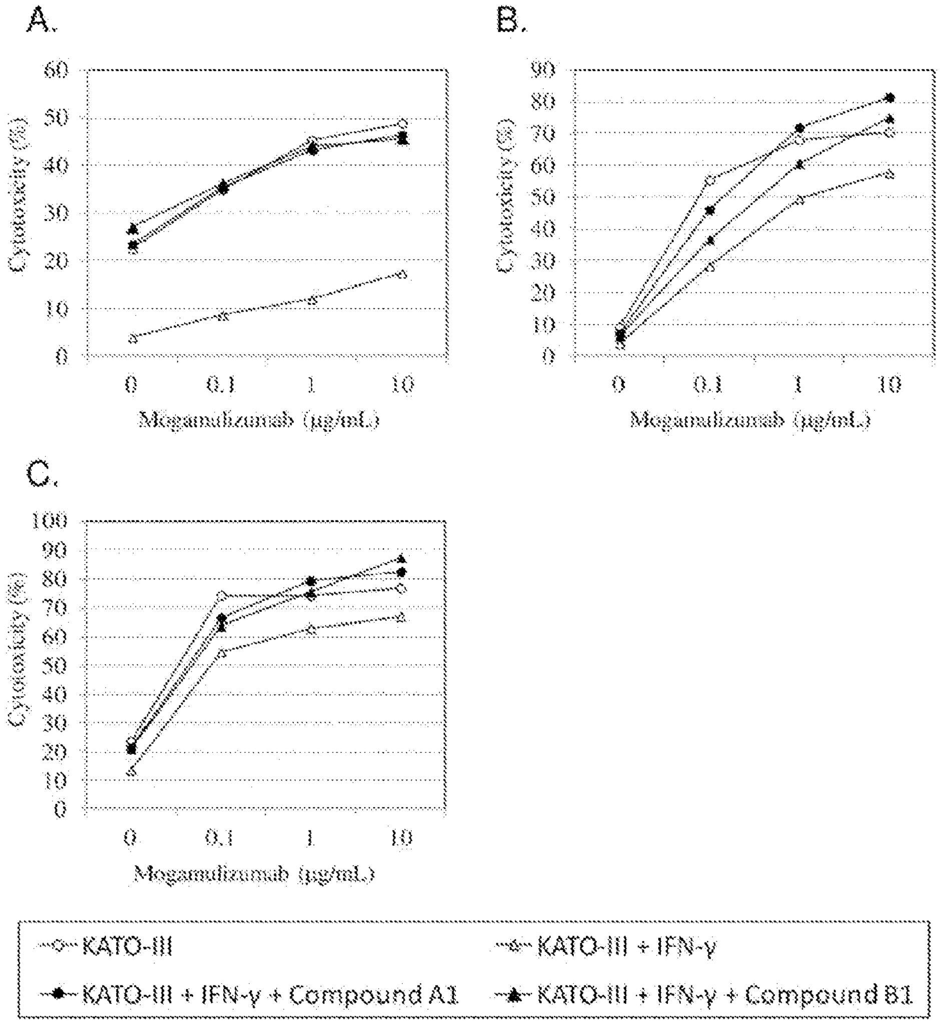
A.
B.
C.
Cytotoxicity (%)
Mogamulizumab (μg/mL)
0
0.1
1
10
KATO-III
KATO-III + IFN-γ
KATO-III + IFN-γ + Compound A1
KATO-III + IFN-γ + Compound B1

[Fig. 4]

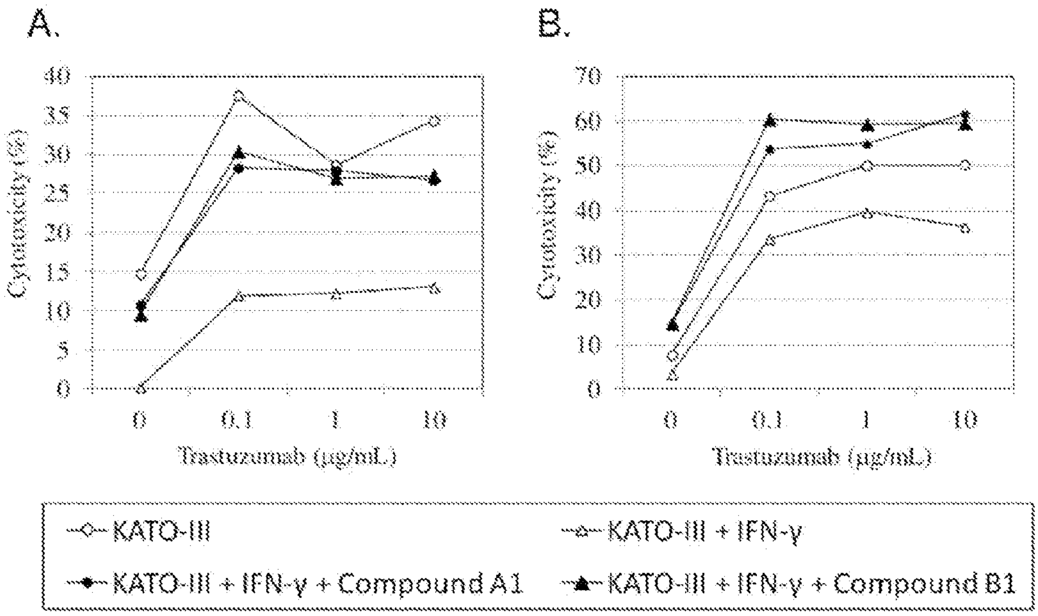
A.
B.
Cytotoxicity (%)
Trastuzumab (μg/mL)
0
0.1
1
10
KATO-III
KATO-III + IFN-γ
KATO-III + IFN-γ + Compound A1
KATO-III + IFN-γ + Compound B1

[Fig. 5]

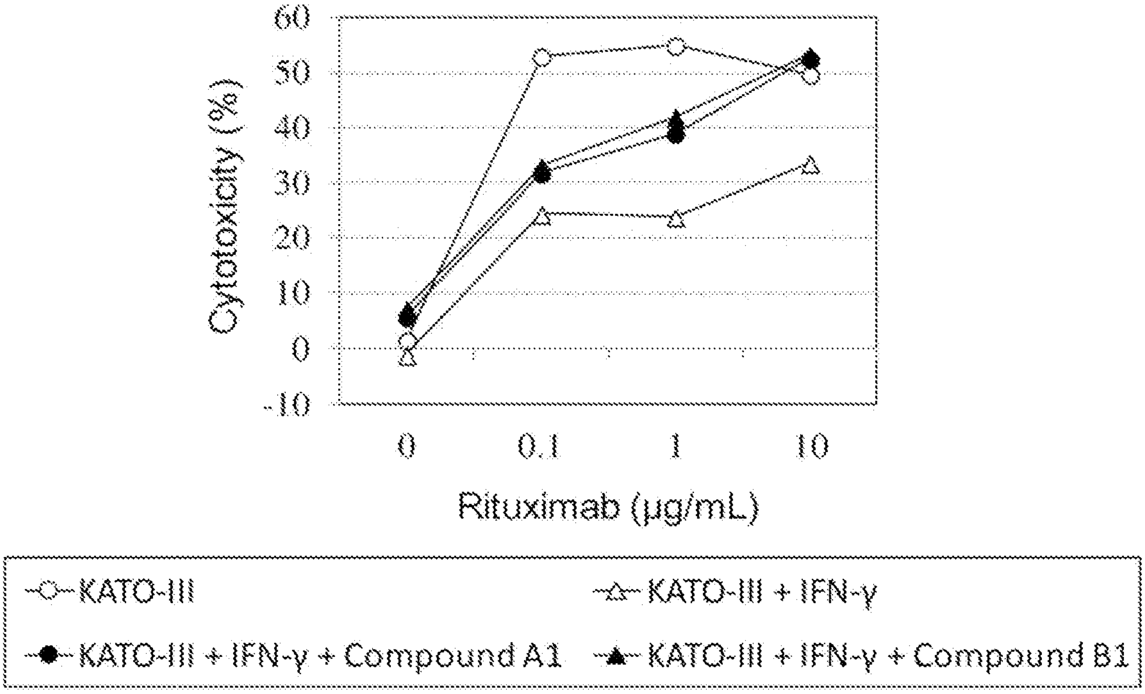
60
50
40
30
20
10
0
-10
Cytotoxicity (%)
0
0.1
1
10
Rituximab (μg/mL)
KATO-III
KATO-III + IFN-γ
KATO-III + IFN-γ + Compound A1
KATO-III + IFN-γ + Compound B1

[Fig. 6]

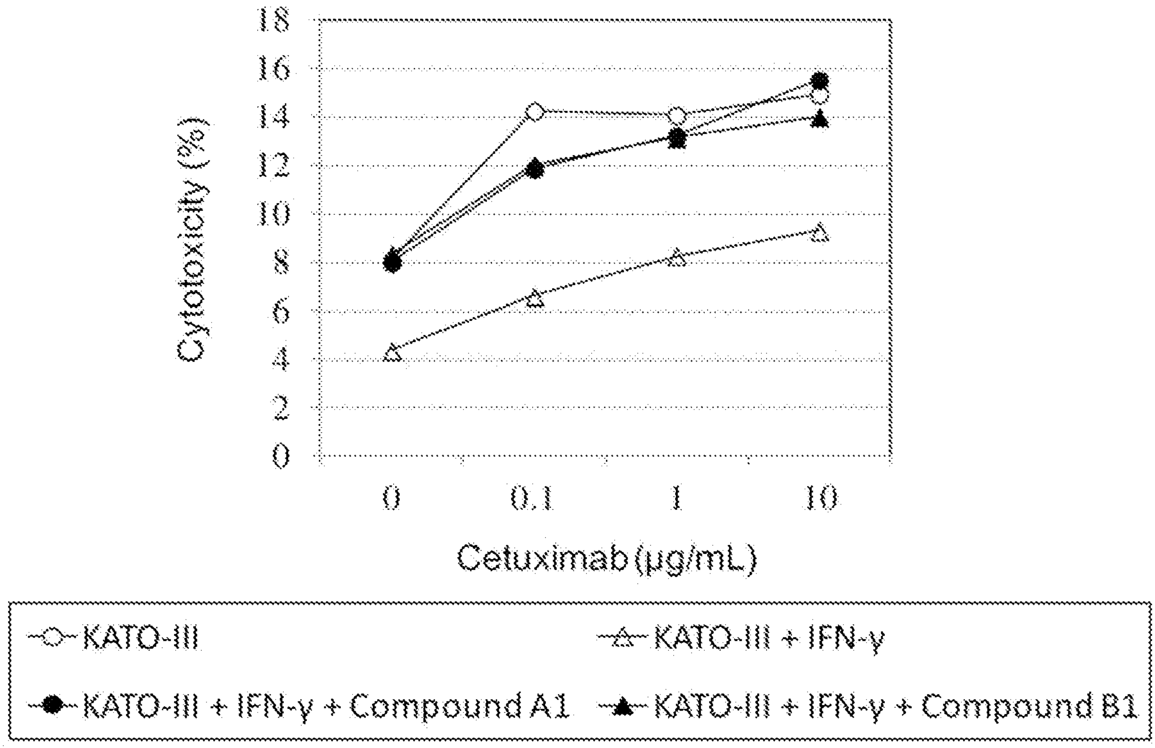
18
16
14
12
10
8
6
4
2
0
Cytotoxicity (%)
0
0.1
1
10
Cetuximab (μg/mL)
KATO-III
KATO-III + IFN-γ
KATO-III + IFN-γ + Compound A1
KATO-III + IFN-γ + Compound B1

[Fig. 7]

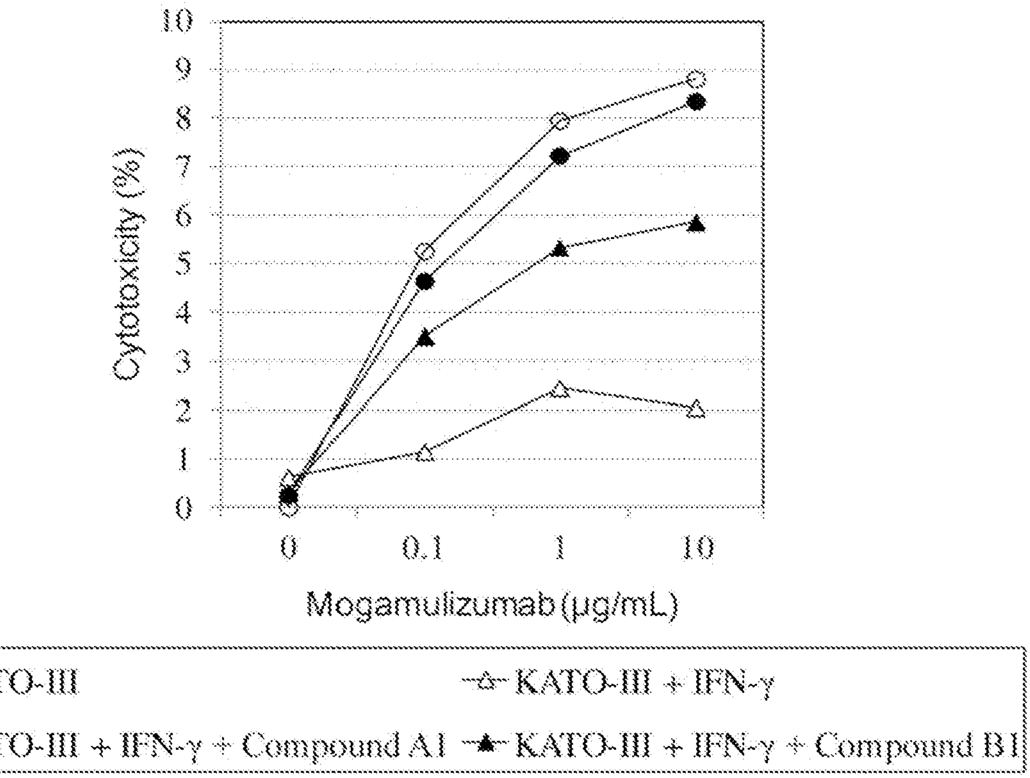
Cytotoxicity (%)
10
9
8
7
6
5
4
3
2
1
0
0
0.1
1
10
Mogamulizumab (μg/mL)
KATO-III
KATO-III + IFN-γ
KATO-III + IFN-γ + Compound A1
KATO-III + IFN-γ + Compound B1

[Fig. 8]

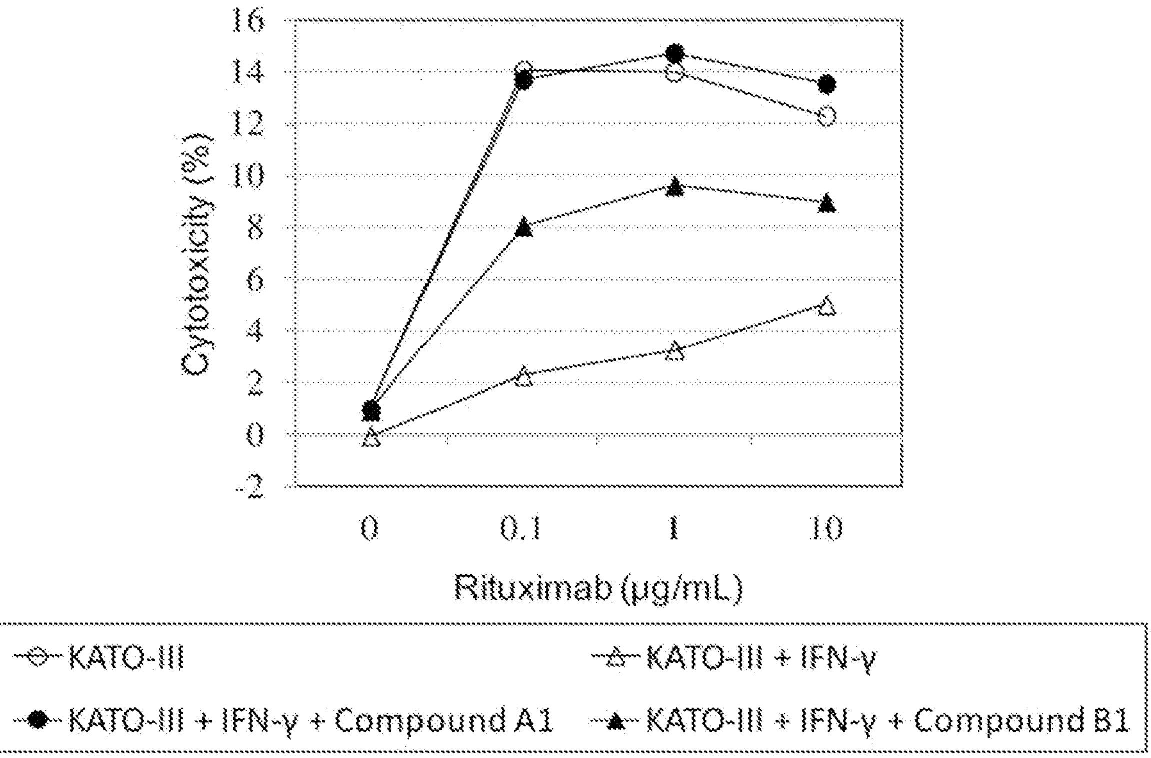
Cytotoxicity (%)
16
14
12
10
8
6
4
2
0
-2
0
0.1
1
10
Rituximab (μg/mL)
KATO-III
KATO-III + IFN-γ
KATO-III + IFN-γ + Compound A1
KATO-III + IFN-γ + Compound B1

[Fig. 9]

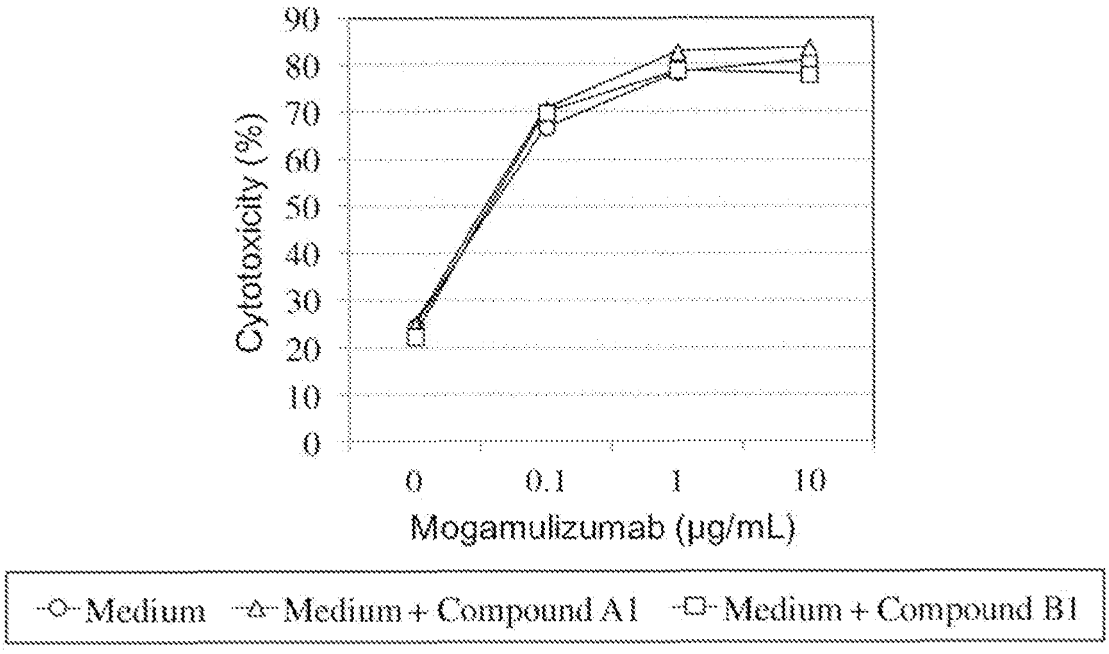
Cytotoxicity (%)
90
80
70
60
50
40
30
20
10
0
0
0.1
1
10
Mogamulizumab (μg/mL)
Medium
Medium + Compound A1
Medium + Compound B1

[Fig. 10]

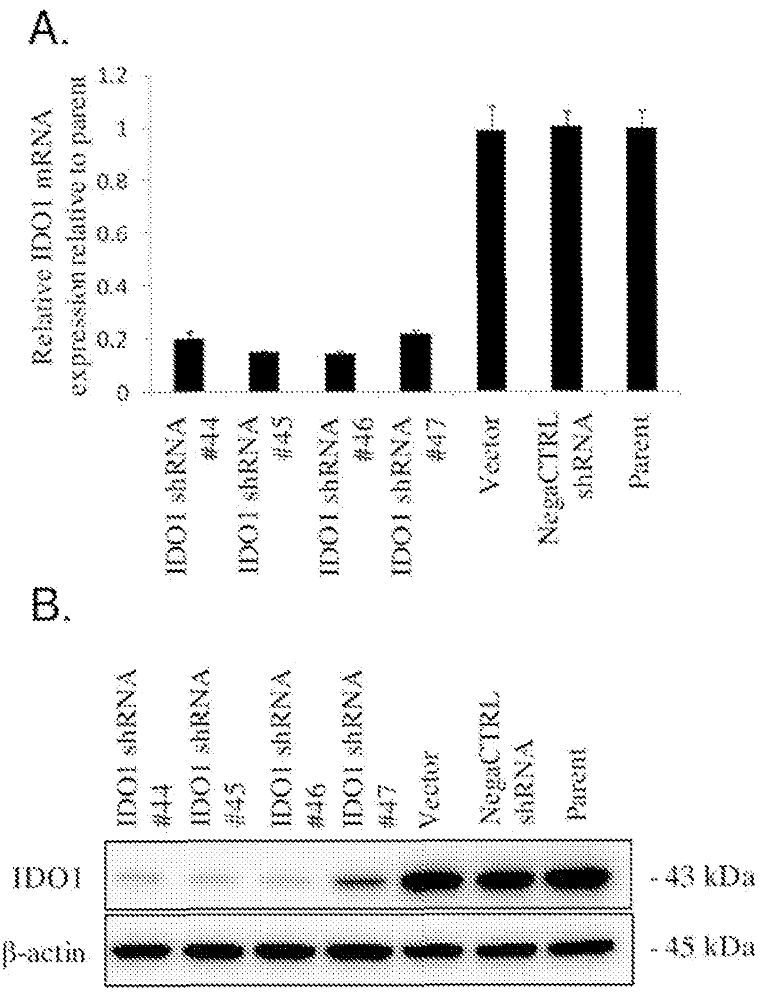
A.
Relative IDO1 mRNA expression relative to parent
1.2
1
0.8
0.6
0.4
0.2
0
IDO1 shRNA #44
IDO1 shRNA #45
IDO1 shRNA #46
IDO1 shRNA #47
Vector
NegaCTRL shRNA
Parent
B.
IDO1 shRNA #44
IDO1 shRNA #45
IDO1 shRNA #46
IDO1 shRNA #47
Vector
NegaCTRL shRNA
Parent
IDO1
β-actin
- 43 kDa
- 45 kDa

[Fig. 11]

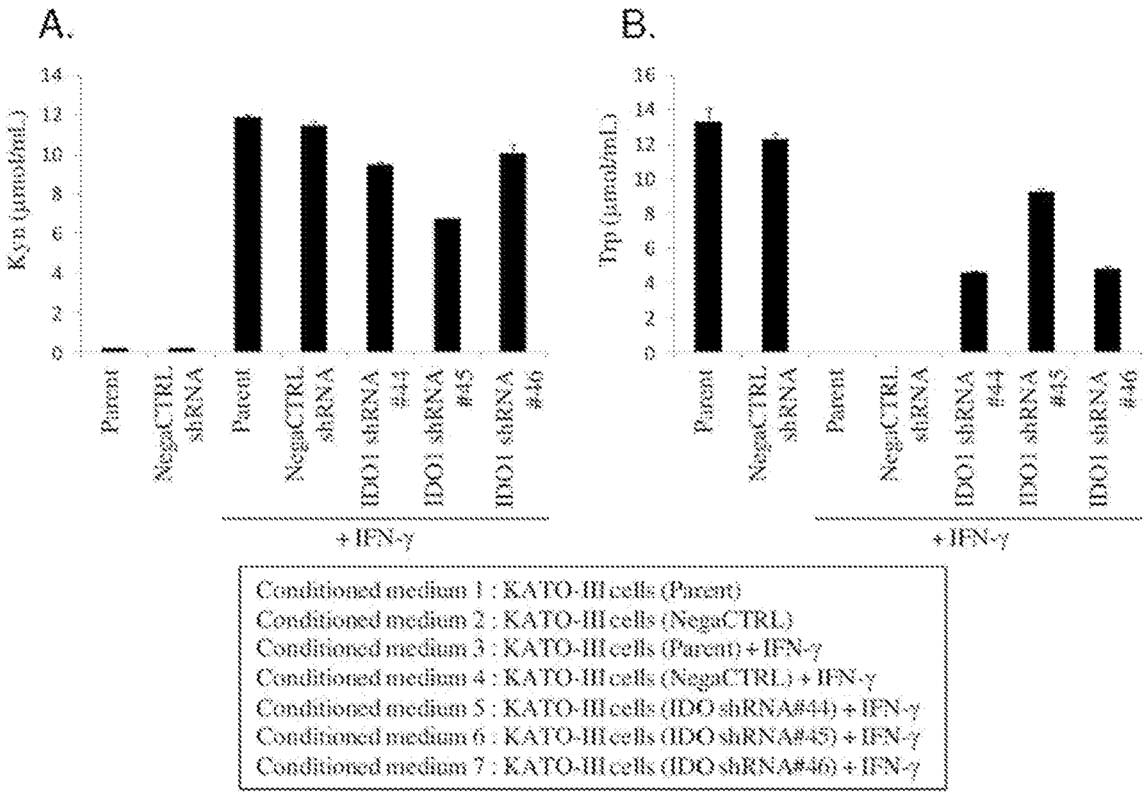
A.
Kyn (μmol/mL)
B.
Trp (μmol/mL)
Parent
NegaCTRL shRNA
Parent
NegaCTRL shRNA
IDO1 shRNA #44
IDO1 shRNA #45
IDO1 shRNA #46
+ IFN-γ
Conditioned medium 1 : KATO-III cells (Parent)
Conditioned medium 2 : KATO-III cells (NegaCTRL)
Conditioned medium 3 : KATO-III cells (Parent) + IFN-γ
Conditioned medium 4 : KATO-III cells (NegaCTRL) + IFN-γ
Conditioned medium 5 : KATO-III cells (IDO shRNA#44) + IFN-γ
Conditioned medium 6 : KATO-III cells (IDO shRNA#45) + IFN-γ
Conditioned medium 7 : KATO-III cells (IDO shRNA#46) + IFN-γ

[Fig. 12]

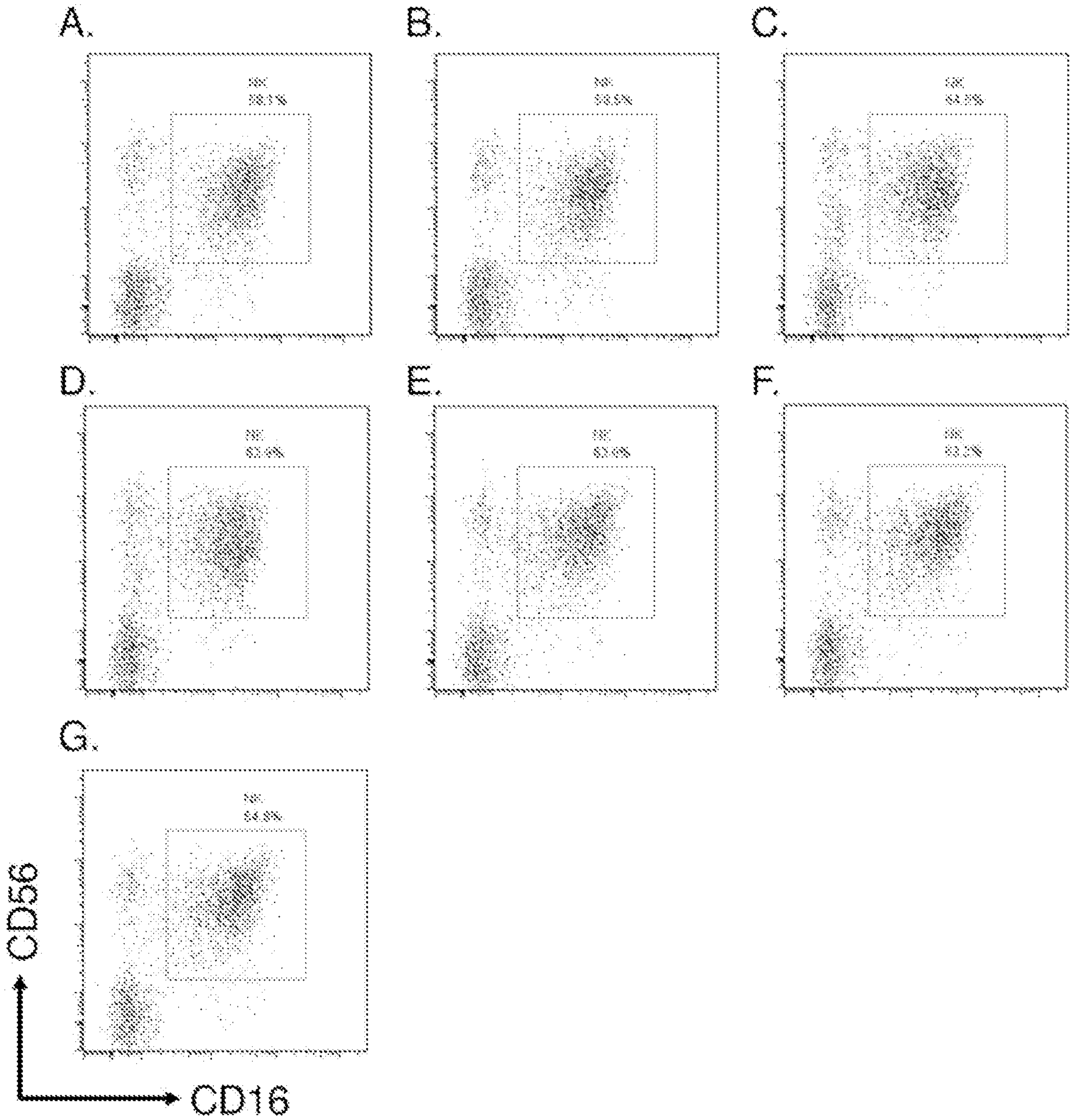

[Fig. 13]

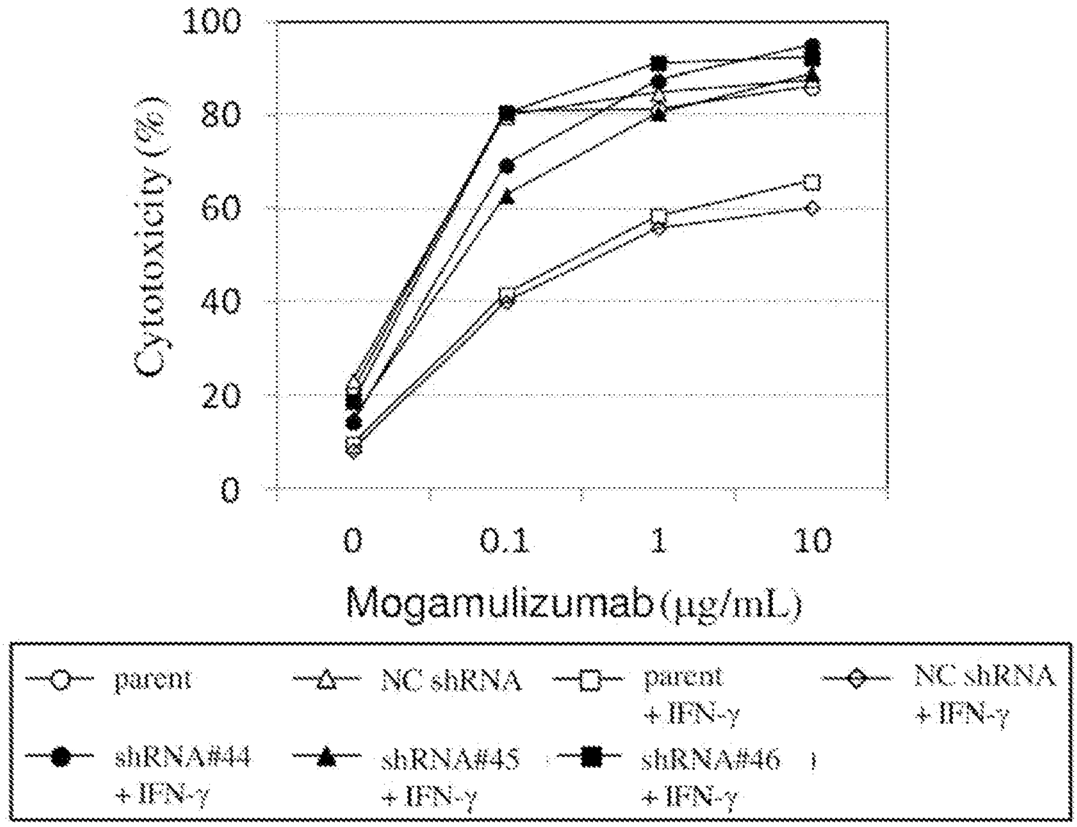

100
80
60
40
20
0
Cytotoxicity (%)
0
0.1
1
10
Mogamulizumab (μg/mL)
parent
NC shRNA
parent + IFN-γ
NC shRNA + IFN-γ
shRNA#44 + IFN-γ
shRNA#45 + IFN-γ
shRNA#46 + IFN-γ


[Fig. 14]

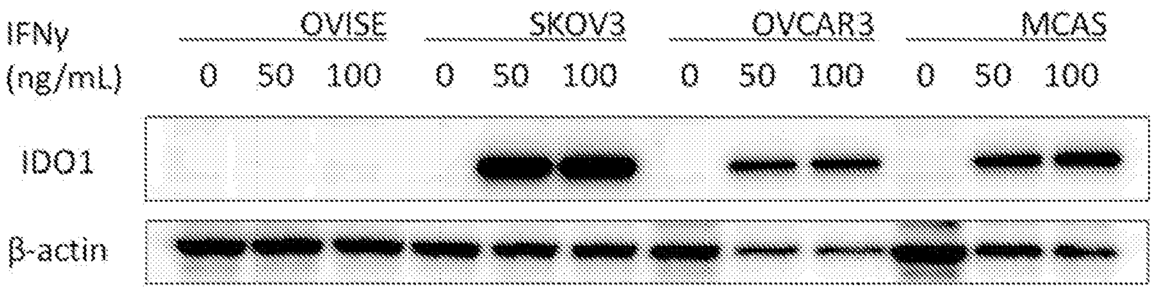

IFNγ (ng/mL)
OVISE
SKOV3
OVCAR3
MCAS
0 50 100 0 50 100 0 50 100 0 50 100
IDO1
β-actin

[Fig. 15]

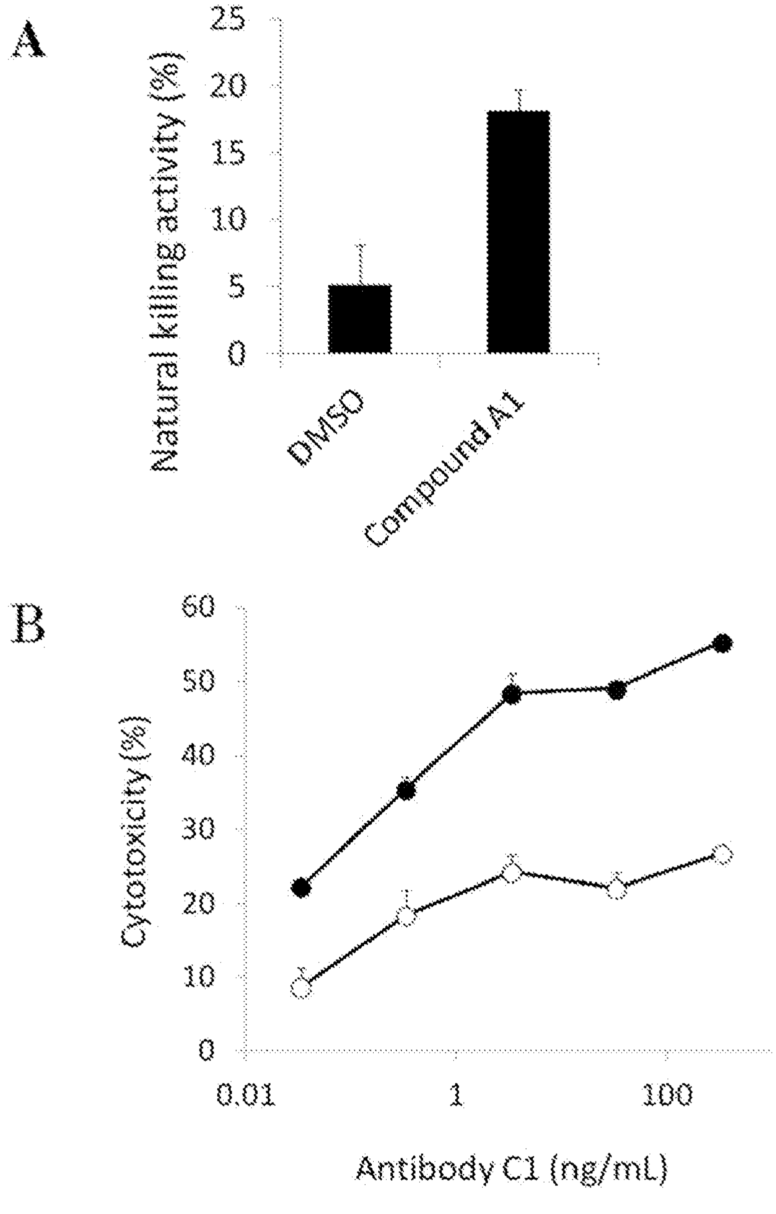
A
Natural killing activity (%)
25
20
15
10
5
0
DMSO
Compound A1
B
Cytotoxicity (%)
60
50
40
30
20
10
0
0.01
1
100
Antibody C1 (ng/mL)

[Fig. 16]

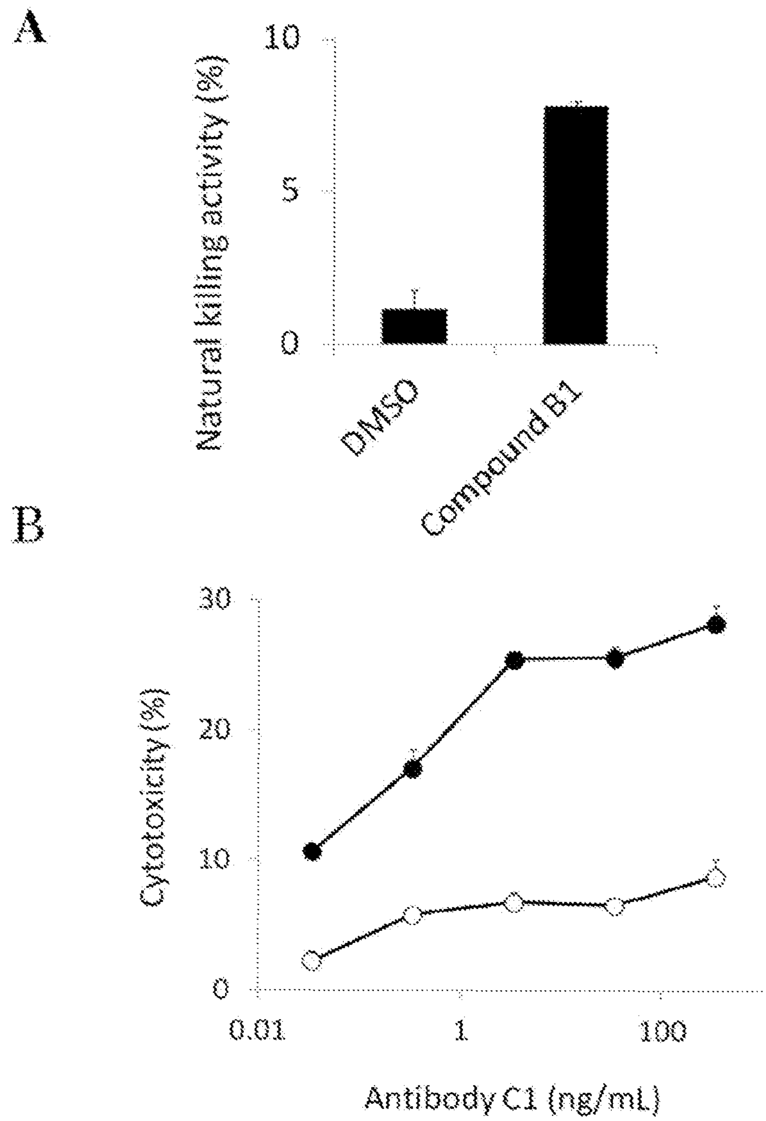
A
Natural killing activity (%)
10
5
0
DMSO
Compound B1
B
Cytotoxicity (%)
30
20
10
0
0.01
1
100
Antibody C1 (ng/mL)

[Fig. 17]

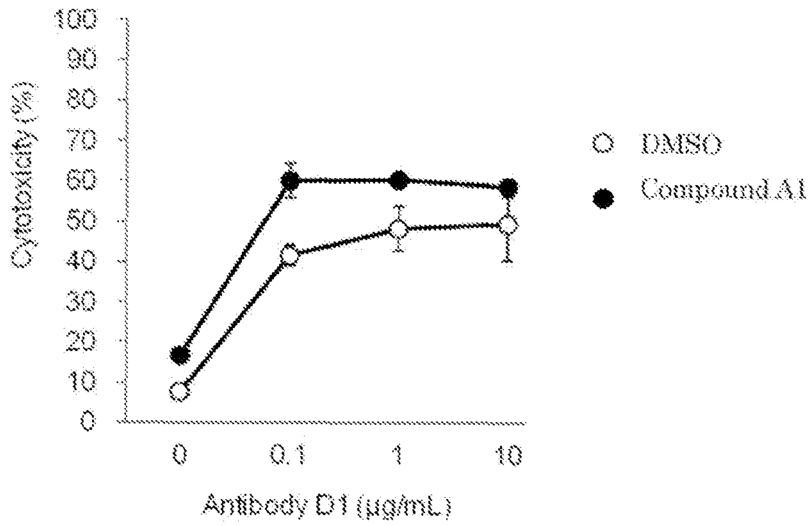
Cytotoxicity (%)
100
90
80
70
60
50
40
30
20
10
0
0
0.1
1
10
Antibody D1 (µg/mL)
DMSO
Compound A1

[Fig. 18]

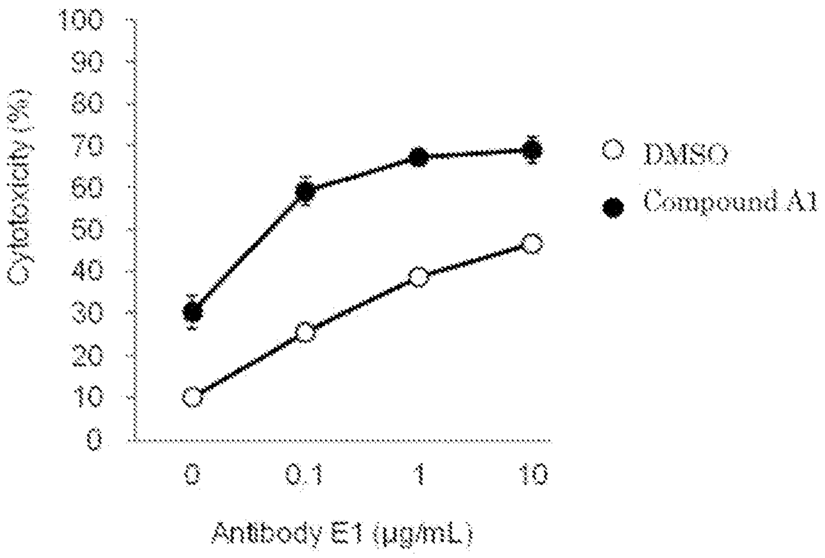
100
90
80
70
60
50
40
30
20
10
0
Cytotoxicity (%)
0
0.1
1
10
Antibody E1 (μg/mL)
DMSO
Compound A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9929310 A
- WO 2010053182 A
- WO 2011142316 A
- WO 2013069765 A
- WO 2006122150 A
- WO 2010005958 A
- WO 2015002918 A
- WO 0164754 A
- WO 0318635 A
- WO 2005057341 A
- US 4968603 A
- US 5677171 A
- US 5821337 A
- WO 2010126066 A
- WO 9627603 A
- WO 2003063792 A
- WO 2009091547 A
- WO 2011155607 A
- WO 201142316 A
- WO 201005958 A
- WO 2014087863 A
- WO 2015186823 A
- WO 2011030841 A


### Non-patent literature cited in the description


- *J. Clin. Invest.,* 2007, vol. 117 (5), 1147-1154
- *J. Exp. Med.,* 2002, vol. 196 (4), 447-457
- *Blood,* 2007, vol. 109 (7), 2871-2877
- *Clin. Cancer Res.,* 2006, vol. 12 (4), 1144-1151
- *Eur. J. Cancer,* 2008, vol. 44 (15), 2266-2275
- *Leukemia,* 2007, vol. 21, 353-355
- *Br. J. Cancer,* 2006, vol. 95 (11), 1555-1561
- *J. Clin. Invest.,* 2004, vol. 114 (2), 280-290
- *Clin. Cancer Res.,* 2005, vol. 11 (16), 6030-6039
- *Leuk. Res.,* 2009, vol. 33 (1), 39-45
- *Leuk. Res.,* 2009, vol. 33 (3), 490-494
- *Dermatology,* 2007, vol. 214 (1), 8-14
- *Cancer Res.,* 2007, vol. 67 (2), 792-800
- *Nat. Immunol.,* 2001, vol. 2 (1), 64-68
- *Nat. Immunol.,* 2001, vol. 2, 64-68
- *Clin. Cancer Res.,* 2005, vol. 11, 5984
- *Br. J. Hematol.,* 2009, vol. 144, 848
- *J Clin Invest,* 01 August 2004, vol. 114 (3), 379-388
- *Journal of Investigative Dermatology,* 2013, vol. 133, 499-508
- *HEPATOLOGY,* 2013, vol. 57 (6), 2358-2368
- Monoclonal Antibodies: Principles and Applications. Wiley-Liss, Inc, 1955
- *Clinical Cancer Research,* 2003, vol. 9, 3625
- *J Invest Dermatol,* 2002, vol. 119, 1405
- *Clin Cancer Res,* 15 January 2015, vol. 21 (2
- **SLAMON et al.** *Science,* 1987, vol. 235, 177-182
- **SLAMON et al.** *Science,* 1989, vol. 244 (707-7), 12
- **BASELGA et al.** *J. Clin. Oncol.,* 1996, vol. 14, 737-744
- **VALENTINE et al.** *J. Biol. Chem.,* 1989, vol. 264 (19), 11282-11287
- **EINFELD et al.** *EMBO J.,* 1988, vol. 7 (3), 711-717
- **ANDERSON et al.** *Blood,* 1984, vol. 63 (6), 1424-1433
- **TEDDER et al.** *J. Immunol.,* 1985, vol. 135 (2), 973-979
- **TEDDER et al.** *Cell. Biochem.,* 1990, vol. 14D, 195
- **W. J. GULLICK et al.** *Cancer Res.,* 1986, vol. 46, 285-292
- **S. COHEN et al.** *J. Biol. Chem.,* 1980, vol. 255, 4834-4842
- **A. B. SCHREIBER et al.** *J. Biol. Chem.,* 1983, vol. 258, 846-853
- **L. HARRIS et al.** *Int. J. Biol. Markers,* 1999, vol. 14, 8-15
- **J. MENDELSOHN ; J. BASELGA.** *Oncogene,* 2000, vol. 19, 6550-6565
- **A. L. ULLRICH et al.** *Nature,* 1984, vol. 307, 418-425
- **J. DOWNWARD et al.** *Nature,* 1984, vol. 307, 521-527
- **C. R. CARLIN et al.** *Mol. Cell. Biol.,* 1986, vol. 6, 257-264
- **F. L. V. MAYES ; M. D. WATERFIELD.** *The EMBO J,* 1984, vol. 3, 531-537

- **G. CARPENTER ; S. COHEN.** *Ann. Rev. Biochem.,* 1979, vol. 48, 193-216
- **M. J. OH et al.** *Clin. Cancer Res.,* 2000, vol. 6, 4760-4763
- *Int J Cancer,* 2006, vol. 119 (2), 243-50
- *Anal Biochem,* 2005, vol. 338 (2), 284-93
- *J Thorac Oncol,* 2012, vol. 7 (5), 833-840
- *J Thorac Cardiovasc Surg,* 2001, vol. 121 (2), 225-33
- *Int J Cancer,* 1997, vol. 74 (2), 193-8
- *Int J Cancer,* 1998, vol. 79 (2), 121-6
- *PLoS One,* 2009, vol. 4 (7), e6292
- **SUN et al.** *Blood,* 1996, vol. 87, 83
- **CHEN et al.** *J Biol Chem,* 2009, vol. 284, 5763
- **BONNET et al.** *Nat Med,* 1997, vol. 3, 730
- **JORDAN et al.** *Leukemia,* 2000, vol. 14, 1777
- *Haematologica,* 2001, vol. 86, 1261
- *Leuk-Lymphoma,* 2006, vol. 47, 207
- **MAJETI et al.** *Proc Natl Acad Sci USA.,* 2009, vol. 106, 3396
- **ANDERSON AC et al.** *Science,* 2007, vol. 318, 1141-3
- **MONNEY L et al.** *Nature,* 2002, vol. 415, 536-41
- **KOGUCHI K et al.** *J Exp Med.,* 2006, vol. 203, 1413-8
- **MAJETI R et al.** *Proc Natl Acad Sci USA,* 03 March 2009, vol. 106 (9), 3396-401
- **CLARK M.** *Chemical Immunology,* 1997, vol. 65, 88
- **GORTER A et al.** *Immunol. Today,* 1999, vol. 20, 576
- *J Exp Med,* 2002, vol. 196 (4), 447-57
- *Nat Rev Cancer,* 2009, vol. 9 (6), 445-52
- *J Exp Med.,* 2002, vol. 196 (4), 447-57
- *Blood,* 2006, vol. 108 (13), 4118-25
- *Methods,* 2001, vol. 25 (4), 402-8
- *Exp Med,* 2002, vol. 196 (4), 447-57
- *Ann Oncol,* 30 June 2015
- *Gynecologic Oncology,* 2009, vol. 115, 185-192
- *Nat Rev Cancer,* 2009, vol. 9, 445-52
- *J Exp Med.,* 2002, vol. 196, 447-57
- *Blood,* 2006, vol. 108, 4118-25